# FASCICULE DE BREVET EUROPEEN

(11) **EP 1 641 453 B1**
(45) Date de publication et mention de la délivrance du brevet: **19.08.2009**
(21) Numéro de dépôt: 04767442.9
(22) Date de dépôt: 24.06.2004
(51) Int. Cl.: A61K 31/425, A61K 31/42, A61P 35/00, C07D 277/66, C07D 277/64, C07D 417/04, C07D 263/56, C07D 417/12, C07D 413/12, C07D 413/04, C07D 519/00

(54) **PRODUIT COMPRENANT AU MOINS UN INHIBITEUR DE PHOSPHATES CDC25 EN ASSOCIATION AVEC AU MOINS UN AUTRE AGENT ANTI-CANCEREUX**
ZUSAMMENSETZUNG DIE MINDESTENS EIN CDC25-PHOSPHATASE INHIBITOR UND MINDESTENS EIN ANDERES ANTI-KREBS MITTEL
PRODUCT CONTAINING AT LEAST ONE PHOSPHATASE CDC25 INHIBITOR COMBINED WITH AT LEAST ONE OTHER ANTICANCER AGENT

(30) Priorité: 25.06.2003 FR 0307649
(43) Date de publication de la demande: 05.04.2006
(60) Demande divisionnaire: 07006144.5
(73) Titulaire: IPSEN PHARMA, 92100 Boulogne-Billancourt (FR)
(72) Inventeur: PREVOST, Grégoire, F-92160 Antony (FR); BREZAK PANNETIER, Marie-Christine, F-92160 Antony (FR); DIOLEZ, Christian, F-91120 Palaiseau (FR)
(74) Mandataire: Bourgouin, André
(86) Numéro de dépôt international: PCT/FR2004/001586
(87) Numéro de publication internationale: WO 2005/000852

(56) Documents cités:
- WO-A-01/34203
- WO-A-97/30053
- WO-A-02/096348
- WO-A-03/050098
- US-A- 5 523 430
- TALAGA, P. ET AL.: "Synthesis of Boc-Cys-Ala-OMe and its Stereoselective Addition to alpa-Methylene-gamma-Butyrolactones" TETRAHEDRON, vol. 45, no. 16, 1989, pages 5029-5038, XP002326456
- ZHU, X. ET AL.: "Synthesis of S-Linked Glycopeptides in Aqueous Solution" J. ORG. CHEM., vol. 68, no. 14, 6 June 2003 (2003-06-06), pages 5641-5651, XP002326455

## Description

La présente invention a pour objet un produit comprenant au moins un inhibiteur de phosphatase Cdc25 en association avec au moins un autre agent anti-cancéreux pour une utilisation thérapeutique simultanée, séparée ou étalée dans le temps dans le traitement du cancer.

Le contrôle de la transition entre les différentes phases du cycle cellulaire durant la mitose ou de la méiose est assurée par un ensemble de protéines dont les activités enzymatiques sont associées à des états différents de phosphorylation. Ces états sont contrôlés par deux grandes classes d'enzymes : les kinases et les phosphatases.

La synchronisation des différentes phases du cycle cellulaire permet ainsi la réorganisation de l'architecture cellulaire à chaque cycle dans l'ensemble du monde vivant (microorganismes, levure, vertébrés, plantes). Parmi les kinases, les kinases dépendantes des cyclines (CDKs) jouent un rôle majeur dans ce contrôle du cycle cellulaire. L'activité enzymatique de ces différentes CDKs est contrôlée par deux autres familles d'enzymes qui travaillent en opposition (Jessus et Ozon, Prog. Cell Cycle Res. (1995), 1, 215-228). La première regroupe des kinases telles que Wee1 et Mik1 qui désactivent les CDKs en phosphorylant certains acides aminés (Den Haese et coll., Mol. Biol. Cell (1995), 6, 371-385). La seconde regroupe des phosphatases telle que Cdc25 qui activent les CDKs en déphosphorylant des résidus tyrosine et thréonine de CDKs (Gould et coll., Science (1990), 250, 1573-1576).

Les phosphatases sont classifiées en 3 groupes : les sérines/thréonines phosphatases (PPases), les tyrosines phosphatases (PTPases) et les phosphatases à double spécificité (DSPases). Ces phosphatases jouent un rôle important dans la régulation de nombreuses fonctions cellulaires.

En ce qui concerne les phosphatases Cdc25 humaines, 3 gènes (Cdc25-A, Cdc25-B et Cdc25-C) codent pour les protéines Cdc25. De plus, des variants issus de splicing alternatif des gènes Cdc25 ont été identifiés (cf. par exemple Baldin et coll., Oncogene (1997), 14, 2485-2495).

Le rôle des phosphatases Cdc25 dans l'oncogénèse est maintenant mieux connu et les mécanismes d'action de ces phosphatases sont illustrés en particulier dans les références suivantes : Galaktionov et coll., Science (1995), 269, 1575-1577 ; Galaktionov et coll., Nature (1996), 382, 511-517 ; et Mailand et coll., Science (2000), 288, 1425-1429.

En particulier, la surexpression des différentes formes de Cdc25 est maintenant reportée dans de nombreuses séries de tumeurs humaines :
- Cancer du sein : cf. Cangi et coll., Résumé 2984, AACR meeting San Francisco, 2000) ;
- Lymphomes : cf. Hernandez et coll., Int. J. Cancer (2000), 89, 148-152 et Hernandez et coll., Cancer Res. (1998), 58, 1762-1767 ;
- Cancers du cou et de la tête : cf. Gasparotto et coll., Cancer Res. (1997), 57, 2366-2368.

Par ailleurs, le groupe de E. Sausville rapporte une corrélation inverse entre le niveau d'expression de Cdc25-B dans un panel de 60 lignées et leurs sensibilités aux inhibiteurs de CDK, suggérant que la présence de Cdc25 puisse apporter une résistance à certains agents antitumoraux et plus particulièrement aux inhibiteurs de CDK (Hose et coll., Proceedings of AACR, Abstract 3571, San Francisco, 2000).

Parmi d'autres cibles, on recherche donc à présent des composés capables d'inhiber les phosphatases Cdc25 afin de les utiliser notamment comme agents anti-cancéreux.

L'invention concerne un produit comprenant au moins un inhibiteur de phosphatase Cdc25 en association avec au moins un autre agent anti-cancéreux pour une utilisation thérapeutique simultanée, séparée ou étalée dans le temps dans le traitement du cancer.

De préférence, l'invention concernera un produit comprenant un inhibiteur de phosphatase Cdc25 en association avec un autre agent anti-cancéreux pour une utilisation thérapeutique simultanée, séparée ou étalée dans le temps dans le traitement du cancer.

Par utilisation thérapeutique simultanée, on entend dans la présente demande une administration de plusieurs principes actifs par la même voie et au même moment. Par utilisation séparée, on entend notamment une administration de plusieurs principes actifs sensiblement au même moment par des voies différentes. Par utilisation thérapeutique étalée dans le temps, on entend une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

Selon l'invention, l'agent anti-cancéreux associé à l'inhibiteur de phosphatase Cdc25 sera de préférence tel qu'il agisse selon une autre voie que les phosphatases Cdc25. En particulier, ledit agent anti-cancéreux associé aura une concentration inhibitrice CI₅₀ d'au moins 50 µM par rapport aux phosphatases Cdc25 ou alors aura une autre activité avec une dose CI₅₀ au moins 10 fois plus faible que celle par rapport aux phosphatases Cdc25. L'association réalisée selon l'invention est telle qu'elle présente une synergie.

Selon l'invention, l'inhibiteur de phosphatase Cdc25 est choisi parmi les dérivés de benzothiazole-4,7-diones et de benzooxazole-4,7-diones répondant à la formule générale **(I)** définie ci-après.

Un certain nombre de dérivés de benzothiazole-4,7-diones et de benzooxazole-4,7-diones est déjà connu.

En particulier, le brevet GB 1 534 275 concerne des herbicides dont le principe actif est un composé répondant à l'une des formules générales 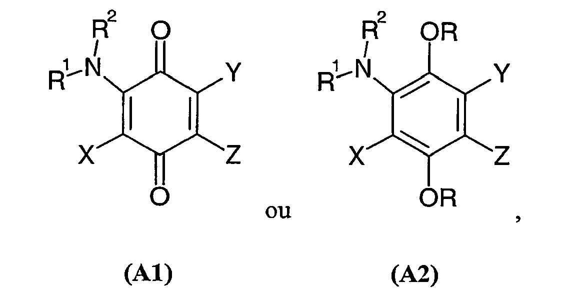 dans lesquelles :
R¹ représente notamment un atome d'hydrogène ou un radical alkyle ou cycloalkyle ;
R² représente notamment un atome d'hydrogène, un radical alkyle ou cycloalkyle ;
X représente notamment un atome halogène ou un radical alkoxy ;
Y et Z peuvent notamment représenter ensemble avec les atomes de carbone qui les portent un cycle thiazole éventuellement substitué par un radical alkyle ; et
R représente notamment un radical alkyle.

Par ailleurs, la demande de brevet PCT WO 99/32115 décrit les composés de formule générale **(A3)** 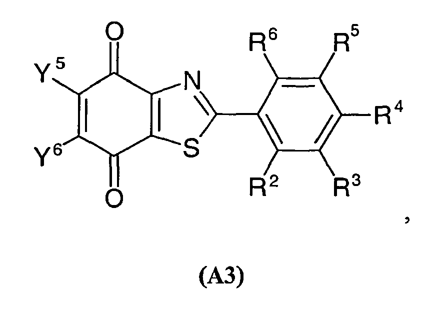 dans laquelle :
les substituants R²-R⁶ sont choisis parmi le groupe constitué d'un atome d'hydrogène, de substituants donneurs d'électrons, de substituants attracteurs d'électrons et de substituants modulateurs d'électrons ;
et Y⁵ et Y⁶ sont notamment choisis parmi le groupe constitué d'un atome d'hydrogène, de substituants donneurs d'électrons, de substituants attracteurs d'électrons et de substituants modulateurs d'électrons.

Dans la demande de brevet PCT WO 99/32115, le terme « substituant donneur d'électrons » fait référence à un groupe fonctionnel ayant tendance à donner de la densité électronique ; sont cités les substituants alkyle, alkényle et alkynyle. Toujours dans cette demande de brevet, « substituant attracteur d'électrons » fait référence à un groupe fonctionnel ayant tendance à attraire de la densité électronique ; sont cités les substituants cyano, acyle, carbonyle, fluoro, nitro, sulfonyle et trihalométhyle. Enfin, un « substituant modulateur d'électrons » est défini dans cette demande comme un groupe fonctionnel ayant tendance à moduler la densité électronique, lequel peut à la fois se attirer et donner des électrons et est donc tel qu'il puisse stabiliser un intermédiaire cationique dans une réaction de substitution électrophile aromatique ; est cité un groupe fonctionnel incluant, par exemple, des substituants amino (par exemple -NH₂, alkylamino ou dialkylamino), hydroxy, alkoxy ou aryle, des substituants hétérocycliques, des atomes halogènes, etc.

Les composés de formule générale **(A3)** sont présentés comme des modulateurs des récepteurs de la ryanodine qui peuvent être utilisés en tant que pesticides ou en tant qu'agents thérapeutiques, par exemple dans le traitement de la défaillance cardiaque congestive, des maux de tête migraineux, de l'hypertension, de la maladie de Parkinson ou de la maladie d'Alzheimer ou dans la prévention de l'avortement prématuré.

Enfin, les dérivés de benzooxazole-4,7-diones de formule générale **(A4)** 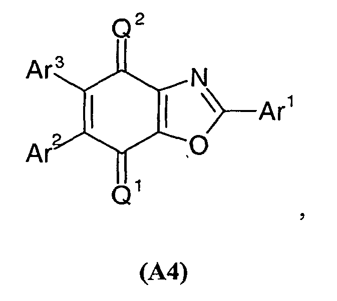 dans laquelle :
Ar¹ représente un radical aryle éventuellement substitué,
chacun de Ar² et Ar³ représente un atome d'hydrogène ou un radical aryle éventuellement substitué, et
chacun de Q¹ et Q² représente notamment O,
sont décrits en tant que constituants actifs de couches photosensibles de photorécepteurs.

Dans la demande de brevet, WO03/055868, la demanderesse a décrit les composés répondant à la formule générale **(I)** 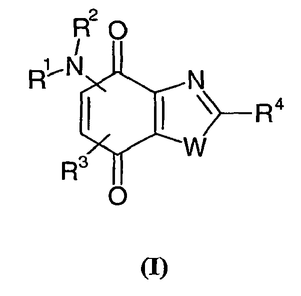 dans laquelle :
R¹ représente un atome d'hydrogène ou un radical alkyle, alkoxyalkyle, alkylthioalkyle, cycloalkyle, -(CH₂)-X-Y, -(CH₂)-Z-NR⁵R⁶ ou un radical -CHR³⁵R³⁶ dans lequel R³⁵ et R³⁶ forment ensemble avec l'atome de carbone qui les porte un radical indanyle ou tétralinyle, ou encore R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle,
R¹ pouvant aussi, lorsque W représente O, représenter en outre un radical aryle carbocyclique éventuellement substitué de 1 à 3 fois par des substituants choisis indépendamment parmi un atome halogène et un radical alkyle, haloalkyle ou alkoxy, X représentant une liaison ou un radical alkylène linéaire ou ramifié comptant de 1 à 5 atomes de carbone,
Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR⁷-, -CO-, -NR⁸-, -O- et -S-, R⁷ représentant un atome d'hydrogène ou un radical alkyle et R⁸ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR⁹ et un radical NR¹⁰R¹¹, R⁹ représentant un atome d'hydrogène ou un radical alkyle ou phényle, et R¹⁰ et R¹¹ représentant indépendamment des radicaux alkyle,
Z représentant une liaison ou un radical alkylène linéaire ou ramifié comptant de 1 à 5 atomes de carbone,
R⁵ et R⁶ étant choisis indépendamment parmi un atome d'hydrogène, un radical alkyle, aralkyle ou -(CH₂)ₙ-OH dans lequel n représente un entier de 1 à 6,
ou R⁵ représentant un radical alkoxycarbonyle, haloalkoxycarbonyle ou aralkoxycarbonyle et R⁶ représentant un atome d'hydrogène ou un radical méthyle,
ou encore R⁵ et R⁶ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹²R¹³-, -O-, -S- et -NR¹⁴-, R¹² et R¹³ représentant indépendamment à chaque fois qu'ils interviennent un atome d'hydrogène ou un radical alkyle, et R¹⁴ représentant un atome d'hydrogène ou un radical alkyle ou aralkyle, ou encore R¹⁴ représentant un radical phényle éventuellement substitué de 1 à 3 fois par des substituants choisis indépendamment parmi un atome halogène et un radical alkyle ou alkoxy,
R² représentant un atome d'hydrogène ou un radical alkyle ou aralkyle ;
ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle ;
R³ représente un atome d'hydrogène, un atome halogène, ou un radical alkyle, haloalkyle, alkoxy ou alkylthio ;
R⁴ représente un radical alkyle, cycloalkyle, cycloalkylalkyle, cyano, amino, -CH₂-COOR¹⁸, -CH₂-CO-NR¹⁹R²⁰ ou -CH₂-NR²¹R²², ou bien R⁴ représente un radical aryle carbocyclique ou hétérocyclique éventuellement substitué de 1 à 4 fois par des substituants choisis indépendamment parmi un atome halogène et un radical alkyle, haloalkyle, alkoxy, haloalkoxy ou NR³⁷R³⁸, ou encore R⁴ représente un radical phényle possédant deux substituants qui forment ensemble un radical méthylènedioxy ou éthylènedioxy,
R¹⁸ représentant un atome d'hydrogène ou un radical alkyle,
R¹⁹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²³ et un radical NR²⁴R²⁵, R²³ représentant un atome d'hydrogène ou un radical alkyle ou phényle, et R²⁴ et R²⁵ représentant indépendamment des radicaux alkyle,
R²⁰ représentant un atome d'hydrogène ou un radical alkyle,
ou encore R¹⁹ et R²⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR²⁶R²⁷-, -O-, -S- et -NR²⁸-, R²⁶ et R²⁷ représentant indépendamment à chaque fois qu'ils interviennent un atome d'hydrogène ou un radical alkyle, et R²⁸ représentant un atome d'hydrogène ou un radical alkyle ou aralkyle, ou encore R²⁸ représentant un radical phényle éventuellement substitué de 1 à 3 fois par des substituants choisis indépendamment parmi un atome halogène et un radical alkyle ou alkoxy,
R²¹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²⁹ et un radical NR³⁰R³¹, R²⁹ représentant un atome d'hydrogène ou un radical alkyle ou phényle, et R³⁰ et R³¹ représentant indépendamment des radicaux alkyle,
R²² représentant un atome d'hydrogène ou un radical alkyle, ou encore R²¹ et R²² formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³²R³³-, -O-, -S- et -NR³⁴-, R³² et R³³ représentant indépendamment à chaque fois qu'ils interviennent un atome d'hydrogène ou un radical alkyle, et R³⁴ représentant un atome d'hydrogène, un radical alkyle ou aralkyle, ou encore R³⁴ représentant un radical phényle éventuellement substitué de 1 à 3 fois par des substituants choisis indépendamment parmi un atome halogène et un radical alkyle ou alkoxy,
R³⁷ et R³⁸ étant choisis indépendamment parmi un atome d'hydrogène et un radical alkyle ou R³⁷ et R³⁸ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³⁹R⁴⁰-, -O-, -S- et
-NR⁴¹-, R³⁹ et R⁴⁰ représentant indépendamment à chaque fois qu'ils interviennent un atome d'hydrogène ou un radical alkyle, et R⁴¹ représentant un atome d'hydrogène ou un radical alkyle ; et
W représente O ou S ;
ou les sels pharmaceutiquement acceptables de composés de formule générale **(I)** définie ci-dessus
en tant qu'inhibiteurs de phosphatases Cdc25, et en particulier des inhibiteurs de la phosphatase Cdc25-C, et/ou de la phosphatase CD 45. Lesdits composés peuvent donc être utilisés pour préparer un médicament destiné à inhiber les phosphatases Cdc25, et en particulier la phosphatase Cdc25-C, et/ou la phosphatase CD 45.

Par alkyle, lorsqu'il n'est pas donné plus de précision, on entend un radical alkyle linéaire ou ramifié comptant de 1 à 12 atomes de carbone, de préférence de 1 à 10 atomes de carbone et plus préférentiellement 1 à 8 atomes de carbone (et notamment de 1 à 6 atomes de carbone). Par alkyle inférieur, lorsqu'il n'est pas donné plus de précision, on entend un radical alkyle linéaire ou ramifié comptant de 1 à 6 atomes de carbone. Par cycloalkyle, lorsqu'il n'est pas donné plus de précision, on entend un radical cycloalkyle comptant de 3 à 7 atomes de carbone. Par aryle carbocyclique ou hétérocyclique, on entend un système carbocyclique ou hétérocyclique de 1 à 3 cycles condensés comprenant au moins un cycle aromatique, un système étant dit hétérocyclique lorsque l'un au moins des cycles qui le composent comporte un hétéroatome (O, N ou S) ; lorsqu'un radical aryle carbocyclique ou hétérocyclique est dit substitué sans qu'il soit donné plus de précision, on entend que ledit radical aryle carbocyclique ou hétérocyclique est substitué de 1 à 3 fois, et de préférence de 1 à 2 fois par des radicaux différents d'un atome d'hydrogène qui, s'ils ne sont pas précisés, sont choisis parmi un atome halogène et les radicaux alkyle ou alkoxy; par ailleurs, lorsqu'il n'est pas donné plus de précision, on entend par aryle un aryle carbocyclique exclusivement. Par haloalkyle, on entend un radical alkyle dont au moins l'un des atomes d'hydrogène (et éventuellement tous) est remplacé par un atome halogène.

Par radicaux cycloalkylalkyle, alkoxy, haloalkyle, haloalkoxy et aralkyle, on entend respectivement les radicaux cycloalkylalkyle, alkoxy, haloalkyle, haloalkoxy et aralkyle dont les radicaux alkyle, cycloalkyle et aryle ont les significations indiquées précédemment.

Lorsqu'il est indiqué qu'un radical est éventuellement substitué de 1 à 3 fois, il est de préférence éventuellement substitué de 1 à 2 fois et plus préférentiellement éventuellement substitué une fois.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Par haloalkyle, on entend notamment le radical trifluorométhyle. Par haloalkoxy, on entend notamment le radical trifluorométhoxy. Par aryle carbocyclique, on entend en particulier les radicaux phényle et naphtyle. Par aralkyle, on entend en particulier les radicaux phénylalkyle, et notamment le radical benzyle. Par système cyclique carboné saturé comptant de 1 à 3 cycles condensés choisis indépendamment parmi des cycles de 3 à 7 chaînons, on entend en particulier les radicaux cyclopropyle, cyclobutyle, cyclohexyle et adamantyle. Par aryle hétérocyclique ou hétéroaryle, on entend en particulier les radicaux thiényle, furannyle, pyrrolyle, imidazolyle, thiazolyle, oxazolyle et pyridyle. Enfin, par halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Dans certains cas, les composés de formule générale **(I)** peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Les produits selon la présente invention comprenant un composé de formule générale **(I)** présentent aussi de façon générale quatre variantes :
- selon une première variante, les composés de formule générale **(I)** qui répondent aussi à la sous-formule générale **(I)₁** 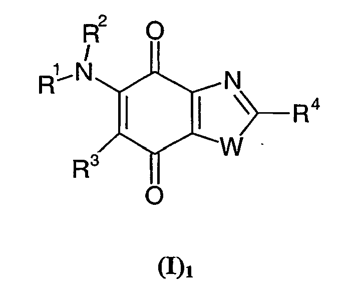 dans laquelle W représente S et R¹, R², R³ et R⁴ ont la même signification que dans la formule générale **(I),** ou leurs sels pharmaceutiquement acceptables, seront les composés de formule générale **(I)** inclus dans le produit de l'invention ;
- selon une deuxième variante, les composés de formule générale **(I)** qui répondent aussi à la sous-formule générale **(I)₂** 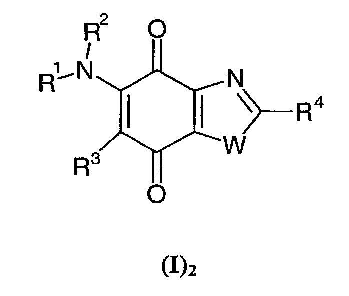 dans laquelle W représente O et R¹, R², R³ et R⁴ ont la même signification que dans la formule générale **(I),** ou leurs sels pharmaceutiquement acceptables, seront les composés de formule générale **(I)** inclus dans le produit de l'invention ;
- selon une troisième variante, les composés de formule générale **(I)** qui répondent aussi à la sous-formule générale **(I)₃** 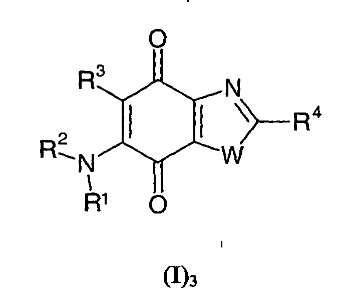 dans laquelle W représente S et R¹, R², R³ et R⁴ ont la même signification que dans la formule générale **(I),** ou leurs sels pharmaceutiquement acceptables, seront les composés de formule générale **(I)** inclus dans le produit de l'invention ; et
- selon une quatrième variante, les composés de formule générale **(I)** qui répondent aussi à la sous-formule générale **(I)₄** 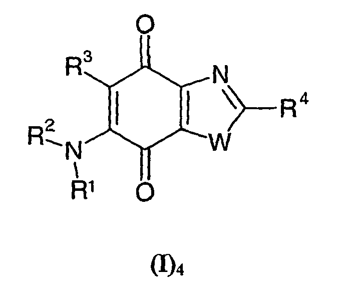 dans laquelle W représente O et R¹, R², R³ et R⁴ ont la même signification que dans la formule générale **(I),** ou leurs sels pharmaceutiquement acceptables, seront les composés de formule générale **(I)** inclus dans le produit de l'invention.

L'invention concerne donc notamment les produits précédemment mentionnés comprenant au moins un composé choisi parmi les composés de formule générale **(I)₁** ou **(I)₂**, ou de leurs sels pharmaceutiquement acceptables. De même, l'invention concerne les produits précédemment mentionnés comprenant au moins un composé choisi parmi les composés de formule générale **(I)₃** ou **(I)₄,** ou leurs sels pharmaceutiquement acceptables.

De préférence, les composés de formule générale **(I), (I)₁, (I)₂, (I)₃** ou **(I)₄** inclus dans un produit selon l'invention possèderont au moins l'une des caractéristiques suivantes :
- R¹ représentant un radical alkyle, cycloalkyle, alkoxyalkyle, -(CH₂)-X-Y, -(CH₂)-Z-NR⁵R⁶ Ou-CHR³⁵R³⁶ ;
- R² représentant un atome d'hydrogène ou le radical méthyle, éthyle ou benzyle ;
- R¹ et R² formant ensemble avec l'atome d'azote un hétérocycle de 4 à 8 chaînons (de préférence de 5 à 7 chaînons, et notamment de 6 chaînons) comportant de 1 à 2 hétéroatomes (et de préférence 2 hétéroatomes), les chaînons nécessaires pour compléter l'hétérocycle étant choisis indépendamment parmi les radicaux -CH₂-, -O- et -NR¹⁷ (et de préférence parmi les radicaux -CH₂- et -NR¹⁷-), R¹⁷ représentant un radical méthyle ou benzyle ;
- R³ représentant un atome d'hydrogène, un atome halogène ou un radical alkyle, alkoxy ou alkylthio ;
- R⁴ représentant un radical alkyle, -CH₂-COOR¹⁸ ou -CH₂-CO-NR¹⁹R²⁰ ou -CH₂-NR²¹R²² ou encore un radical aryle carbocyclique ou hétérocyclique éventuellement substitué de 1 à 4 fois (et notamment de 1 à 3 fois) par des substituants choisis indépendamment parmi un atome halogène et un radical alkyle, haloalkyle, alkoxy ou NR³⁷R³⁸.

D'une façon générale, on préférera, pour un produit selon l'invention, les composés de formule générale **(I)** dans lesquels W représente un atome de soufre. Une autre alternative intéressante pour un produit selon l'invention consistera néanmoins à y inclure des composés de formule générale **(I)** dans lesquels W représente un atome d'oxygène.

Par ailleurs, le radical X représentera de préférence une liaison ou un radical alkylène linéaire comptant de 1 à 5 atomes de carbone. De préférence aussi, le radical Y représentera un système cyclique carboné saturé comptant de 1 à 3 cycles condensés choisis indépendamment parmi des cycles de 3 à 7 chaînons, ou Y représentera un radical aryle carbocyclique éventuellement substitué (de préférence éventuellement substitué par 1 à 3 radicaux choisis parmi un atome halogène et un radical alkyle, haloalkyle, alkoxy, haloalkoxy, SO₂NHR⁹ ou NR¹⁰R¹¹, et plus préférentiellement éventuellement substitué par 1 à 3 radicaux choisis parmi un atome halogène et un radical alkyle, alkoxy, SO₂NHR⁹ ou NR¹⁰R¹¹) ou encore Y représentera un radical aryle hétérocyclique éventuellement substitué, ledit radical aryle hétérocyclique étant de préférence choisi parmi les radicaux aryle à 5 chaînons (et notamment parmi les radicaux imidazolyle, thiényle ou pyridinyle) et de préférence éventuellement substitué par 1 à 3 radicaux choisis parmi un atome halogène et un radical alkyle, haloalkyle, alkoxy, haloalkoxy, SO₂NHR⁹ ou NR¹⁰R¹¹, et plus préférentiellement éventuellement substitué par 1 à 3 radicaux choisis parmi un atome halogène et un radical alkyle, alkoxy, SO₂NHR⁹ ou NR¹⁰R¹¹ ; R⁹ représentera de préférence un atome d'hydrogène et R¹⁰ et R¹¹ représenteront de préférence des radicaux choisis indépendamment parmi les radicaux alkyle. Le radical Z représentera de préférence un radical alkylène comptant de 1 à 5 atomes de carbone, et en particulier un radical -(CH₂)ₚ- dans lequel p représente un entier de 1 à 3 (p étant de préférence égal à 1 ou 2 et plus préférentiellement égal à 1). De préférence également, R⁵ et R⁶ seront choisis indépendamment parmi un atome d'hydrogène et un radical alkyle, ou encore R⁵ et R⁶ formeront ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence par 1 à 3 radicaux méthyle) ; de façon encore plus préférentielle, R⁵ et R⁶ seront choisis indépendamment parmi des radicaux alkyle ou alkoxycarbonyle (et en particulier R⁵ et R⁶ seront chacun un radical méthyle ou *tert*-butoxycarbonyle) ou R⁵ et R⁶ formeront ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence par 1 à 3 radicaux méthyle). R¹⁸ représentera de préférence un atome d'hydrogène ou le radical méthyle ou éthyle.

De plus, les radicaux R⁷, R¹²,R¹³, R¹⁵, R¹⁶, R²⁶, R²⁷, R³⁹ et R⁴⁰ seront de préférence choisis indépendamment parmi un atome d'hydrogène et un radical méthyle et les radicaux R⁸, R¹⁴, R¹⁷, R²⁸ et R⁴¹ seront de préférence choisis indépendamment parmi un atome d'hydrogène et un radical méthyle ou benzyle.

En outre, en ce qui concerne R¹⁹ et R²⁰, on préférera les cas dans lesquels R¹⁹ représente un atome d'hydrogène, un radical alkyle ou un radical benzyle et R²⁰ représente un atome d'hydrogène ou le radical méthyle, ainsi que ceux dans lesquels R¹⁹ et R²⁰ forment ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués par 1 à 3 radicaux alkyle (et de préférence éventuellement substitués par 1 à 3 radicaux méthyle).

Par ailleurs, en ce qui concerne R²¹ et R²², on préférera les cas dans lesquels R²¹ représente un atome d'hydrogène, un radical alkyle ou un radical benzyle et R²² représente un atome d'hydrogène ou le radical méthyle, ainsi que ceux dans lesquels R²¹ et R²² forment ensemble avec l'atome d'azote qui les porte un hétérocycle de 4 à 7 chaînons comportant de 1 à 2 hétéroatomes, ledit hétérocycle étant alors de préférence l'un des radicaux azétidinyle, pyrrolidinyle, pipéridinyle, pipérazinyle, homopipérazinyle, morpholinyle et thiomorpholinyle éventuellement substitués. En ce qui concerne les radicaux R³², R³³ et R³⁴ correspondants, ceux-ci seront de préférence tels que R³² et R³³ soient choisis indépendamment parmi un atome d'hydrogène et un radical alkyle et de préférence parmi un atome d'hydrogène et un radical méthyle (R³² et R³³ représentant encore plus préférentiellement tous deux des atomes d'hydrogène) et que R³⁴ représente un atome d'hydrogène, un radical alkyle ou un radical phényle éventuellement substitué de 1 à 3 fois par des substituants choisis indépendamment parmi un atome halogène et un radical alkyle ou alkoxy (R³⁴ représentant de façon encore plus préférentielle un atome d'hydrogène ou un radical méthyle ou phényle).

De plus, en ce qui concerne R³⁵ et R³⁶, on préférera les cas dans lesquels R³⁵ et R³⁶ forment ensemble avec l'atome de carbone qui les porte un radical indanyle ou R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle.

Par ailleurs, en ce qui concerne R³⁷ et R³⁸, on préférera les cas dans lesquels R³⁷ et R³⁸ représentent indépendamment des radicaux choisis parmi les radicaux alkyle.

Enfin, lorsque R⁴ est un radical aryle carbocyclique ou hétérocyclique éventuellement substitué de 1 à 4 fois, l'on préfère qu'il soit choisi dans le groupe consistant en des radicaux aryle carbocycliques et hétérocycliques éventuellement substitués de 1 à 3 fois par des substituants choisis indépendamment parmi un atome halogène et un radical alkyle, haloalkyle, alkoxy, haloalkoxy ou NR³⁷R³⁸ (et en particulier de 1 à 3 fois par des substituants choisis indépendamment parmi un atome halogène et un radical alkyle, haloalkyle, alkoxy ou haloalkoxy) et le radical 2,3,4,5-tétrafluorophényle. Plus préférentiellement, lorsque R⁴ est un radical aryle carbocyclique ou hétérocyclique éventuellement substitué de 1 à 4 fois, R⁴ sera choisi dans le groupe consistant en des radicaux aryle carbocycliques et hétérocycliques éventuellement substitués de 1 à 2 fois par des substituants choisis indépendamment un atome halogène, un radical alkyle, haloalkyle, alkoxy, haloalkoxy ou NR³⁷R³⁸ (et en particulier 1 à 2 fois par des substituants choisis indépendamment parmi un atome halogène et un radical alkyle, haloalkyl, alkoxy ou haloalkoxy), un radical 3,4,5-trihalophényle et le radical 2,3,4,5-tétrafluorophényle.

Plus préférentiellement, les composés de formule générale **(I), (I)₁, (I)₂, (I)₃** ou **(I)₄** inclus dans un produit selon l'invention possèderont au moins l'une des caractéristiques suivantes :
- R¹ représentant un radical alkyle, cycloalkyle, ou -(CH₂)-Z-NR⁵R⁶ ;
- R² représentant un atome d'hydrogène ou le radical méthyle ;
- R³ représentant un atome d'hydrogène, un atome halogène ou le radical méthoxy ;
- R⁴ représentant un radical alkyle, -CH₂-NR²¹R²², ou encore un radical aryle carbocyclique ou hétérocyclique éventuellement substitué de 1 à 4 fois (et notamment de 1 à 3 fois) par des substituants choisis indépendamment parmi un atome halogène et un radical alkyle, ou NR³⁷R³⁸.

Encore plus préférentiellement, les composés de formule générale **(I), (I)₁, (I)₂, (I)₃** ou **(I)₄** inclus dans un produit selon l'invention possèderont au moins l'une des caractéristiques suivantes :
- R¹ représentant un radical-(CH₂)-Z-NR⁵R⁶ ;
- R² représentant un atome d'hydrogène ;
- R³ représentant un atome d'hydrogène ou un atome halogène (ledit atome halogène étant de préférence un atome de chlore ou de brome) ;
- R⁴ représentant un radical alkyle ou encore un radical phényle, pyridyle, thiényle ou furannyle éventuellement substitué par 1 à 4 (de préférence 1 à 3) atomes halogènes ou par un radical NR³⁷R³⁸.

De façon encore plus particulièrement préférée, les composés de formule générale **(I), (I)₁, (I)₂, (I)₃** ou **(I)₄** inclus dans un produit selon l'invention possèderont au moins l'une des caractéristiques suivantes :
- R³ représentant un atome d'hydrogène ou un atome de chlore (et plus préférentiellement un atome d'hydrogène) ;
- R⁴ représentant un radical alkyle ou encore un radical phényle, pyridyle, thiényle furannyle éventuellement substitué par 1 à 4 (de préférence 1 à 3) atomes halogènes (et en particulier R⁴ représentant un radical alkyle, et de préférence un radical alkyle comptant de 1 à 4 atomes de carbone, et plus préférentiellement encore un radical méthyle ou éthyle).

Selon une variante particulière de l'invention, W représente O. Dans ce cas particulier, l'on préférera que R¹ représente un radical aryle, et en particulier un radical phényle, éventuellement substitué de 1 à 3 fois par des substituants choisis indépendamment parmi un atome halogène et un radical alkyle, haloalkyle ou alkoxy. Plus préférentiellement, toujours lorsque W représente O, l'on préférera que R¹ représente un radical phényle éventuellement substitué par un atome halogène (ledit atome halogène étant de préférence un atome de fluor).

Selon un aspect particulier de l'invention, R⁴ représentera un radical phényle ou un radical aryle hétérocyclique de 5 à 6 chaînons éventuellement substitué de 1 à 4 fois (et de préférence de 1 à 3 fois) par des substituants choisis parmi le groupe consistant en des atomes halogènes, le radical trifluorométhyle et le radical trifluorométhoxy (et de préférence choisis parmi le groupe consistant en des atomes halogènes et le radical trifluorométhyle). En particulier, ledit aryle hétérocyclique de 5 à 6 chaînons éventuellement substitué sera un cycle pyridine, thiophène, furanne ou pyrrole éventuellement substitué.

Un autre aspect particulier de cette invention concerne l'utilisation de composés de formule générale **(I)** dans laquelle W représente S, R³ représente un atome d'hydrogène, le substituant -NR¹R² (les préférences indiquées précédemment pour R¹ et R² restant applicables) est attaché à la position 5 du cycle benzothiazoledione et R⁴ est choisi parmi les radicaux alkyle, cycloalkylalkyl, -CH₂-COOR¹⁸, -CH₂-CO-NR¹⁹R²⁰ et -CH₂-NR²¹R²² (R⁴ étant de préférence alkyle ou cycloalkylalkyle et plus préférentiellement alkyle selon cet aspect particulier de l'invention).

Pour un produit selon l'invention, les composés de formule générale **(I)** décrits (le cas échéant sous forme de sels ou de mélanges) dans les exemples 1 à 138 de composés de formule générale **(I),** ou les sels pharmaceutiquement acceptables de tels composés, seront particulièrement préférés. Parmi les composés des exemples 1 à 138 de composés de formule générale **(I)** et leurs sels pharmaceutiquement acceptables, les composés des exemples 1 à 14, 18 à 39, 48 à 52, 55, 57, 58 et 60 à 138 présenteront d'une manière générale plus d'intérêt pour être inclus dans un produit selon cette invention.

De plus, les composés de formule générale **(I)** décrits (le cas échéant sous forme de sels ou de mélanges) dans les exemples 2 à 5, 16, 19 à 26, 32, 34, 38 à 40, 43 à 47, 55 à 58, 60 à 77, 79 à 98 et 101 à 115 de composés de formule générale **(I),** ou les sels pharmaceutiquement acceptables de tels composés, seront encore plus particulièrement préférés pour être inclus dans un produit selon l'invention.

En outre, les composés de formule générale **(I)** décrits (le cas échéant sous forme de sels ou de mélanges) dans les exemples 2, 19, 20, 23, 24, 34, 57, 60, 62, 63, 67 à 77, 80 à 92, 94, 96 à 98, 103, 104, 106 et 110 à 113 de composés de formule générale **(I),** ou les sels pharmaceutiquement acceptables de tels composés, seront tout particulièrement préférés pour être inclus dans un produit selon l'invention:

De façon particulièrement préférée, les produits selon l'invention comprenant un composé de formule générale **(I)** incluront un composé choisi parmi les composés suivants :
- 5-{[2-(diméthylammo)éthyl]ammo}-2-métbyl-1,3-benzothiazole-4,7-dione ;
- 2-méthyl-5-[(2-pyrrolidin-1-yléthyl)amino]-1,3-benzothiazole-4,7-dione ;
- 2-méthyl-5-[(2-pipéridin-1-yléthyl)amino]-1,3-benzothiazole-4,7-dione ;
- 2-(2-chloro-6-fluorophényl)-5-{[2-(diméthylamino)éthyl]amino}-1,3-benzothiazole-4,7-dione ;
et les sels pharmaceutiquement acceptables de ces derniers.

Selon un aspect encore plus préféré de l'invention, les produits selon l'invention comprenant un composé de formule générale **(I)** incluront la 5-{[2-(diméthylamino)éthyl]amino}-2-méthyl-1,3-benzothiazole-4,7-dione ou un de ses sels pharmaceutiquement acceptables.

L'agent anti-cancéreux associé à l'inhibiteur de phosphatase Cdc25 pourra être choisi parmi des inhibiteurs de topoisomérases de type I et/ou II comme par exemple la camptothécine et ses analogues, la doxorubicine ou l'amsacrine.

En ce qui concerne les analogues de la camptothécine pouvant être associés à l'inhibiteur de phosphatases Cdc25, ceux-ci pourront être des analogues comportant un cycle lactonique E à six chaînons (tels par exemple les composés décrits dans la demande de brevet PCT WO 94/11376), des analogues comportant un cycle lactonique E à sept chaînons (tels par exemple les homocamptothécines - les composés décrits dans la demande de brevet PCT WO 97/00876) ou des analogues tétracycliques ouverts (tels par exemple les composés décrits dans la demande de brevet PCT WO 99/33829). De préférence, l'analogue de la camptothécine sera choisi parmi le groupe composé du diflomotécan, de la (+)-9-chloro-5-éthyl-5-hydroxy-10-méthyl-12-(4-méthylpipéridinométhyl)-4,5,13,15-tétrabydro-1H,3H-oxépino[3',4':6,7]indolizino[1,2-c]quinoline-3,15-dione et ses sels (en particulier son chlorhydrate aussi connu sous le nom de BN-80927) ainsi que le composé connu sous le nom de code SN-38.

Par homocamptothécine, on entend dans la présente demande tout analogue de la camptothécine dans lequel le motif pentacyclique de la camptothécine naturelle a été modifié par remplacement, dans le cycle E, de l'α-hydroxylactone naturelle de la camptothécine par une β-hydroxylactone.

Selon une variante particulière de l'invention, les analogues de la camptothécine associés à l'inhibiteur de phosphatases Cdc25 seront des analogues comportant un cycle lactonique E à sept chaînons. Ceux-ci seront de préférence des homocamptothécines, et en particulier des homocamptothécines choisies parmi les composés de formule générale **(III)** 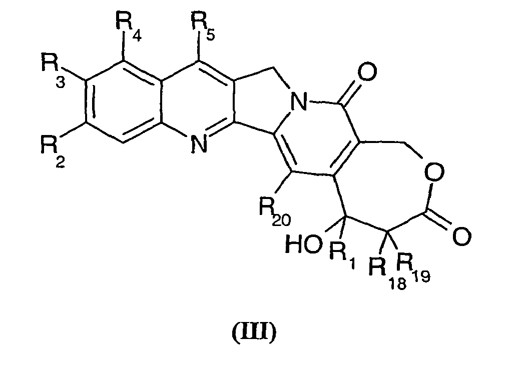 sous forme racémique, d'énantiomère ou toutes combinaisons de ces formes, dans laquelle
- R₁: représente un alkyle inférieur, un alkényle inférieur, un alkynyl inférieur, un haloalkyle inférieur, un alkoxy inférieur alkyle inférieur ou un alkylthio inférieur alkyle inférieur;
- R₂, R₃ et R₄: représentent, indépendamment, i) H, halo, halo alkyle inférieur, alkyle inférieur, alkényle inférieur, cyano, cyano alkyle inférieur, nitro, nitro alkyle inférieur, amido, amido alkyle inférieur, hydrazino, hydrazino alkyle inférieur, azido, azido alkyle inférieur, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆, ou ii) les radicaux substitués (c'est-à-dire, substitué une à quatre fois sur le groupe aryle ou l'hétérocycle) ou non substitués suivants : (CH₂)ₙ[N=X], OC(O)[N=X], (CH₂)ₘOC(O)[N=X] (dans lequel [N=X], dans cette invention, représente un groupe hétérocyclique de 4 à 7 chaînons avec l'atome d'azote N, qui est un membre du groupe hétérocyclique, et X représente les membres restants, nécessaires pour compléter le groupe hétérocyclique, sélectionnés parmi le groupe constitué de O, S, CH₂, CH, N, NR₉ et COR₁₀), aryle ou aryle alkyle inférieur, dans lesquels les substituants éventuels sont choisis parmi le groupe constitué par un alkyle inférieur, halo, nitro, amino, alkylamino inférieur, haloalkyle inférieur, hydroxy alkyle inférieur, alkoxy inférieur, et alkoxy inférieur alkyle inférieur ; ou R₂ et R₃ forment ensemble une chaîne à 3 ou 4 chaînons, dans laquelle les éléments de la chaîne sont sélectionnés parmi le groupe constitué de CH, CH₂, O, S, N ou NR₉;
- R₅: représente i) H, halo, halo alkyle inférieur, alkyle inférieur, alkoxy inférieur, alkoxy inférieur alkyle inférieur, alkylthio inférieur alkyle inférieur, cycloalkyle, cycloalkyle alkyle inférieur, cyano, cyano alkyle, alkyle inférieur sulphonyl alkyle inférieur, hydroxy alkyle inférieur, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇, (CH₂)ₘS(O)_{q}R₁₁, (CH₂)ₘP(O)R₁₂R₁₃, (CH₂)₂P(S)R₁₂R₁₃, ou ii) l'un des radicaux substitués (c'est-à-dire une à quatre fois sur le groupe aryle ou hétéroaryle) ou non substitués suivants : (CH₂)ₙ[N=X], OC(O)[N=X], (CH₂)ₘOC(O)[N=X], aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur et alkoxy inférieur alkyle inférieur;
- R₆ et R₇: représentent, indépendamment, i) H, un alkyle inférieur, hydroxy alkyle inférieur, alkyle inférieur amino alkyle inférieur, amino alkyle inférieur, cycloalkyle, cycloalkyle alkyle inférieur, alkényle inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, et alkoxy inférieur alkyle inférieur;
- R₈: représente i) H, un alkyle inférieur, hydroxy alkyle inférieur, amino, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, ou alkoxy inférieur alkyle inférieur;
- R₉: représente H, un alkyle inférieur, halo alkyle inférieur, aryle, ou aryle substitué par un ou plusieurs groupes choisis parmi le radical alkyle inférieur, halo, nitro, amino, alkyle inférieur amino, halo alkyle inférieur, hydroxy alkyle inférieur, alkoxy inférieur, ou alkoxy inférieur alkyle inférieur;
- R₁₀: représente H, un alkyle inférieur, halo alkyle inférieur, alkoxy inférieur, aryle, ou aryle substitué (c'est-à-dire, présentant un à quatre substituants sur le groupe aryle) par un ou plusieurs groupes choisis parmi le radical alkyle inférieur, halo alkyle inférieur, hydroxy alkyle inférieur, ou alkoxy inférieur alkyle inférieur;
- R₁₁: représente un alkyle inférieur, aryle, (CH₂)ₘOR₁₄, (CH₂)ₘSR₁₄, (CH₂)₂NR₁₄R₁₅ ou (CH₂)ₘ[N=X];
- R₁₂ et R₁₃: représentent, indépendamment, un alkyle inférieur, aryle, alkoxy inférieur, aryloxy ou amino;
- R₁₄ et R₁₅: représentent, indépendamment, H, un alkyle inférieur ou aryle;
- R₁₈ et R₁₉: représentent, indépendamment, H, halo, alkyl inférieur, alkoxy inférieur ou hydroxy;
- R₂₀: représente H ou halo;
- m: est un nombre entier compris entre 0 et 6;
- n: est 1 ou 2; et
- q: représente un nombre entier de 0 à 2; et [N=X] représente un groupe hétérocyclique de 4 à 7 chaînons, X représentant la chaîne nécessaire pour compléter ledit groupe hétérocyclique et sélectionnée dans le groupe constitué de O, S, CH₂, CH, N, NR₉ et COR₁₀;
et les sels pharmaceutiquement acceptables de ces derniers.

De préférence le composé de formule générale **(III)** est tel que R₂ représente H ou halo ; R₃ représente H, un alkyle inférieur ou halo ; R₄ représente H ou halo ; R₅ représente H, un alkyle inférieur ou un groupe (CH₂)ₙ[N=X] substitué ou non substitué dans lequel le substituant éventuel est un alkyle inférieur ; ou un sel pharmaceutiquement acceptable de ce dernier.

Les composés de formule générale **(III)** ou leurs sels pharmaceutiquement acceptables seront plus particulièrement choisis parmi le diflomotécan et la (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione et ses sels pharmaceutiquement acceptables (en particulier son chlorhydrate aussi connu sous le nom de BN-80927).

Parmi les cancers destinés à être traités par un produit selon l'invention, on peut notamment citer le cancer du sein, les lymphomes, les cancers du cou et de la tête, le cancer du poumon, le cancer du colon, le cancer de la prostate et le cancer du pancréas.

L'invention a aussi pour objet une méthode de traitement du cancer, ladite méthode comprenant l'administration d'une dose thérapeutiquement efficace d'un produit selon l'invention au patient ayant besoin de ce traitement.

Les compositions pharmaceutiques contenant un produit de l'invention peuvent se présenter sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé dé l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour un médicament selon l'invention est comprise entre 0,1 mg à 10 g suivant le type de composé actif utilisé.

Conformément à l'invention, on peut préparer les composés inclus dans des produits selon l'invention par exemple par les procédés décrits ci-après.

### PRÉPARATION DE CERTAINES COMPOSÉS INCLUS DANS DES PRODUIS SELON L'INVENTION

### Préparation des composés de formule générale (I)

Les procédés de préparation ci-après sont donnés à titre illustratif et l'homme du métier pourra leur faire subir les variations qu'il juge utiles, aussi bien en ce qui concerne les réactifs que les conditions et techniques des réactions.

### Méthode générale

D'une façon générale, les composés de formule générale **(I)** peuvent être préparés selon la procédure résumée dans le schéma 1 ci-après.

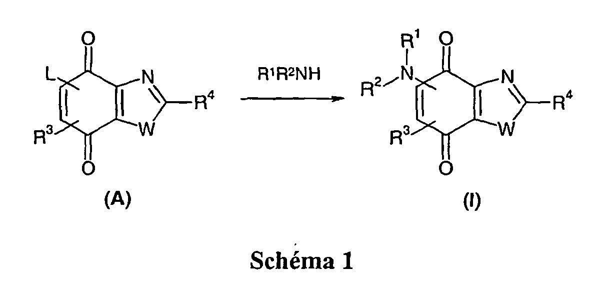

Selon cette méthode, les composés de formule générale **(I),** dans laquelle R¹, R², R³, R⁴ et W sont tels que décrits ci-dessus, sont obtenus par traitement des composés de formule générale **(A),** dans laquelle L représente un radical méthoxy, un atome halogène ou un atome d'hydrogène et R³, R⁴ et W ont la même signification que dans la formule générale **(I),** avec des amines de formule générale NR¹R²H dans un solvant protique tel que le méthanol ou l'éthanol, à une température comprise entre 0 °C et 50 °C et éventuellement en présence d'une base telle que, par exemple, la diisopropyléthylamine (Yasuyuki Kita et coll., J. Org. Chem. (1996), 61, 223-227).

Dans le cas particulier où les composés de formule générale **(A)** sont tels que L et R³ représentent chacun un atome halogène, les composés de formule générale **(I)** peuvent être obtenus sous la forme d'un mélange des 2 isomères de position, mais il est alors possible de les séparer par chromatographie sur colonne de silice dans un éluant approprié.

Alternativement, les composés de formule générale **(I)** dans lesquels R³ représente un atome halogène (Hal) peuvent être obtenus, schéma 1*bis*, à partir des composés de formule générale **(I)** dans lesquels R³ représente un atome d'hydrogène, par exemple par action de N-chlorosuccinimide ou N-bromosuccinimide dans un solvant aprotique tel que le dichlorométhane ou le tétrahydrofuranne (Paquette et Farley, J. Org. Chem. (1967), 32, 2725-2731), par action d'une solution aqueuse d'hypochlorite de sodium (eau de Javel) dans un solvant tel que l'acide acétique (Jagadeesh et coll., Synth Commun. (1998), 28, 3827-3833), par action de Cu(II) (dans un mélange CuCl₂/HgCl₂) en présence d'une quantité catalytique d'iode dans un solvant tel que l'acide acétique à chaud (Thapliyal, Synth. Commun. (1998), 28, 1123-1126), par action d'un agent tel que le dichloroiodate de benzyltriméthylammonium en présence de NaHCO₃ dans un solvant tel qu'un mélange dichlorométhane / méthanol (Kordik et Reitz, J. Org. Chem. (1996), 61, 5644-5645), ou encore par utilisation de chlore, de brome ou d'iode dans un solvant tel que le dichlorométhane (J. Renault, S. Giorgi-Renault et coll., J. Med. Chem. (1983), 26, 1715-1719).

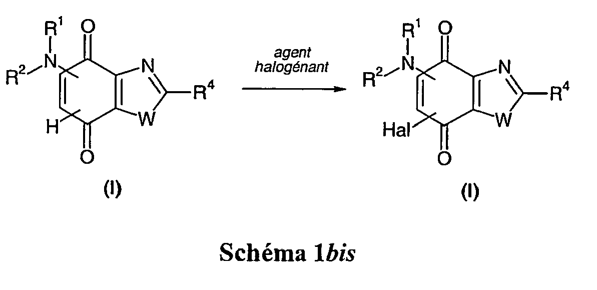

Alternativement également, les composés de formule générale **(I)** dans lesquels R³ représente un radical alkoxy ou alkylthio peuvent être obtenus, schéma 1*ter*, à partir des composés de formule générale **(I)** dans lesquels R³ représente un atome halogène, par exemple par action d'un alcool de formule générale R^{3'}-OH ou d'un thiol de formule générale R^{3'}-SH (R^{3'} étant tel que R³ = R^{3'}O ou R^{3'}S) dans un solvant tel que l'éthanol anhydre en présence d'une base telle que, par exemple, la diisopropyléthylamine.

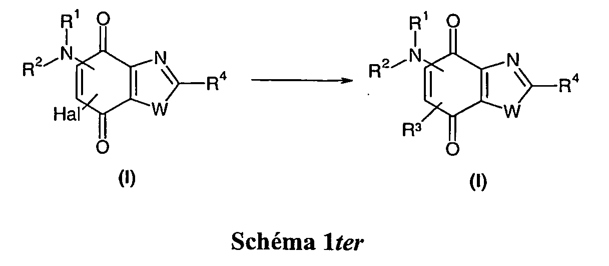

### Préparation des intermédiaires de formule générale (A)

Les composés de formule générale **(A)** dans lesquels L, R³, R⁴ et W sont tels que définis ci-dessus peuvent être obtenus, schéma 2, à partir des composés de formule générale **(B)** dans lesquels L, R³, R⁴ et W sont tels que définis ci-dessus et :
- l'un de Q et Q' représente un radical amino ou hydroxyle et l'autre représente un atome d'hydrogène ; ou
- Q et Q' représentent chacun un radical amino ; ou
- Q et Q' représentent chacun un radical hydroxyle ; ou enfin
- Q et Q' représentent chacun un radical méthoxy.

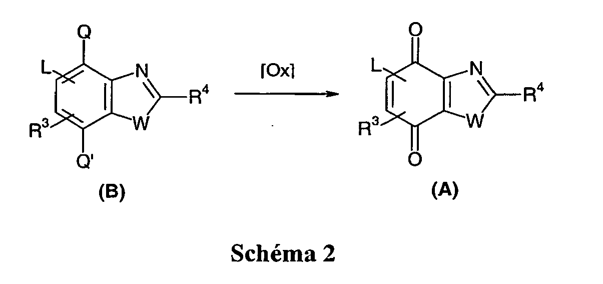

Dans le cas où les composés de formule générale **(B)** sont tels que Q et Q' représentent des radicaux méthoxy, les composés de formule générale **(A)** sont obtenus par traitement par du nitrate de cérium(IV) et d'ammonium (Beneteau et coll., Eur. J. Med. Chem. (1999), 34(12), 1053-1060). Dans les autres cas, les composés de formule générale **(A)** sont obtenus par oxydation des composés de formule générale **(B),** par exemple par utilisation de FeCl₃ en milieu acide (Antonini et coll., Heterocycles (1982), 19(12), 2313-2317) ou de sel de Fremy (nitrosodisulfonate de potassium). (Ryu et coll., Bioorg. Med. Chem. Lett. (2000), 10, 461-464), ou par l'utilisation d'un réactif comportant un iode hypervalent tel que le [bis(acétoxy)iodo]benzène ou le [bis(trifluoroacétoxy)iodo]benzène dans l'acétonitrile aqueux à une température de préférence comprise entre -20 °C et la température ambiante (soit environ 25 °C), et de préférence à environ -5 °C (Kinugawa et coll., Synthesis, (1996), 5, 633-636).

Dans le cas particulier où L et R³ représentent des atomes halogènes, les composés de formule générale **(A)** peuvent être obtenus, schéma 3, par halooxydation des composés de formule générale **(B)** dans lesquels L et R³ représentent des atomes d'hydrogène et Q et/ou Q' est (sont) choisi(s) parmi un radical amino et un radical hydroxy par action, par exemple, de perchlorate de potassium ou de sodium en milieu acide (Ryu et coll., Bioorg. Med. Chem. Lett. (1999), 9, 1075-1080).

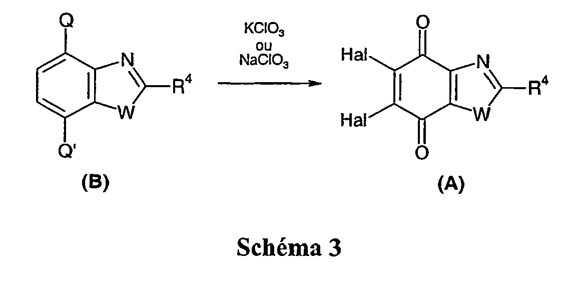

### Préparation des intermédiaires de formule générale (B)

Certains des composés de formule générale **(B)** dans lesquels L, R³, R⁴, Q, Q' et W sont tels que définis ci-dessus sont des produits industriels connus disponibles chez les fournisseurs usuels.

S'ils ne sont pas commerciaux et dans le cas particulier où Q ou Q' représente un radical amino, les composés de formule générale **(B)** peuvent notamment être obtenus à partir des dérivés nitro de formule **(B.ii)** dans lesquels Q ou Q' représente un radical nitro par des méthodes de réduction bien connues de l'homme de l'art telles que, par exemple l'hydrogénation en présence d'un catalyseur palladié ou le traitement par du chlorure d'étain dans l'acide chlorhydrique. S'ils ne sont pas commerciaux, les composés de formule **(B.ii)** peuvent eux-mêmes être obtenus à partir des composés de formule générale **(B.i)** dans lesquels les positions correspondant aux radicaux Q et Q' sont substituées par des atomes d'hydrogène par des méthodes de nitration bien connues de l'homme de l'art telles que, par exemple, le traitement par un mélange d'acide nitrique et d'acide sulfurique (cf. le schéma 4 où seul le cas dans lequel les composés de formule générale **(B)** sont tels que Q = NH₂ et Q' = H est représenté).

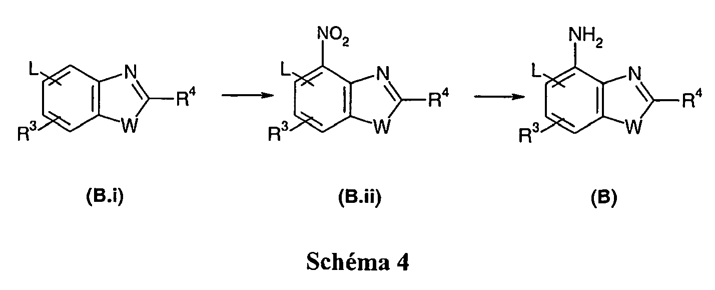

Alternativement, les composés de formule générale **(B)** non commerciaux dans lesquels Q représente un radical amino, Q' un atome d'hydrogène et W un atome d'oxygène, peuvent être obtenus par traitement des tétrahydrobenzoxazoles de formule générale **(B.vi)** par le chlorhydrate d'hydroxylamine pour donner les oximes de formule générale **(B.v),** eux-mêmes traités par de l'acide polyphosphorique à chaud (cf. Young Kook Koh et coll., J. Heterocyclic Chem. (2001), 38, 89-92) pour fournir les composés de formule générale **(B).** Les composés de formule générale **(B.vi)** peuvent eux-mêmes être obtenus à partir des 1,3-dicétones cycliques de formule générale **(B.viii)** tout d'abord par conversion en diazodicétones de formule générale **(B.vii)** par réaction de diazotransfert, par exemple, par action de tosyl azide ou de 4-acétamidobenzènesulfonylazide en présence de triéthylamine dans un solvant tel que le dichlorométhane ou le chloroforme anhydres (V. V. Popic et coll., Synthesis (1991), 3, 195-198) suivie d'une cycloaddition de ces diazodicétones de formule générale **(B.vii)** par des nitriles de formule générale R⁴-CN en présence d'un catalyseur de type Rhodium (II) (Y. R. Lee, Heterocycles (1998), 48, 875-883) (cf. schéma 4*bis*).

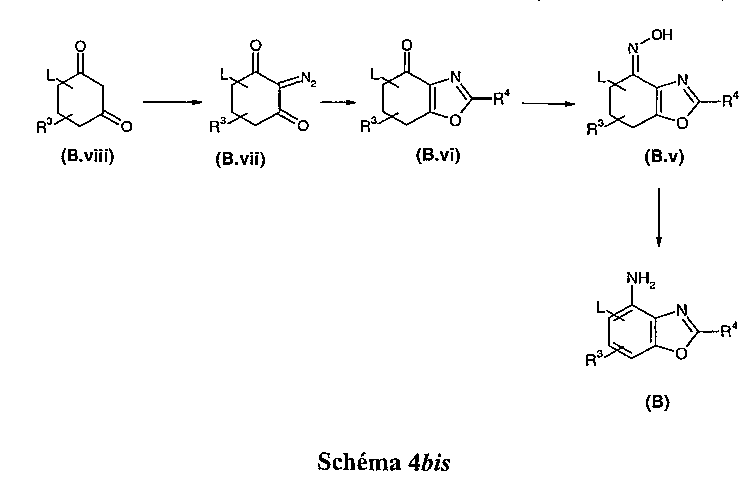

S'ils ne sont pas commerciaux et dans le cas particulier où Q représente hydroxyle, Q' un atome d'hydrogène et W un atome d'oxygène, les composés de formule générale **(B)** peuvent être obtenus par aromatisation des oxazolocyclohexanones de formule générale **(B.vi).** Une tell aromatisation peut être effectuée en deux étapes comme montré dans le Schéma 4*ter*, d'abord une halogénation en position α du carbonyle (qui conduit aux intermédiaires de formule générale **(B.ix)** dans laquelle Hal est un atome halogène), puis β-élimination de l'halogène par traitement avec une base. L'halogénation peut être faite, par exemple, à l'aide de brome dans de l'acide acétique à température ambiante, de tribromure pyridinium dans de l'acide acétique à 50 °C, de bromure de cuivre (II) dans de l'acétate d'éthyle ou de l'acétonitrile au reflux, ou aussi de chlorure de phenylsélényle dans de l'acétate d'éthyle à température ambiante. L'élimination de l'halogénure résultant peut être effectuée par du diazabicyclo[5.4.0]undec-7-ène (DBU) dans du tétrahydrofuranne à température ambiante ou par du carbonate de lithium dans du diméthylformamide. Des exemples de ces réactions sont fournis par M. Tany et coll., Chem. Pharm. Bull. (1996), 44, 55-61; M.A. Ciufolini et coll., J. Am. Chem. Soc. (1995), 117, 12460-12469; et M.E. Jung et L.S. Starkey, Tetrahedron (1997), 53, 8815-8824.

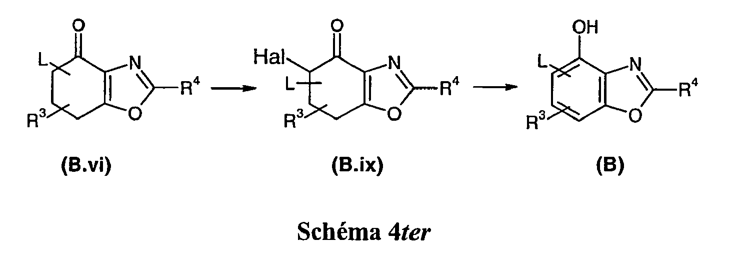

S'ils ne sont pas commerciaux et dans le cas particulier où R⁴ représente un radical -CH₂-NR²¹R²², les composés de formule générale **(B)** peuvent être obtenus, schéma 5, à partir des composés de formule générale **(B.iii)** dans lesquels R⁴ représente le radical méthyle, que l'on soumet d'abord à une réaction de bromation radicalaire à l'aide de *N*-bromosuccinimide en présence d'un initiateur tel que le 2,2'-azobis(2-méthylpropionitrile) ou le dibenzoylperoxyde dans un solvant aprotique tel que le tétrachlorure de carbone (CCl₄) à une température de préférence comprise entre la température ambiante (i.e. environ 25 °C) et 80 °C et sous irradiation par une lampe UV (Mylari et coll., J. Med. Chem. (1991), 34, 108-122), suivie d'une substitution de l'intermédiaire de formule générale **(B.iv)** par des amines de formule HNR²¹R²² avec R²¹ et R²² sont tels que définis ci-dessus.

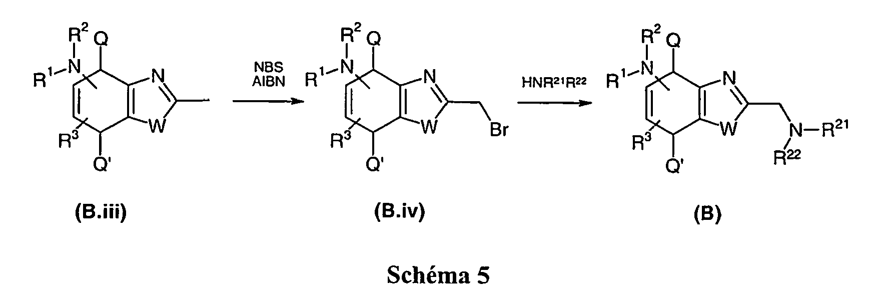

Alternativement, les composés de formule générale **(B)** non commerciaux dans lesquels R⁴ représente un radical -CH₂-NR²¹R²² peuvent être obtenus selon la méthode représentée dans le schéma 4 plus haut, à partir des composés de formule générale **(B.i)** dans laquelle R⁴ représente un radical-CH₂-NR²¹R²², ceux-ci étant eux-mêmes obtenus à partir des composés de formule générale **(B.i)** dans laquelle R⁴ représente un radical CH₂-Br par substitution par des amines de formule HNR²¹R²² avec R²¹ et R²² tels que définis ci-dessus. Les composés de formule générale **(B.i)** dans laquelle R⁴ représente un radical CH₂-Br peuvent être obtenus, comme décrit ci-dessus, à partir des composés de formule générale (**B.i)** dans lesquels R⁴ représente le radical méthyle, que l'on soumet à une réaction de bromation radicalaire.

S'ils ne sont pas commerciaux et dans le cas particulier où R⁴ représente un radical -CH₂-CO-NR¹⁹R²⁰, les composés de formule générale **(B)** peuvent être obtenus à partir des composés de formule générale **(B)** dans lesquels R⁴ représente le radical -CH₂-COOH, par les méthodes classiques de la synthèse peptidique (M. Bodansky, The Practice of Peptide Synthesis, 145 (Springer-Verlag, 1984)), par exemple dans le tétrahydrofuranne, le dichlorométhane ou le diméthylformamide en présence d'un réactif de couplage tel que le cyclohexylcarbodiimide (DCC), le 1,1'-carbonyldiimidazole (CDI) *(*J. Med. Chem. (1992), 35(23), 4464-4472) ou l'hexafluorophosphate de benzotriazol-1-yl-oxy-tris-pyrrolidino-phosphonium (PyBOP) (Coste et coll., Tetrahedron Lett. (1990), 31, 205).

Les composés de formule générale **(B)** dans lesquels R⁴ représente -CH₂-COOH peuvent être obtenus à partir des composés de formule générale **(B)** dans lesquels R⁴ représente le radical -CH₂COOR¹⁸ dans lequel R¹⁸ représente un radical alkyle par hydrolyse de la fonction ester dans des conditions connues de l'homme du métier.

Les composés de formule générale **(B)** dans lesquels W représente S, Q et Q' représentent chacun un radical méthoxy et L représente un atome halogène ou un atome d'hydrogène peuvent être obtenus, schéma 6, par traitement des N-(2,5-diméthoxyphényl)thioamides de formule générale **(B.x)** par une solution aqueuse de ferricyanure de potassium en milieu sodique à température ambiante (Lyon et coll., J. Chem. Soc., Perkin Trans. 1 (1999), 437-442). Les composés de formule générale **(B.x)** peuvent eux-mêmes être obtenus en partant des 2,5-diméthoxyanilines acylées correspondantes de formule générale **(B.xii)**, par exemple par action d'un chlorure d'acide de formule générale R⁴COCl ou d'un acide carboxylique de formule générale R⁴COOH activé selon des méthodes connues de l'homme du métier, pour donner les N-(2,5-dimethoxyphenyl)amides de formule générale **(B.xi)** eux-mêmes convertis en les thioamides de formule générale **(B.x)** par action de réactif de Lawesson dans du toluène au reflux.

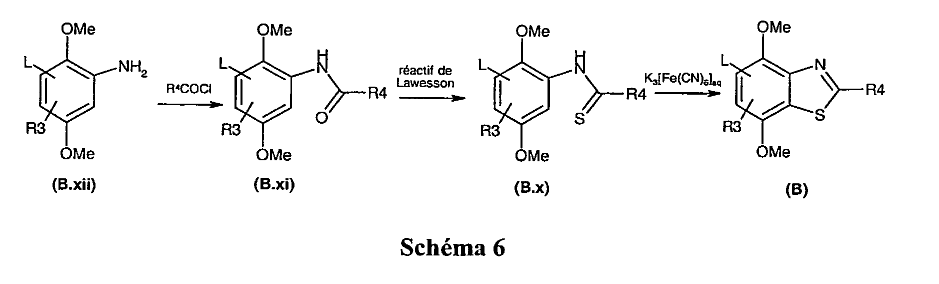

Dans les autres cas, les composés de formule générale **(B)** peuvent être obtenus, schéma 6*bis*, à partir des composés de formule générale **(C)** dans lesquels L, R³ et W sont tels que définis ci-dessus et Q ou Q' représente le radical NO₂ par condensation avec l'orthoester de formule générale R⁴C(OR)₃ dans laquelle R est un radical alkyle, par exemple en présence d'une quantité catalytique d'un acide tel que, par exemple, l'acide paratoluènesulfonique, à une température comprise entre la température ambiante et 200°C et de préférence à environ 110 °C (Jenkins et coll., J. Org. Chem. (1961), 26, 274) ou encore dans un solvant protique tel que l'éthanol à une température comprise entre la température ambiante (i.e. environ 25 °C) et 80 °C et de préférence à environ 60 °C (Scott et coll., Synth. Commun. (1989), 19, 2921). Un certain nombre d'orthoesters sont des produits industriels connus disponibles chez les fournisseurs usuels. La préparation d'orthoesters en traitant des composés nitriles variés par du gaz chlorhydrique dans un alcool est connue de l'homme du métier.

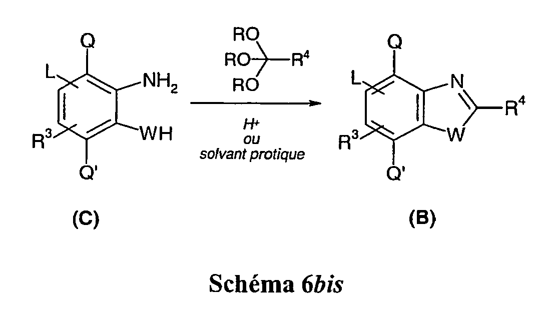

Les composés de formule générale **(B)** dans lesquels L, R³, R⁴ et W sont tels que définis ci-dessus et Q ou Q' représente le radical NO₂ peuvent aussi être obtenus à partir des composés de formule générale **(C)** dans lesquels L, R³, R⁴ et W sont tels que définis ci-dessus et l'un de Q et Q' représente le radical NO₂ tandis que l'autre représente un atome d'hydrogène par condensation de ces derniers avec un chlorure d'acide de formule R⁴-COCl sous atmosphère inerte et dans un solvant polaire et légèrement basique tel que la N-méthyl-2-pyrrolidinone (Brembilla et coll., Synth. Commun (1990), 20, 3379-3384) ou par condensation de ces derniers avec un acide carboxylique de formule générale R⁴-COOH en présence d'acide polyphosphorique à haute température (Ying-Hung So et coll., Synth. Commun. (1998), 28, 4123-4135) ou en présence d'acide borique dans un solvant tel que le xylène au reflux (M. Terashima, Synthesis (1982), 6, 484-485).

Les composés de formule générale **(B)** dans lesquels L, R³, R⁴ et W sont tels que définis ci-dessus et Q ou Q' représente le radical NO₂ peuvent également être obtenus à partir des composés de formule générale **(C)** dans lesquels L, R³, R⁴ et W sont tels que définis ci-dessus et l'un de Q et Q' représente le radical NO₂ tandis que l'autre représente un atome d'hydrogène par condensation avec un aldéhyde de formule générale R⁴-CHO puis traitement de la base de Schiff obtenue par un agent oxydant tel que le [bis(acétoxy)iodo]benzène, le chlorure ferrique ou le diméthylsulfoxyde (Racane et coll., Monatsh. Chem. (1995), 126(12), 1375-1381) ou par déshydratation par l'acide acétique glacial à une température comprise entre la température ambiante (i.e. environ 25 °C) et 100 °C (Katritzky et Fan, J. Heterocyclic Chem. (1988), 25, 901-906).

Les composés de formule générale **(B)** dans lesquels L, R³, R⁴ et W sont tels que définis ci-dessus et l'un de Q et Q' représente le radical NO₂ tandis que l'autre représente un atome d'hydrogène peuvent encore être obtenus à partir des composés de formule générale **(C)** par condensation avec un nitrile de formule générale R⁴-CN dans un mélange de solvants du type méthanol / acide acétique glacial à une température comprise entre la température ambiante (i.e. environ 25 °C) et 100 °C (Nawwar et Shafik, Collect. Czech Client. Commun. (1995), 60(12), 2200-2208).

### Préparation_des intermédiaires_de formule_générale_(C)

Certains des composés de formule générale **(C)** dans lesquels L, R³, Q, Q' et W sont tels que définis ci-dessus sont des produits industriels connus disponibles chez les fournisseurs usuels.

Certains composés de formule générale **(C)** dans lesquels l'un de Q et Q' représente le radical NO₂ tandis que l'autre représente un atome d'hydrogène peuvent être obtenus à partir des composés de formule générale **(D)** 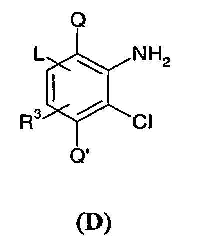 dans lesquels L, R³, Q et Q' sont tels que définis ci-dessus par réaction, dans le cas où W représente S, avec du sulfure de sodium hydraté à une température comprise entre la température ambiante (i.e. environ 25 °C) et 100 °C (Katritzky et Fan, J. Heterocyclic Chem. (1988), 25, 901-906).

Enfin, dans le cas particulier où W représente O, les composés de formule générale **(C)** sont des produits industriels connus disponibles chez les fournisseurs usuels ou peuvent être synthétisés à partir de tels produits selon des méthodes courantes pour l'homme du métier.

### Séparation_de_mélanges de régioisomères

Dans certains cas, il peut arriver que les composés de formule générale **(I)** préparés selon les métyhodes susmentionnées soient obtenus sous forme de mélanges de régioisomères.

Dans de telles situations, le mélange peut être séparé grâce à des techniques standard de chromatographie liquide sur colonne ou sur couche mince préparative (en utilisant un support tel que de la silice ou encore un gel comme un gel de polydextranes réticulés formant un réseau tridimensionnel comme un gel de type Sephadex^{®} LH-20). L'homme du métier choisira l'éluant le mieux adapté à la séparation du mélange; un tel éluant pourra être par exemple un mélange ternaire isopropanol/acétate d'éthyle/eau 1/1/1.

### Préparation des composés de formule générale (III)

Les composés de formule générale **(III)** ont notamment été décrits dans la demande de brevet PCT WO 97/00876.

En ce qui concerne les températures auxquelles il est fait référence dans le présent texte, le terme « environ *XX* °C » indique que la température en question correspond à un intervalle de, plus ou moins 10 °C autour de la température de *XX* °C, et de préférence à un intervalle de plus ou moins 5 °C autour de la température de *XX* °C. En ce qui concerne les autres valeurs numériques auxquelles il est fait référence dans le présent texte, le terme « environ *YY* » indique que la valeur en question correspond à un intervalle de plus ou moins 10% autour de la valeur *YY*, et de préférence à un intervalle de plus ou moins 5% autour de la valeur *YY*.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES DE COMPOSÉS DE FORMULE GÉNÉRALE (I)

### Méthode employée_pour_la_mesure_du_temps_de_rétention_(t.r.) et_du_pic moléculaire (MH+)

Les composés sont caractérisés par leur temps de rétention (t.r.), exprimé en minutes, déterminé par chromatographie liquide (CL), et leur pic moléculaire (MH+) déterminé par spectrométrie de masse (SM), un spectromètre de masse simple quadripôle (Micromass, modèle Platform) équipé d'une source électrospray est utilisé avec une résolution de 0,8 da à 50% de vallée.
Pour les exemples 1 à 138 ci-après, les conditions d'élution correspondant aux résultats indiqués sont les suivantes: passage d'un mélange acétonitrile-eau-acide trifluoroacétique 50-950-0,2 (A) à un mélange acétonitrile-eau 950-50 (B) par un gradient linéaire sur une période de 8,5 minutes, puis élution avec le mélange B pur pendant 10,5 minutes.

### Exemple 1 : 2-méthyl-5-{[2-(4-morpholinyl)éthyl]amino}-1,3-benzothiazole-4,7-dione:

51,2 µl (0,39 mmol ; 3 équivalents) de 4-(2-aminoéthyl)morpholine sont ajoutés à 27 mg (0,129 mmol) de 5-méthoxy-2-méthyl-4,7-dioxobenzothiazole en solution dans 2 ml d'éthanol anhydre. Le mélange réactionnel est agité au reflux pendant 18 heures puis le solvant est évaporé sous pression réduite. Le résidu est purifié sur colonne de silice (éluant : méthanol à 5% dans du dichlorométhane). Le composé attendu est obtenu sous forme d'une poudre rouge.
RMN ¹H (DMSO d6, 400 MHz, δ): 7,45 (t, 1H, NH); 5,49 (s, 1H, CH); 3,58-3,55 (m, 4H, 2 CH₂) ; 3,26 (t, 2H, CH₂) ; 2,75 (s, 3H, CH₃) ; 2,54 (t, 2H, CH₂) ; 2,42-2,40 (m, 4H, 2 CH₂).
SM-CL : MH+ = 308,25 ; t.r. = 6,89 min.

### Exemple 2 : chlorhydrate de 5-{[2-(diméthylamino)éthyl]amino]-2-méthyl-1,3-benzothiazole-4,7-dione :

### 2.1) 5-{[2-(diméthylamino)éthyl]amino}-2-méthyl-1,3-benzothiazole-4,7-dione:

Ce composé est obtenu de façon analogue à celle employée pour le composé de l'exemple 1.
RMN ¹H (DMSO d6, 400 MHz, δ) : 7,34 (t, 1H, NH) ; 5,48 (s, 1H, CH); 3,24-3,20 (m, H, CH₂) ; 2,77 (s, 3H, CH₃) ; 2,47 (m, 2H, CH₂) ; 2,18 (s, 6H, 2 CH₃).
SM-CL : MH+ = 266,27 ; t.r. = 6,83 min.

### 2.2) chlorhydrate de 5-{[2-(diméthylamino)éthyl]amino}-2-méthyl-1,3-benzothiazole-4,7-dione :

0,166 g de l'intermédiaire 2.1 sont dissous dans 1,88 ml (1,88 mmol; 3 éq.) d'une solution molaire d'acide chlorhydrique dans de l'éther et le mélange réactionnel est agité pendant 3 heures à température ambiante. Le précipité résultant est recueilli par filtration, lavé avec de l'éther éthylique et séché sous pression réduite pour donner un solide rouge foncé. Point de fusion : 138-140 °C.
RMN ¹H (DMSO d6, 400 MHz, δ) : 10,00 (s, 1H, NH⁺); 7,78 (t, 1H, NH) ; 5,68 (s, 1H, CH); 3,59-3,55 (m, 2H, CH₂); 3,32-3,27 (m, 2H, CH₂) ; 2,85-2,80 (s, 6H, 2 CH₃) ; 2,76 (s, 3H, CH₃).
SM-CL : MH+ = 266,12 ; t.r. = 6,92 min.

### Les composés des exemples 3 à 14 sont obtenus de façon analogue à celle employée pour l'exemple 1.

### Exemple 3 : 5-{[6-(diméthylamino)hexyl]amino}-2-méthyl-1,3-benzothiazole-4,7-dione :

SM-CL : MH+ = 322,33 ; t.r. = 7,36 min.

### Exemple 4 : 5-{[3-(diméthylamino)-2,2-diméthylpropyl]amino}-2-méthyl-1,3-benzothiazole-4,7-dione :

RMN ¹H (DMSO d6, 400 MHz, δ): 8,62 (t, 1H, NH); 5,45 (s, 1H, CH); 3,07-3,06 (m, 2H, CH₂) ; 2,74 (s, 3H, CH₃) ; 2,29-2,30 (m, 2H, CH₂); 2,27 (s, 6H, 2CH₃) ; 0,93 (s, 6H, 2 CH₃).
LC-MS : MH+ = 308,32 ; t.r. = 7,16 min.

### Exemple 5 : 2-méthyl-5-{[3-(4-méthyl-1-pipérazinyl)propyl]amino}-1,3-benzothiazole-4,7-dione :

RMN ¹H (DMSO d6, 400 MHz, δ): 8,14 (t, 1H, NH); 5,46 (s, 1H, CH); 3,25-3,26 (m, 2H, CH₂); 3,21-3,19 (m, 2H, CH₂); 2,74 (s, 3H, CH₃) ; 2,49-2,48 (m, 2H, CH₂) ; 2,37-2,32 (m, 6H, 3CH₂); 2,16 (s, 3H, CH₃) ; 1,72 (t, 2H, CH₂).
SM-CL : MH+ = 335,34 ; t.r. = 6,87 min.

### Exemple 6 : 5-[(1-éthylhexyl)amino]-2-méthyl-1,3-benzothiazole-4,7-dione :

SM-CL : MH+ = 307,32 ; t.r. = 11,45 min.

### Exemple 7 : 5-[(1-adamantylméthyl)amino]-2-méthyl-1,3-benzothiazole-4,7-dione :

SM-CL : MH+ = 343,31 ; t.r. = 11,73 min.

### Exemple 8 : 2-méthyl-5-[(2-thiénylmèthyl)amino]-1,3-benzothiazole-4,7-dione :

SM-CL : MH+ = 291,16 ; t.r. = 9,24 min.

### Exemple 9 : 5-[(3-chlorobenzyl)amino]-2-méthyl-1,3-benzothiazole-4,7-dione :

SM-CL : MH+ = 319,24 ; t.r. = 9,95 min.

### Exemple 10 : 2-méthyl-5-[(4-pyridinylméthyl)amino]-1,3-benzothiazole-4,7-dione :

SM-CL : MH+ = 286,13 ; t.r. = 6,97 min.

### Exemple 11 : 2-méthyl-5-(propylamino)-1,3-benzothiazole-4,7-dione :

SM-CL : MH+ = 237,16 ; t.r. = 8,74 min.

### Exemple 12 : 5-{[3-(1H-imidazol-1-yl)propyl]amino}-2-méthyl-1,3-benzothiazole-4,7-dione :

SM-CL : MH+ = 303,17 ; t.r. = 7,07 min.

### Exemple 13 : 4-{2-[(2-méthyl-4,7-dioxo-4,7-dihydro-1,3-benzothiazol-5-yl)amino]éthyl} benzènesulfonamide :

SM-CL : MH+ = 378,10 ; t.r. = 8,31 min.

### Exemple 14 : 5-(4-benzyl-1-pipérazinyl)-2-méthyl-1,3-benzothiazole-4,7-dione :

SM-CL : MH+ = 354,19 ; t.r. = 7,53 min.

### Exemple 15 : 5-anilino-2-éthyl-1,3-benzoxazole-4,7-dione ou 6-anilino-2-éthyl-1,3-benzoxazole-4,7-dione :

### 15.1) 2-éthyl-4-nitro-1,3-benzoxazole :

Un mélange de 2-amino-3-nitrophénol (1 éq.), de triéthyl orthopropionate (2 éq.) et d'acide *p*-toluènesulfonique (en quantité catalytique) est agité à 110 °C jusqu'à disparition de l'aminophénol vérifiée par chromatographie sur couche mince (2 h). Après refroidissement, le mélange réactionnel est repris au toluène et évaporé sous vide puis traité à l'isopropanol. Le précipité résultant est recueilli par filtration, lavé avec de l'isopropanol et de l'isopentane, puis séché sous pression réduite pour donner un solide brun-violet.
RMN ¹H (DMSO d6, 400 MHz, δ): 8,15 (dd, 2H) ; 7,58 (t, 1H) ; 3,06 (q, 2H) ; 1,38 (t, 3H).
SM-CL : MH+ = 193,02 ; t.r. = 9,23 min.

### 15.2) 2-éthyl-1,3-benzoxazol-4-amine :

Le 2-éthyl-4-nitro-1,3-benzoxazole est hydrogéné sous une pression de 8 bars en présence de charbon palladié à 10% (0,01 éq.) en employant du méthanol comme solvant. Le catalyseur est séparé par filtration et le méthanol est éliminé sous pression réduite. Le résidu est repris dans l'éther éthylique pour donner un solide violet-pâle qui est recueilli par filtration et séché. Point de fusion : 46 °C.
RMN ¹H (DMSO d6, 400 MHz, δ): 6,97 (t, 1H) ; 6,72 (d, 1H); 6,47, d, 1H); 5,45 (s, 2H) ; 2,87 (q, 2H) ; 1,32 (t, 3H).
SM-CL : MH+ = 162,99 ; t.r. = 8,72 min.

### 15.3) 2-éthyl-1,3-benzoxazole-4,7-dione :

Une solution de [bis(trifluoroacétoxy)iodo]benzène (2,2 éq.) dans un mélange d'acétonitrile et d'eau (80/20) est ajouté au goutte-à-goutte à une solution de 2-éthyl-1,3-benzoxazol-4-amine (1 éq.) dans un même mélange acétonitrile/eau maintenue à -5 °C. Le milieu réactionnel est ensuite dilué avec de l'eau et extrait au dichlorométhane. La phase organique résultante est lavée avec de l'eau, séchée sur sulfate de sodium et concentrée pour donner une pâte brune. Une purification par chromatographie à moyenne pression sur gel de silice donne, après reprise dans de l'éther diisopropylique, un solide cristallin jaune. Point de fusion : 99 °C.
RMN ¹H (CDCl₃, 400 MHz, δ): 6,75 (dd, 2H) ; 2,99 (q, 2H) ; 1,45 (t, 3H).
SM-CL : MH+ = 177,83 ; t.r. = 8,29 min.

### 15.4) 5-anilino-2-éthyl-1,3-benzoxazole-4,7-dione ou 6-anilino-2-éthyl-1,3-benzoxazole-4,7-dione :

Un mélange de 2-éthyl-1,3-benzoxazole-4,7-dione (1 éq) et d'aniline (1,1 éq.) dans de l'éthanol est maintenu sous agitation pendant 1 heure. Le milieu réactionnel tourne au violet foncé. Après concentration, le résidu est purifié par chromatographie à moyenne pression sur silice pour donner une poudre violette. Point de fusion : 200 °C.
RMN ¹H (DMSO d6, 400 MHz, δ) : 9,38 (s, 1H) ; 7,44 (t, 2H) ; 7,36 (d, 2H) ; 7,22 (t, 1H) ; 5,69 (s ; 1H) ; 2,94 (q, 2H) ; 1,29 (t, 3H).
SM-CL : MH+ = 269,11 ; t.r. = 9,76 min.

### Exemple 16 : 5-anilino-6-chloro-2-éthyl-1,3-benzoxazole-4,7-dione ou 6-anilino-5-ehloro-2-éthyl-1,3-benioxazole-4,7-dione :

Une solution d'un mélange de 5-anilino-2-éthyl-1,3-benzoxazole-4,7-dione et 6-anilino-2-éthyl-1,3-benzoxazole-4,7-dione (1 éq.) dans l'acide acétique est traitée par du *N*-chlorosuccinimide (1,1 éq.) à température ambiante. Le milieu réactionnel est maintenu sous agitation pendant 2 heures avant d'être concentré, repris dans de l'éthanol et concentré de nouveau. Le résidu est purifié par chromatographie à moyenne pression sur silice pour donner une poudre violette. Point de fusion : 159 °C.
RMN ¹H (DMSO d6, 400 MHz, δ) : 9,39 (s, 1H) ; 7,30 (t, 2H); 7,11 (m, 3H) ; 2,96 (q, 2H) ; 1,30 (t, 3H).
SM-CL : MH+ = 303,01 ; t.r. = 10,28 min.

### Exemple 17 : 2-éthyl-5-[(4-fluorophényl)amino]-1,3-benzoxazole-4,7-dione ou 2-éthyl-6-[(4-fluorophényl)amino]-1,3-benzoxazole-4,7-dione :

Le protocole expérimental utilisé est identique à celui décrit pour l'exemple 15, la 4-fluoroaniline remplaçant l'aniline dans la quatrième et dernière étape. Point de fusion : 232 °C.
RMN ¹H (DMSO d6, 400 MHz, δ) : 9,38 (s, 1H) ; 7,37 (t, 2H) ; 7,26 (t, 2H) ; 5,57 (s, 1H) ; 2,93 (q, 2H) ; 1,30 (t, 3H).
SM-CL : MH+ = 287,09 ; t.r. = 9,88 min.

### Les composés des exemples 18 à 31 sont obtenus de façon analogue à celle décrite pour l'exemple 1.

### Exemple 18 : 5-[(2-méthoxyéthyl)amino]-2-méthyl-1,3-benzothiazole-4,7-dione :

SM-CL : MH+ = 253,20 ; t.r. = 8,00 min.

### Exemple 19 : 2-méthyl-5-[(2-pyrrolidin-1-yléthyl)amino]-1,3-benzothiazole-4,7-dione :

RMN ¹H (DMSO d6, 400 MHz, δ) : 7,45 (m, 1H, NH); 5,47 (s, 1H, CH) ; 3,28-3,23 (m, 2H, CH₂) ; 2,75 (s, 3H, CH₃) ; 2,66-2,63 (m, 2H, CH₂) ; 2,48-2,49 (m, 4H, 2CH₂) ; 1,68-1,67 (m, 4H, 2CH₂).
SM-CL : MH+ = 292,13 ; t.r. = 7,11 min.

### Exemple 20 : 2-méthyl-5-[(2-pipéridin-1-yléthyl)amino]-1,3-benzothiazole-4,7-dione :

SM-CL : MH+ = 306,24 ; t.r. = 7,22 min.

### Exemple 21 : 5-{[2-(diisopropylamino)éthyl]amino}-2-méthyl-1,3-benzothiazole-4,7-dione :

SM-CL : MH+ = 322,33 ; t.r. = 7,37 min.

### Exemple 22 : 5-[(1-benzylpyrrolidin-3-yl)amino]-2-méthyl-1,3-benzothiazole-4,7-dione :

SM-CL : MH+ = 354,28 ; t.r. = 7,70 min.

### Exemple 23 : 5-{[3-(diméthylamino)propyl]amino}-2-méthyl-1,3-benzothiazole-4,7-dione :

SM-CL : MH+ = 280,15 ; t.r. = 7,01 min.

### Exemple 24 : 2-méthyl-5-{[2-(1-méthylpyrrolidin-2-yl)éthyl]amino}-1,3-benzothiazole-4,7-dione :

SM-CL : MH+ = 306,30 ; t.r. = 7,23 min.

### Exemple 25 : 2-méthyl-5-{[3-(2-méthylpipéridin-1-yl)propyl]amino}-1,3-benzothiazole-4,7-dione :

SM-CL : MH+ = 334,29 ; t.r. = 7,38 min.

### Exemple 26: 5-{[4-(diméthylamino)butyl]amino}-2-méthyl-1,3-benzothiazole-4,7-dione :

SM-CL : MH+ = 294,16 ; t.r. = 7,11 min.

### Exemple 27: 5-{[5-(diméthylamino)pentyl]amino}-2-méthyl-1,3-benzothiazole-4,7-dione :

SM-CL : MH+ = 308,16 ; t.r. = 7,22 min.

### Exemple 28 : 5-(2,3-dihydro-1H-indén-1-ylamino)-2-méthyl-1,3-benzothiazole-4,7-dione :

SM-CL : MH+ = 311,26 ; t.r. = 10,16 min.

### Exemple 29 : 5-{benzyl[2-(diméthylamino)éthyl]amino}-2-méthyl-1,3-benzothiazole-4,7-dione :

RMN ¹H (DMSO d6, 400 MHz, δ) : 7,37-7,28 (m, 5H, H arom.) ; 5,61 (s, 1H, CH) ; 4,57 (s, 2H, CH₂) ; 3,71-3,68 (m, 2H, CH₂) ; 2,75 (s, 3H, CH₃) ; 2,39-2,37 (m, 2H, CH₂) ; 1,95 (s, 6H, 2 CH₃).
SM-CL : MH+ = 365,10 ; t.r. = 7,70 min.

### Exemple 30 : méthyl{3-[(2-méthyl-4,7-dioxo-4,7-dihydro-1,3-benzothiazol-5-yl)amino]propyl}carbamate de tert-butyle :

RMN ¹H (DMSO d6, 400 MHz, δ): 7,75 (m, 1H, NH) ; 5,45 (s, 1H, CH); 3,22-3,18 (m, 2H, CH₂); 3,15-3,12 (m, 2H, CH₂); 2,76 (m, 3H, CH₃) ; 2,75 (s, 3H, CH₃) ; 1,78-1,75 (m, 2H, CH₂) ; 1,35 (m, 9H, 3 CH₃).
SM-CL : MH+ = 366,15 ; t.r. = 9,61 min.

### Exemple 31 : 3-[(2-méthyl-4,7-dioxo-4,7-dihydro-1,3-benzothiazol-5-yl)amino]propylcarbamate de tert-butyle :

SM-CL : MH+ = 352,22 ; t.r. = 9,09 min.

### Exemple 32 : chlorhydrate de 2-méthyl-5-{[3-(méthylamino)propyl]amino}-1,3-benzothiazole-4,7-dione :

25 mg (68,5 µmol) du composé de l'exemple 30 sont mis en suspension dans 10 ml de diéthyléther. 4 ml d'une solution molaire d'acide chlorhydrique dans de l'éther sont ajoutés puis le mélange réactionnel est agité à température ambiante pendant 2 heures. Le précipité résultant est recueilli par filtration, lavé avec de l'éther puis séché sous pression réduite pour donner un solide brun-rouge.
RMN ¹H (DMSO d6, 400 MHz, δ): 8,61 (m, 2H, NH₂⁺); 7,84-7,81 (m, 1H, NH) ; 5,55 (s, 1H, CH) ; 3,29-3,24 (m, 2H, CH₂) ; 2,91-2,88 (m, 2H, CH₂) ; 2,75 (s, 3H, CH₃) ; 2,53-2,52 (m, 3H, CH₃) ; 1,89-1,86 (m, 2H, CH₂).
SM-CL : MH+ = 266,06 ; t.r. = 7,04 min.

### Exemple 33 : 5-[(3-aminopropyl)amino]-2-méthyl-1,3-benzothiazole-4,7-dione :

20 mg (57 µmol) du composé de l'exemple 30 sont mis en suspension dans 10 ml de diéthyléther. 840 µl d'une solution molaire d'acide chlorhydrique dans de l'éther sont ajoutés puis le mélange réactionnel est agité à température ambiante pendant 2 heures. Le précipité résultant est recueilli par filtration, lavé avec de l'éther puis séché sous pression réduite pour donner un solide brun-rouge.
RMN ¹H (DMSO d6, 400 MHz, δ) : 7,84-7,78 (m, 3H, NH, NH₂) ; 5,56 (s, 1H, CH) ; 3,28-3,23 (m, 2H, CH₂) ; 2,86-2,81 (m, 2H, CH₂) ; 2,75 (s, 3H, CH₃) ; 1,85-1,82 (m, 2H, CH₂).
SM-CL : MH+ = 280,15 ; t.r. = 7,01 min.

### Exemple 34 : 6-chloro-5-{[2-(diméthylamino)éthyl]amino}-2-méthyl-1,3-benzothiazole-4,7-dione :

58,6 mg (0,22 mmol) d'intermédiaire 2.1 sont mis en solution dans 5 ml d'acide acétique. 32,5 mg (0,24 mmol ; 1,1 éq.) de *N*-chlorosuccinimide sont ajoutés et le mélange réactionnel est agité pendant 3 heures à température ambiante. Après concentration, le résidu est purifié par chromatographie sur colonne de silice (éluant : dichlorométhane/méthanol 90/10) et le produit attendu est obtenu, après reprise dans de l'éther éthylique, sous forme d'une poudre violette.
RMN ¹H (DMSO d6, 400 MHz, δ) : 7,31 (m, 1H, NH) ; 3,79-3,74 (m, 2H, CH₂) ; 2,75 (s, 3H, CH₃) ; 2,47-2,44 (m, 2H, CH₂) ; 2,13 (s, 6H, 2 CH₃).
SM-CL : MH+ = 300,09 ; t.r. = 7,17 min.

### Exemple 35 : 6-bromo-5-{[2-(diméthylamino)éthyl]amino}-2-méthyl-1,3-benzothiazole-4,7-dione :

102 mg (0,38 mmol) d'intermédiaire 2.1 sont mis en solution dans 10 ml d'acide acétique. 77,3 mg (0,43 mmol ; 1,1 éq.) de *N*-bromosuccinimide sont ajoutés et le mélange réactionnel est agité pendant 3 heures à température ambiante. Après concentration sous pression réduite, le résidu est purifié par chromatographie sur colonne de silice (éluant : dichlorométhane/méthanol 90/10) et le produit attendu est obtenu, après reprise dans de l'éther éthylique, sous forme d'une poudre violette.
RMN ¹H (DMSO d6, 400 MHz, δ) : 7,24 (m, 1H, NH) ; 3,78-3,74 (m, 2H, CH₂) ; 2,75 (s, 3H, CH₃) ; 2,45-2,42 (m, 2H, CH₂) ; 2,11 (s, 6H, 2 CH₃).
SM-CL : MH+ = 343,97 ; t.r. = 7,22 min.

### Exemple 36: 6-(butylthio)-5-{[2-(diméthylamino)éthyl]amino}-2-méthyl-1,3-benzothiazole-4,7-dione :

À 33 mg (96 µmol) du composé de l'exemple 35 en solution dans 4 ml d'éthanol anhydre sont ajoutés 20 µl (0,115 mmol; 1,2 éq.) de diisopropyléthylamine et 16 µl (0,154 mmol ; 1,6 éq.) de butanethiol. Le mélange réactionnel est maintenu sous agitation pendant 24 heures à 60 °C, puis après concentration sous pression réduite, le résidu est purifié par chromatographie sur colonne de silice (éluant : dichlorométhane/méthanol 95/5) et le produit attendu est obtenu, après reprise dans de l'éther éthylique, sous forme d'une poudre violette.
RMN ¹H (DMSO d6, 400 MHz, δ) : 7,56 (m, 1H, NH) ; 3,84-3,83 (m, 2H, CH₂) ; 2,75 (s, 3H, CH₃) ; 2,64-2,60 (t, 2H, CH₂); 2,45-2,42 (m, 2H, CH₂) ; 2,20 (s, 6H, 2 CH₃) ; 1,44-1,46 (m, 2H, CH₂) ; 1,37-1,33 (m, 2H, CH₂); 0,85-0,82 (t, 3H, CH₃).

### Exemple 37: 5-{[2-(diméthylamino)éthyl]amino}-2-(morpholin-4-ylméthyl)-1,3-benzothiazole-4,7-dione :

### 37.1) 2-(bromométhyl)-5-méthoxy-1,3-benzothiazole :

2,58 g (14,5 mmol ; 1,3 éq.) de *N*-bromosuccinimide et une pointe de spatule d'aza-bis-isobutyronitrile sont ajoutés à 2 g (11,16 mmol) de 2-méthyl-5-méthoxy-1,3-benzothiazole en solution dans 25 ml de tétrachlorure de carbone anhydre. Le mélange réactionnel est chauffé au reflux sous irradiation pendant 6 heures, avec ajout d'une pointe de spatule d'aza-bis-isobutyronitrile toutes les 2 heures. Après retour à température ambiante, l'insoluble formé est filtré, le solvant est évaporé sous pression réduite et le résidu purifié par chromatographie sur colonne de silice (éluant : acétate d'éthyle/heptane 1/4). Le produit attendu est obtenu sous forme d'un solide blanc.
RMN ¹H (DMSO d6, 400 MHz, δ) : 7,98-7,96 (m, 1H, H arom.) ; 7,54-7,53 (m, 1H, H arom.) ; 7,13-7,10 (m, 1H, H arom.) ; 5,09 (s, 2H, CH₂) ; 3,84 (s, 3H, CH₃).
SM-CL : MH+ = 258,38 ; t.r. = 10,36 min.

### 37.2) 5-méthoxy-2-(morpholin-4-ylméthyl)-1,3-benzothiazole :

678 µl de diisopropyléthylamine (3,9 mmol ; 2 éq.) sont ajoutés à 0,5 g de l'intermédiaire 37.1 en solution dans 20 ml de toluène anhydre. 187 µl (2,14 mmol ; 1,1 éq.) de morpholine et une pointe de spatule d'iodure de sodium sont ajoutés à la solution précédente, puis le mélange réactionnel est maintenu sous agitation à 80 °C pendant 3 heures. Après refroidissement, le milieu réactionnel est lavé avec de l'eau (3 fois 20 ml), puis la phase organique est séchée sur sulfate de magnésium et concentrée. Une purification par chromatographie sur colonne de silice (éluant : acétate d'éthyle/heptane 1/1) permet d'obtenir le produit attendu sous forme d'un solide beige.
RMN ¹H (DMSO d6, 400 MHz, δ) : 7,91-7,89 (m, 1H, H arom.) ; 7,47-7,46 (m, 1H, H arom.) ; 7,05-7,02 (m, 1H, H arom.) ; 3,92 (s, 2H, CH₂) ; 3,82 (s, 3H, CH₃) ; 3,63-3,61 (m, 4H, 2CH₂) ; 2,56-2,53 (m, 4H, 2CH₂).
SM-CL : MH+ = 265,10 ; t.r. = 7,55 min.

### 37.3) 5-méthoxy-2-(morpholin-4-ylméthyl)-4-nitro-1,3-benzothiazole :

84 mg (0,83 mmol ; 1,2 éq.) de nitrate de potassium sont ajoutés par portions à une solution à 0 °C de 0,2 g (0,76 mmol) d'intermédiaire 37.2 dans 0,7 ml d'acide sulfurique concentré. Après retour à température ambiante, le mélange réactionnel est agité pendant 18 heures, neutralisé par addition d'une solution aqueuse de soude 10*M* puis extrait par 3 fois 50 ml de dichlorométhane. La phase organique résultante est séchée sur sulfate de magnésium et concentrée, puis purifiée par chromatographie sur colonne de silice (éluant : acétate d'éthyle / heptane 1/1). Le produit attendu est obtenu sous forme d'une huile jaune.
RMN ¹H (DMSO d6, 400 MHz, δ) : 8,26-8,24 (m, 1H, H arom.) ; 7,48-7,46 (m, 1H, H arom.); 3,98-3,96 (2s, 5H, CH₃, CH₂); 3,63-3,61 (m, 4H, 2CH₂); 2,59-2,56 (m, 4H, 2 CH₂).
SM-CL : MH+ = 310,11 ; t.r. = 8,03 min.

### 37.4) 5-méthoxy-2-(morpholin-4-ylméthyl)-1,3-benzothiazol-4-amine :

0,93 g (4,11 mmol ; 5 éq.) de chlorure d'étain sont ajoutés à une solution de 0,254 g (0,822 mmol) d'intermédiaire 37.3 dans 7 ml d'acide chlorhydrique concentré. Le mélange réactionnel est maintenu sous agitation pendant 3 heures à 70 °C. Après retour à température ambiante, le milieu est dilué par addition de 20 ml d'acétate d'éthyle, puis neutralisé par une solution saturée en NaHCO₃ et enfin lavé par 3 fois 20 ml d'eau. Les phases organiques sont regroupées, séchées sur sulfate de magnésium et concentrées pour fournir le produit attendu sous forme d'une poudre beige.
RMN ¹H (DMSO d6, 400 MHz, δ) : 7,12-7,10 (m, 1H, H arom.) ; 7,02-7,00 (m, 1H, H arom.); 5,04 (s, 2H, NH₂); 3,88 (s, 2H, CH₂) ; 3,81 (s, 3H, CH₃) ; 3,63-3,60 (m, 4H, 2 CH₂) ; 2,55-2,52 (m, 4H, 2CH₂).
SM-CL : MH+ = 280,11 ; t.r. = 7,29 min.

### 37.5) 5-méthoxy-2-(morpholin-4-ylméthyl)-1,3-benzothiazole-4,7-dione :

Une solution de 84 mg (0,31 mmol ; 1,8 éq.) de sel de Frémy, dissous dans 14 ml d'une solution tampon (0,3*M*) d'hydrogénophosphate de sodium, est ajoutée à 0,0483 mg (0,17 mmol) d'intermédiaire 37.4 en solution dans 10 ml d'acétone. Le mélange réactionnel est agité pendant 18 heures à température ambiante, puis extrait par 3 fois 30 ml de dichlorométhane, les phases organiques étant ensuite lavées avec 2 fois 20 ml d'eau. Les phases organiques sont ensuite regroupées, séchées sur sulfate de magnésium puis concentrées sous pression réduite. Le résidu est purifié par chromatographie sur colonne de silice (éluant : acétate d'éthyle / heptane 1/1) et le produit attendu est obtenu sous forme d'une huile jaune.
SM-CL : MH+ = 295,06 ; t.r. = 7,11 min.

### 37.6) 5-{[2-(diméthylamino)éthyl]amino}-2-(morpholin-4-ylméthyl)-1,3-benzothiazole-4,7-dione :

Le protocole expérimental utilisé est identique à celui décrit pour l'exemple 1, l'intermédiaire 37.5 remplaçant le 5-méthoxy-2-méthyl-4,7-dioxobenzothiazole.
SM-CL : MH+ = 351,38 ; t.r. = 3,07 min.

### Exemple 38 : 5-{[2-(diméthylamino)éthyl]amino}-2-[(4-phénylpipérazin-1-yl)méthyl]-1,3-benzothiazole-4,7-dione :

Le protocole expérimental utilisé est identique à celui décrit pour l'exemple 37, la *N*-phénylpipérazine remplaçant la morpholine dans la deuxième étape.
SM-CL : MH+ = 426,18 ; t.r. = 7,39 min.

### Exemple 39 : 5-{[2-(diméthylamino)éthyl]amino}-2-(pipéridin-1-ylméthyl)-1,3-benzothiazole-4,7-dione :

Le protocole expérimental utilisé est identique à celui décrit pour l'exemple 37, la pipéridine remplaçant la morpholine dans la deuxième étape.
SM-CL : MH+ = 349,13 ; t.r. = 2,82 min.

*Les composés des exemples 40 à 52 sont obtenus de façon analogue à celle décrite pour l'exemple 15, les amines primaires ou secondaires adéquates remplaçant l'aniline dans la quatrième et dernière étape.*

### Exemple 40 : 5-{[2-(diméthylamino)éthyl]amino}-2-éthyl-1,3-benzoxazole-4,7-dione ou 6-{[2-(diméthylamino)éthyl]amino}-2-éthyl-1,3-benzoxazole-4,7-dione :

Point de fusion: 123°C.
RMN ¹H (DMSO d6, 400 MHz, δ): 7,39 (t, 1H, NH) ; 5,30 (s, 1H, CH); 3,30-3,31 (m, 2H, CH₂); 3,24-3,20 (m, 2H, CH₂) ; 2,95-2,88 (q, 2H, CH₂); 2,17 (s, 6H, 2 CH₃) ; 1,30 (t, 3H, CH₃).
SM-CL : MH+ = 264,13 ; t.r. = 7,02 min.

### Exemple 41 : 2-[(2-éthyl-4,7-dioxo-4,7-dihydro-1,3-benzoxazol-5-yl)(méthyl)amino]éthylcarbamate de tert-butyle ou 2-[(2-éthyl-4,7-dioxo-4,7-dihydro-1,3-benzoxazol-6-yl)(méthyl)amino]éthylcarbamate de tert-butyle :

Point de fusion: 135 °C.
RMN ¹H (DMSO d6, 400 MHz, δ): 7,82 (t, 1H, NH); 5,36 (s, 1H, CH); 3,38-3,36 (m, 2H, CH₂) ; 3,30-3,27 (m, 2H, CH₂) ; 2,93-2,88 (q, 2H, CH₂) ; 2,79 (s, 3H, CH₃) ; 1,37-1,26 (m, 12H, 4 CH₃).
SM-CL : MH+ = 350,14 ; t.r. = 9,72 min.

### Exemple 42 : 2-[(2-éthyl-4,7-dioxo-4,7-dihydro-1,3-benzoxazol-5-yl)amino]éthylcarbamate de tert-butyle ou 2-[(2-éthyl-4,7-dioxo-4,7-dihydro-1,3-benzoxazol-6-yl)amino]éthylcarbamate de tert-butyle :

Point de fusion : 173 °C.
RMN ¹H (DMSO d6, 400 MHz, δ): 7,73 (t, 1H, NH); 6,97 (t, 1H,, NH); 5,36 (s, 1H, CH); 3,20-3,17 (m, 2H, CH₂); 3,15-3,12 (m, 2H, CH₂); 2,93-2,88 (q, 2H, CH₂) ; 1,36 (s, 9H, 3CH₃); 1,28 (t, 3H, CH₃).
SM-CL : MH+ = 336,23 ; t.r. = 9,24 min.

### Exemple 43 : 5-{[3-(diméthylamino)propyl]amino}-2-éthyl-1,3-benzoxazole-4,7-dione ou 6-{[3-(diméthylamino)propyl]amino}-2-éthyl-1,3-benzoxazole-4,7-dione :

Point de fusion: 101 °C.
RMN ¹H (DMSO d6, 400 MHz, δ): 8,09 (t, 1H, NH); 5,28 (s, 1H, CH) ; 3,21-3,16 (m, 2H, CH₂); 2,93-2,88 (q, 2H, CH₂) ; 2,28-2,25 (m, 2H, CH₂) ; 2,13 (s, 6H, 2 CH₃) ; 1,71-1,67 (m, 2H, CH₂) ; 1,28 (t, 3H, CH₃).
SM-CL : MH+ = 278,19 ; t.r. = 7,09 min.

### Exemple 44 : 2-éthyl-5-{[2-(1-méthylpyrrolidin-2-yl)éthyl]amino}-1,3-benzoxazole-4,7-dione ou 2-éthyl-6-{[2-(1-méthylpyrrolidin-2-yl)éthyl]amino}-1,3-benzoxazole-4,7-dione :

Point de fusion : 121 °C.
RMN ¹H (DMSO d6, 400 MHz, δ): 8,11 (t, 1H, NH); 5,24 (s, 1H, CH) ; 3,19-3,17 (m, 2H, CH₂); 2,95-2,93 (m, 1H, CH); 2,92-2,87 (q, 2H, CH₂) ; 2,21 (s, 3H, CH₃) ; 2,16-2,05 (m, 2H, CH₂); 1,88-1,84 (m, 2H, CH₂) ; 1,63-1,57 (m, 4H, 2 CH₂) ; 1,28 (t, 3H, CH₃).
SM-CL : MH+ = 304,20 ; t.r. = 7,20 min.

### Exemple 45 : 5-{[4-(diméthylamino)butyl]amino}-2-éthyl-1,3-benzoxazole-4,7-dione ou 6-{[4-(diméthylamino)butyl]amino}-2-éthyl-1,3-benzoxazole-4,7-dione :

RMN ¹H (DMSO d6, 400 MHz, δ): 8,06 (t, 1H, NH); 5,28 (s, 1H, CH); 3,17-3,12 (m, 2H, CH₂) ; 2,93-2,88 (q, 2H, CH₂) ; 2,22-2,19 (m, 2H, CH₂) ; 2,11 (s, 6H, 2CH₃) ; 1,61-1,56 (m, 2H, CH₂) ; 1,46-1,42 (m, 2H, CH₂); 1,28 (t, 3H, CH₃).
SM-CL : MH+ = 292,20 ; t.r. = 7,10 min.

### Exemple 46 : 2-éthyl-5-[(4-pyrrolidin-1-ylbutyl)amino]-1,3-benzoxazole-4,7-dione ou 2-éthyl-6-[(4-pyrrolidin-1-ylbutyl)amino]-1,3-benzoxazole-4,7-dione :

Point de fusion : 102 °C.
RMN ¹H (DMSO d6, 400 MHz, δ): 7,95 (t, 1H, NH); 5,28 (s, 1H, CH); 3,17-3,13 (m, 2H, CH₂); 2,93-2,87 (q, 2H, CH₂) ; 2,41-2,37 (m, 6H, 3CH₂); 1,63-1,58 (m, 2H, CH₂) ; 1,49-1,45 (m, 2H, CH₂) ; 1,28 (t, 3H, CH₃).
SM-CL : MH+ = 318,20 ; t.r. = 7,30 min.

### Exemple 47 : 5-{[5-(diméthylamino)pentyl]amino}-2-éthyl-1,3-benzoxazole-4,7-dione ou 6-{[5-(diméthylamino)pentyl]amino}-2-éthyl-1,3-benzoxazole-4,7-dione :

RMN ¹H (DMSO d6, 400 MHz, δ) : 7,83 (t, 1H, NH) ; 5,27 (s, 1H, CH) ; 3,17-3,13 (m, 2H, CH₂) ; 2,93-2,87 (q, 2H, CH₂); 2,18-2,14 (m, 2H, CH₂) ; 2,09 (s, 6H, 2CH₃) ; 1,58-1,54 (m, 2H, CH₂) ; 1,41-1,38 (m, 2H, CH₂) ; 1,28 (t, 3H, CH₃).
SM-CL : MH+ = 306,20 ; t.r. = 7,30 min.

### Exemple 48 : mélange de 5-{[6-(diméthylamino)hexyl]amino}-2-éthyl-1,3-benzoxazole-4,7-dione et 6-{[6-(diméthylamino)hexyl]amino}-2-éthyl-1,3-benzoxazole-4,7-dione :

SM-CL : MH+ = 320,20 ; t.r. = 7,50 min.

### Exemple 49 : mélange de 2-éthyl-5-(4-méthylpipérazin-1-yl)-1,3-benzoxazole-4,7-dione et 2-éthyl-6-(4-méthylpipérazin-1-yl)-1,3-benzoxazole-4,7-dione :

SM-CL : MH+ = 276,10 ; t.r. = 7,10 min.

### Exemple 50 : mélange de 2-éthyl-5-[(1-éthylhexyl)amino]-1,3-benzoxazole-4,7-dione et 2-éthyl-6-[(1-éthylhexyl)amino]-1,3-benzoxazole-4,7-dione :

SM-CL : MH+ = 305,20 ; t.r. = 11,50 min.

### Exemple 51 : mélange de 5-azocan-1-yl-2-éthyl-1,3-benzoxazole-4,7-dione et 6-azocan-1-yl-2-éthyl-1,3-benzoxazole-4,7-dione :

SM-CL : MH+ = 289,20 ; t.r. = 10,40 min.

### Exemple 52 : mélange de 2-éthyl-5-morpholin-4-yl-1,3-benzoxazole-4,7-dione et 2-éthyl-6-morpholin-4-yl-1,3-benzoxazole-4,7-dione :

SM-CL : MH+ = 263,10 ; t.r. = 8,60 min.

### Exemple 53 : 6-chloro-5-{[2-(diméthylamino)éthyl]amino}-2-éthyl-1,3-benzoxazole-4,7-dione ou 5-chloro-6-{[2-(diméthylamino)éthyl]amino}-2-éthyl-1,3-benzoxazole-4,7-dione :

Le protocole expérimental utilisé est identique à celui décrit pour l'exemple 34, le composé de l'exemple 40 remplaçant l'intermédiaire 2.1. Point de fusion :110°C. RMN ¹H (DMSO d6, 400 MHz, δ): 7,35 (t, 1H, NH) ; 3,78-3,74 (m, 2H, CH₂) ; 2,94-2,89 (q, 2H, CH₂); 2,48-2,45 (m, 2H, CH₂) ; 2,15 (s, 6H, 2CH₃) ; 1,28 (t, 3H, CH₃).
SM-CL : MH+ = 298,10 ; t.r. = 7,20 min.

### Exemple 54 : 6-bromo-5-{[2-(diméthylamino)éthyl]amino}-2-éthyl-1,3-benzoxazole-4,7-dione ou 5-bromo-6-{[2-(diméthylamino)éthyl]amino}-2-éthyl-1,3-benzoxazole-4,7-dione :

Le protocole expérimental utilisé est identique à celui décrit pour l'exemple 35, le composé de l'exemple 40 remplaçant l'intermédiaire 2.1.
RMN ¹H (DMSO d6, 400 MHz, δ): 7,27 (t, 1H, NH) ; 3,78-3,74 (m, 2H, CH₂) ; 2,94-2,89 (q, 2H, CH₂) ; 2,46-2,43 (m, 2H, CH₂); 2,13 (s, 6H, 2 CH₃) ; 1,26 (t, 3H, CH₃).
SM-CL : MH+ = 342,00 ; t.r. = 7,30 min.

### Exemple 55 : 5-{[2-(diméthylamino)éthyl]amino}-2-éthyl-6-méthyl-1,3-benzoxazole-4,7-dione :

### 55.1) 2-diazo-5-métlaylcyclohexane-1,3-dione :

À une solution de 5 g (39,6 mmol) de 5-méthylcyclohexane-1,3-dione dans 100 ml de dichlorométhane, on ajoute 12,25 ml (87,2 mmol ; 2,2 éq.) de triéthylamine et 8,57 g (35,67 mmol ; 0,9 éq.) de 4-acétamidobenzenesulfonylazide. Le mélange réactionnel est agité pendant 75 min à température ambiante, puis refroidi à 0 °C et filtré sur un lit de silice. Après concentration sous pression réduite, la solution est lavée avec 3 fois 50 ml d'eau. Les phases organiques sont regroupées, séchées sur sulfate de sodium et concentrées. Le solide résultant est repris dans de l'éther éthylique puis filtré et séché sous pression réduite. Il est utilisé dans l'étape suivante sans autre purification.
SM-CL : MH+ = 153,49 ; t.r. = 7,21 min.

### 55.2) 2-éthyl-6-méthyl-6,7-dihydro-o-1,3-benzoxazol-4(5H)-one :

A une solution de 4,9 g (32,2 mmol) d'intermédiaire 55.1 dans 50 ml de propionitrile, on ajoute 285 mg (0,644 mmol ; 0,02 éq.) d'acétate de rhodium. Le mélange réactionnel est maintenu sous agitation sous atmosphère inerte d'argon à 60 °C pendant 2 heures. Le solvant est ensuite évaporé et le résidu est purifié par chromatographie sur colonne de silice (éluant : acétate d'éthyle/heptane 1/1). Le produit attendu est obtenu sous forme d'une huile jaune.
RMN ¹H (DMSO d6, 400 MHz, δ): 3,02-2,97 (m, 1H, CH) ; 2,80-2,74 (q, 2H, CH₂); 2,68-2,61 (m, 1H, CH₂) ; 2,44-2,39 (m, 2H, CH₂); 2,34-2,30 (m, 1H, CH₂) ; 1,23 (t, 3H, CH₃) ; 1,08 (s, 3H, CH₃).
SM-CL : MH+ = 180,25 ; t.r. = 8,55 min.

### 55.3) oxime de (4E)-2-éthyl-6-ntéthyl-6,7-dihydro-1,3-benzoxazol-4(5H)-one :

A une solution de 1,39 g (7,76 mmol) d'intermédiaire 55.2 dans 200 ml de méthanol, on ajoute 647 mg (9,31 mmol; 1,2 éq.) de chlorhydrate d'hydroxylamine et 764 mg (9,31 mmol ; 1,2 éq.) d'acétate d'ammonium. Le mélange réactionnel est agité pendant 90 min au reflux du méthanol, puis le solvant est évaporé, le résidu est repris dans 50 ml d'eau puis neutralisé à l'aide d'une solution saturée en NaHCO₃. Le produit attendu est extrait par 2 fois avec 50 ml d'acétate d'éthyle puis lavé par 2 fois avec 30 ml d'eau. Les phases organiques sont regroupées, séchées sur sulfate de sodium et concentrées sous pression réduite. Le produit souhaité est obtenu sous forme d'un solide jaune foncé, utilisé sans autre purification dans l'étape suivante.
SM-CL : MH+ = 195,09 ; t.r. = 8,73 min.

### 55.4) 2-éthyl-6-méthyl-1,3-benzoxazol-4-amine :

1,45 g (7,46 mmol) d'intermédiaire 55.3 sont dissous dans 25 g d'acide polyphosphorique. Après 1 heure d'agitation à 140 °C, la solution est hydrolysée par addition d'eau glacée, puis neutralisée par une solution aqueuse de soude à 50%. Le produit obtenu est extrait avec du dichlorométhane, et la phase organique est lavée par 3 fois avec 25 ml d'eau, séchée sur sulfate de sodium et concentrée sous pression réduite. Le produit souhaité est obtenu après purification par chromatographie sur colonne de silice (éluant : dichlorométhane/éthanol 98/2).
SM-CL : MH+ = 177,21 ; t.r. = 9,12 min.

### 55.5) 2-éthyl-6-méthyl-1,3-benzoxazole-4,7-dione:

Le protocole expérimental utilisé est identique à celui décrit pour l'étape 15.3 de l'exemple 15, l'intermédiaire 55.4 remplaçant l'intermédiaire 15.2.
RMN ¹H (DMSO d6, 400 MHz, δ) : 6,72 (s, 1H, CH) ; 2,98-2,93 (q, 2H, CH₂) ; 2,04 (s, 3H, CH₃) ; 1,30 (t, 3H, CH₃).
SM-CL : MH+ = 192,06 ; t.r. = 8,93 min.

### 55.6) 5-{[2-(diméthylamino)éthzyl]amino}-2-éthyl-6-méthyl-1,3-benzoxazole-4,7-dione:

Le protocole expérimental utilisé est identique à celui décrit pour l'étape 15.4 de l'exemple 15, l'intermédiaire 55.5 remplaçant l'intermédiaire 15.3 et la *N,N*-diméthyléthylènediamine remplaçant l'aniline. Point de fusion : 135 °C.
RMN ¹H (DMSO d6, 400 MHz, δ) : 6,63 (t, 1H, NH) ; 3,62-3,58 (m, 2H, CH₂) ; 2,92-2,86 (q, 2H, CH₂) ; 2,44-2,41 (m, 2H, CH₂) ; 2,14 (s, 6H, 2 CH₃) ; 1,97 (s, 3H, CH₃) ; 1,27 (t, 3H, CH₃).
SM-CL : MH+ = 278,12 ; t.r. = 7,27 min.

### Exemple 56: 2-cyclopropyl-5-{[2-(diméthylamino)éthyl]amino}-1,3-benzoxazole-4,7-dione ou 2-cyclopropyl-6-{[2-(diméthylamino)éthyl]amino}-1,3-benzoxazole-4,7-dione :

Le protocole expérimental utilisé est identique à celui décrit pour l'exemple 55, la cyclohexane-1,3-dione remplaçant la 5-méthylcyclohexane-1,3-dione dans la première étape et le cyclopropanecarbonitrile remplaçant le propionitrile dans la deuxième étape. Point de fusion: 155 °C.
RMN ¹H (DMSO d6, 400 MHz, δ) : 7,35 (t, 1H, NH); 5,27 (s, 1H, CH); 3,30-3,18 (m, 2H, CH₂); 2,49-2,46 (m, 2H, CH₂); 2,28-2,25 (m, 1H, CH); 2,17 (s, 6H, 2 CH₃) ; 1,18-1,07 (m, 4H, 2 CH₂).
SM-CL : MH+ = 276,10 ; t.r. = 7,10 min.

### Exemple 57 : mélange de 5-{[2-(diméthylamino)éthyl]amino}-2-phényl-1,3-benzoxazole-4,7-dione et 6-{[2-(diméthylamino)éthyl]amino}-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la *N,N*-diméthyléthylènediamine remplaçant l'aniline dans la quatrième et dernière étape. Point de fusion : 147 °C.
RMN ¹H (DMSO d6, 400 MHz, δ): 8,15-8,08 (m, 2H, H arom.); 7,70-7,61 (m, 3H, H arom.); 7,33 (t, 1H, NH); 5,38 (s, 1H, CH); 3,26-3,21 (m, 4H, 2 CH₂) ; 2,19 (s, 6H, 2 CH₃).
Les deux composants du mélange peuvent être caractérisés par les déplacements RMN (400 MHz) du seul proton du cycle benzoxazoledione qui sont 5,38 et 5,39 ppm. SM-CL : MH+ = 312,20 ; t.r. = 7,70 min.

### Exemple 58 : mélange de 5-{[6-(diméthylamino)hexyl]amino}-2-phényl-1,3-benzoxazole-4,7-dione et 6-{[6-(diméthylamino)hexyl]amino}-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la 6-(diméthylamino)hexylamine remplaçant l'aniline dans la quatrième et dernière étape.
Les deux composants du mélange peuvent être caractérisés par les déplacements RMN (400 MHz) du seul proton du cycle benzoxazoledione qui sont 5,34 et 5,35 ppm. SM-CL : MH+= 368,20 ; t.r. = 8,10 min.

### Exemple 59 : 5-[(1-éthylhexyl)aminol-2-phényl-1,3-benzoxazole-4,7-dione ou 6-[(1-éthylhexyl)amino]-2-phényl-1,3-benzoxazole-4,7-dione :

Le protocole expérimental utilisé est identique à celui décrit pour l'exemple 15, l'orthobenzoate de triméthyle remplaçant l'orthopropionate de triéthyle dans la première étape et la 2-éthylhexylamine remplaçant l'aniline dans la quatrième et dernière étape. SM-CL : MH+ = 353,20 ; t.r. = 12,50 min.

### Exemple 60 : mélange de 2-(2,6-difluorophényl)-5-[(2-pyrrolidin-1-yléthyl)amino]-1,3-benzoxazole-4,7-dione et 2-(2,6-difluorophényl)-6-[(2-pyrrolidin-1-yléthyl)amino]-1,3-benzoxazole-4,7-dione :

### 60.1) 2-(2,6-difluorophényl)-4-nitro-1,3-benzoxazole:

À une solution de 5 g (32,4 mmol) de 2-amino-3-nitrophénol et de 5,12 g (32,4 mmol ; 1 éq.) d'acide 2,6-difluorobenzoïque dans 50 ml de xylène, on ajoute 2 g (32,4 mmol ; 1 éq.) d'acide borique. Le mélange est chauffé au reflux du xylène pendant 8 heures avec élimination de l'eau formée par un Dean-Stark. Après retour à température ambiante, le milieu réactionnel est dilué par 100 ml d'acétate d'éthyle et neutralisé par une solution aqueuse de soude à 10%. La phase organique est lavée par 3 fois avec 50 ml d'eau puis avec une solution saturée en NaCl avant d'être séchée sur sulfate de sodium, filtrée et concentrée sous pression réduite. Le 2-(2,6-difluorophényl)-4-nitro-1,3-benzoxazole est utilisé sans autre purification dans l'étape suivante.
SM-CL : MH+ = 277,00 ; t.r. = 10,45 min.

### 60.2) 2-(2,6-difluorophényl)-1,3-benzoxazol-4-amine:

À une solution de 3,5 g (12,7 mmol) de 2-(2,6-difluorophényl)-4-nitro-1,3-benzoxazole dans 60 ml d'acide chlorhydrique concentré, on ajoute 14,3 g (63,5 mmol ; 5 éq.) de chlorure d'étain. Le mélange est agité pendant 2 heures à 60 °C, puis, après retour à température ambiante et addition de 100 ml d'eau, est neutralisé par une solution aqueuse de soude à 50%. Le précipité formé est filtré sur lit de Célite et lavé avec de l'éthanol. La solution résultante est concentrée sous pression réduite, puis le produit souhaité est extrait par 3 fois avec 50 ml d'acétate d'éthyle. Les phases organiques sont regroupées, lavées par 2 fois avec 30 ml d'une solution saturée en chlorure de sodium, séchées sur sulfate de sodium et concentrées sous pression réduite. La 2-(2,6-difluorophényl)-1,3-benzoxazol-4-amine est utilisée sans autre purification dans l'étape suivante.
SM-CL : MH+ = 247,08 ; t.r. = 10,02 min.

### 60.3) 2-(2,6-difluorophéfzyl)-1,3-benzoxazole-4,7-dione:

Le protocole expérimental utilisé est identique à celui décrit pour l'étape 15.3 de l'exemple 15, l'intermédiaire 60.2 remplaçant l'intermédiaire 15.2. Le produit attendu est obtenu sous forme de cristaux jaunes.
SM-CL : MH+ = 261,93 ; t.r. = 9,62 min.

### 60.4) mélange de 2-(2,6-dfluorophényl)-5-[(2-pyrrolidin-1-yléthyl)amino]-1,3-benzoxazole-4,7-dione et 2-(2,6-difluorophényl)-6-[(2-pyrrolidin-1-yléthyl)amino]-1,3-benzoxazole-4,7-dione:

Le protocole expérimental utilisé est identique à celui décrit pour l'étape 15.4 de l'exemple 15, l'intermédiaire 60.3 remplaçant l'intermédiaire 15.3 et la (2-aminoéthyl)pyrrolidine remplaçant l'aniline. Point de fusion : 150 °C.
RMN ¹H (DMSO d6, 400 MHz, δ) : 7,78-7,76 (m, 1H, H arom.); 7,43-7,37 (m, 2H, H arom.); 5,41 (s, 1H, CH); 3,38-3,36 (m, 2H, CH₂); 3,28-3,26 (m, 4H, 2 CH₂); 2,68-2,64 (m, 2H, CH₂); 1,70-1,67 (m, 4H, 2 CH₂).
Les deux composants du mélange peuvent être caractérisés par les déplacements RMN (400 MHz) du seul proton du cycle benzoxazoledione qui sont 5,40 et 5,42 ppm. SM-CL : MH+ = 373,99 ; t.r. = 7,76 min.

*Les composés des exemples 61 à 65 sont obtenus de façon analogue à celle décrite pour l'exemple 60.*

### Exemple 61 : mélange de 2-[4-(diéthylamino)phényl]-5-{[2-(diméthylamino)éthyl]amino}-1,3-benzoxazole-4,7-dione et 2-[4-(diéthylamino)phényl]-6-{[2-(diméthylamino)éthyl]amino}-1,3-benzoxazole-4,7-dione :

RMN ¹H (DMSO d6, 400 MHz, δ): 7,91-7,89 (d, 2H, H arom.) ; 6,83-6,81 (d, 2H, H arom.) ; 5,29 (s, 1H, CH) ; 3,47-3,42 (m, 4H, 2 CH₂) ; 3,41-3,38 (m, 2H, CH₂); 3,25-3,21 (m, 2H, CH₂); 2,19 (s, 6H, 2 CH₃); 1,12 (t, 6H, 2 CH₃).
Les deux composants du mélange peuvent être caractérisés par les déplacements RMN (400 MHz) du seul proton du cycle benzoxazoledione qui sont 5,29 et 5,30 ppm. SM-CL : MH+ = 383,20 ; t.r. = 8,30 min.

### Exemple 62 : mélange de 2-[4-(diéthylamino)phényl]-5-[(2-pyrrolidin-1-yléthyl)amino]-1,3-benzoxazole-4,7-dione et 2-[4-(diéthylamino)phényl]-6-[(2-pyrrolidin-1-yléthyl)amino]-1,3-benzoxazole-4,7-dione :

RMN ¹H (DMSO d6, 400 MHz, δ): 7,91-7,88 (d, 2H, H arom.); 6,83-6,81 (d, 2H, H arom.); 5,29 (s, 1H, CH); 3,47-3,42 (m, 4H, 2 CH₂); 3,37-3,35 (m, 2H, CH₂); 3,26-3,23 (m, 4H, 2 CH₂); 2,66 (t, 2H, CH₂); 1,70-1,68 (m, 4H, 2 CH₂) ; 1,14 (t, 6H, 2 CH₃).
Les deux composants du mélange peuvent être caractérisés par les déplacements RMN (400 MHz) du seul proton du cycle benzoxazoledione qui sont 5,28 et 5,29 ppm. SM-CL : MH+ = 409,10 ; t.r. = 8,40 min.

### Exemple 63 : mélange de 2-(4-chlorophényl)-5-{[2-(diméthylamino)éthyl]amino}-1,3-benzoxazole-4,7-dione et 2-(4-chlorophényl)-6-{[2-(diméthylamino)éthyl]amino}-1,3-benzoxazole-4,7-dione :

Point de fusion: 169 °C.
Les deux composants du mélange peuvent être caractérisés par les déplacements RMN (400 MHz) du seul proton du cycle benzoxazoledione qui sont 5,39 et 5,41 ppm. SM-CL : MH+ = 346,20 ; t.r. = 8,10 min.

### Exemple 64 : mélange de 2-(4-chlorophényl)-5-{[3-(diméthylamino)propyl]amino}-1,3-benzoxazole-4,7-dione et 2-(4-chlorophényl)-6-{[3-(diméthylamino)propyl]amino}-1,3-benzoxazole-4,7-dione :

SM-CL : MH+ = 360,10 ; t.r. = 8,10 min.

### Exemple 65 : mélange de 2-(4-chlorophényl)-5-{[4-(diméthylamino)butyl]amino}-1,3-benzoxazole-4,7-dione et 2-(4-chlorophényl)-6-{[4-(diméthylamino)butyl]amino}-1,3-benzoxazole-4,7-dione :

RMN ¹H (DMSO d6, 400 MHz, δ): 8,13-8,09 (m, 2H, H arom.) ; 7,70-7,67 (m, 2H, H arom.); 5,36 (s, 1H, CH); 3,18-3,15 (m, 2H, CH₂); 2,25-2,21 (m, 2H, CH₂); 2,13 (s, 6H, 2 CH₃); 1,62-1,58 (m, 2H, CH₂); 1,48-1,44 (m, 2H, CH₂).
Les deux composants du mélange peuvent être caractérisés par les déplacements RMN (400 MHz) du seul proton du cycle benzoxazoledione qui sont 5,35 et 5,37 ppm. SM-CL : MH+ = 374,10 ; t.r. = 8,20 min.

### Exemple 66 : mélange de 2-(2-fluorophényl)-5-{[2-(diméthylamino)éthyl]amino}-1,3-benzoxazole-4,7-dione et 2-(2-fluorophényl)-6-{[2-(diméthylamino)éthyl]amino}-1,3-benzoxazole-4,7-dione :

### 66.1) 2-diazocyclohexane-1,3-dione:

Un mélange de 4-acétamidobenzenesulfonylazide (25 g, 104 mmol) et de triéthylamine (36 ml, 250 mmol) dans du dichlorométhane maintenu à une température inférieure à 30°C par refroidissement externe est traité au goutte-à-goutte par une solution de cyclohexane-1,3-dione (13 g, 115 mmol) dans 200 ml de dichlorométhane. Le mélange réactionnel est agité pendant 75 min à température ambiante puis filtré sur Célite. Après concentration à environ 300 ml, le filtrat est lavé à l'eau puis séché sur sulfate de sodium. Le solide jaune-brun (14 g ; 88%) obtenu par évaporation du solvant sous pression réduite est similaire à celui obtenu dans l'exemple 55.1, et est utilisé tel quel dans l'étape suivante.
RMN ¹H (DMSO-*d*₆, δ): 1,93 (m, 2H) ; 2,50 (t, 4H).
RMN ¹³C (DMSO-*d*₆, δ): 18,20 ; 36,68 ; 190,96.

### 66.2) 2-(2-fluorophényl)-6,7-dihydro-1,3-benzoxazol-4(5H)-one :

Un mélange d'acétate de rhodium (32 mg, 72 µmol) et de 2-fluorobenzonitrile (2,31 ml ; 22 mmol) dans du perfluorobenzène (5 ml) est traité à 60 °C au goutte-à-goutte par une solution de diazocyclohexanedione (obtenue à l'étape 66.1 ; 1 g ; 7,24 mmol) dans 5 ml de perfluorobenzène. Le milieu réactionnel est maintenu à 60 °C jusqu'à épuisement du dégagement d'azote (1h ; CCM sur SiO₂: 2% MeOH/CH₂Cl₂). Après refroidissement à température ambiante et filtration, le solvant du filtrat est évaporé. Le résidu est purifié par chromatographie (SiO₂: AcOEt/heptane : 1/1) pour donner une poudre jaune clair.
RMN ¹H (CDCl₃, δ): 2,31 (m, 2H) ; 2,66 (m, 2H,) ; 3,09 (t, 2H) ; 7,19-7,28 (m, 2H); 7,48-7,50 (m, 1H); 8,15-8,19 (m, 1H).
SM-CL : MH+ = 232,08 ; t.r. = 9,28 min.

### 66.3) 5-bromo-2-(2-fluorophényl)-6,7-dihydro-1,3-benzoxazol-4(5H)-one :

Une solution de l'intermédiaire 66.2 (470 mg, 2 mmol) dans l'acide acétique (5 ml) est traitée avec du brome dans de l'acide acétique (0,2*M*; 10 ml ; 2 mmol) pendant 4 jours à température ambiante (CCM sur SiO₂ : AcOEt/heptane: 1/1). Le milieu réactionnel est ensuite dilué avec de l'eau et extrait à l'aide de dichlorométhane. Les phases organiques sont regroupées, lavées avec une solution saturée de bicarbonate puis avec une solution de disulfite de sodium à 5%. Après séchage sur sulfate de sodium et élimination des volatiles sous pression réduite, on obtient une huile jaune qui est purifiée par chromatographie (SiO₂: AcOEt/heptane: 1/1) pour donner une poudre blanche.
RMN ¹H (DMSO-*d*₆, δ): 2,49 (m, 2H); 2,73 (m, 1H,); 3,15 (m, 2H) ; 4,95 (t, 1H,); 7,39-7,48 (m, 2H) ; 7,63-7,67 (m, 1H) ; 8,03-8,08 (t, 1H).
SM-CL : MH+ = 309,93 ; t.r. = 10,08 min.

### 66.4) 2-(2-fluorophényl)-4-hydroxy-1,3-benzoxazole :

L'intermédiaire 66.3 (6,52 g ; 21 mmol) en solution dans du tétrahydrofuranne (100 ml) est traité au goutte-à-goutte par du diazabicyclo[5.4.0]undec-7-ène (4,7 ml ; 31 mmol). Lorsque la réaction est complète (1,5 h ; CCM sur SiO₂: AcOEt/heptane: 1/1), le mélange réactionnel est étendu avec de l'acétate d'éthyle puis lavé successivement avec de l'acide chlorhydrique 1*N* et une solution saturée de chlorure de sodium. Les phases organiques regroupées sont séchées et concentrées pour donner un résidu brun qui est purifié par chromatographie (SiO₂: AcOEt/heptane: 1/1) pour donner une poudre beige.
RMN ¹H (DMSO-*d*₆, δ): 6,80 (d, 1H); 7,19-7,26 (m, 2H) ; 7,41-7,49 (m, 2H) ; 7,65 (m, 1H); 8,18 (t, 1H) ; 10,43 (s, 1H).
SM-CL : MH+ = 230,07 ; t.r. = 10,03 min.

### 66.5) 2-(2-fluorophényl)-1,3-benzoxazole-4,7-dione :

Le protocole expérimental utilisé est identique à celui décrit pour l'étape 15.3 de l'exemple 15, l'intermédiaire 66.4 remplaçant l'intermédiaire 15.2. On obtient une poudre jaune.
RMN ¹H (DMSO-*d*₆, δ): 6,94 (large, 2H); 7,45-7,54 (m, 2H); 7,74 (m, 2H); 8,18 (t, 1H).
SM-CL : MH+ = 244,04; t.r. = 9,73 min.(61%) et MH₃+ = 246,06; t.r. = 8,70 min.

### 66.6) Mélange de 2-(2-fluorophényl)-5-{[2-(diméthylanzino)éthyl]amino}-1,3-benzoxazole-4,7-dione et 2-(2-fluonophérzyl)-6-{[2-(diméthylamino)éthyl]amino}-1,3-benzoxazole-4,7-dione :

Le protocole expérimental utilisé est identique à celui décrit pour l'étape 15.4 de l'exemple 15, l'intermédiaire 66.5 remplaçant l'intermédiaire 15.3 et la *N,N*-diméthyléthylènediamine remplaçant l'aniline. On obtient une poudre rubis Point de fusion: 191 °C.
RMN ¹H (DMSO-*d*₆, δ): 2,19 (s, 6H) ; 2,5 (m, 2H) ; 3,27 (m, 2H); 5,41 (s, 1H); 7,42-7,52 (m, 3H) ; 7,70 (m, 2H) ; 8,13 (m, 1H).
Les deux composants du mélange peuvent être caractérisés par les déplacements RMN (400 MHz) du seul proton du cycle benzoxazoledione qui sont 5,40 et 5,41 ppm.
SM-CL : MH+ = 330,14 ; t.r. = 7,69 min.

### Exemple 67 : mélange de 2-(2-fluorophényl)-5-[(2-pyrrolidin-1-yléthyl)amino]-1,3-benzoxazole-4,7-dione et 2-(2-fluorophényl)-6-[(2-pyrrolidin-1-yléthyl)amino]-1,3-benzoxazole-4,7-dione :

Le protocole expérimental utilisé est identique à celui décrit pour l'exemple 66, la *N*-(2-aminoéthyl)-pyrrolidine remplaçant la *N,N*-diméthyléthylènediamine. Point de fusion : 152 °C.
Les deux composants du mélange peuvent être caractérisés par les déplacements RMN (400 MHz) du seul proton du cycle benzoxazoledione qui sont 5,39 et 5,41 ppm.
SM-CL : MH+ = 356,1 ; t.r. = 7,8 min.

### Exemple 68 : mélange de 2-(2-bromophényl)-5-{[2-(diméthylamino)éthyl]amino}-1,3-benzoxazole-4,7-dione et 2-(2-bromophényl)-6-{[2-(diméthylamino)éthyl]amino}-1,3-benzoxazole-4,7-dione :

### 68.1) 2-(2-bromophényl)-6,7-dihydro-1,3-benzoxazol-4(5H)-one :

Le protocole expérimental utilisé est identique à celui décrit pour l'étape 66.2, le 2-bromobenzonitrile remplaçant le 2-fluorobenzonitrile. On obtient un solide jaune.
SM-CL : MH+ = 292,0 ; t.r. = 9,8 min.

### 68.2) 5-bromo-2-(2-bromophényl)-6,7-dihydro-1,3-benzoxazol-4(5H)-one :

Un mélange de l'intermédiaire 68.1 (6,6 g, 22 mmol) et de CuBr₂ (10 g ; 45 mmol) dans de l'acétate d'éthyle (250 ml) additionné d'environ 1 ml d'acide acétique est porté au reflux durant 3,5 h (CCM sur SiO₂: AcOEt/heptane: 1/1). Le milieu réactionnel est ensuite filtré sur Célite, le filtrat est évaporé sous pression réduite et le résidu est purifié sur colonne (SiO₂: AcOEt/heptane : 1/1) pour donner une poudre jaune clair.
SM-CL : MH+ = 371,8 ; t.r. = 10,5 min.

### 68.3) 2-(2-bromophényl)-5-{[2-(diméthylamino)éthyl]amino}-1,3-benzoxazole-4,7-dione :

Ce composé est obtenu à partir de l'intermédiaire 68.2 selon les modes opératoires décrits pour les étapes 66.4, 66.5 et 66.6. Point de fusion : 138 °C.
Les deux composants du mélange peuvent être caractérisés par les déplacements RMN (400 MHz) du seul proton du cycle benzoxazoledione qui sont 5,41 et 5,43 ppm.
SM-CL : MH+ = 390,0 ; t.r. = 7,9 min.

### Exemple 69 : mélange de 2-(2-bromophényl)-5-[(2-pyrrolidin-1-yléthyl)amino]-1,3-benzoxazole-4,7-dione et 2-(2-bromophényl)-6-[(2-pyrrolidin-1-yléthyl)amino]-1,3-benzoxazole-4,7-dione :

Le protocole expérimental utilisé est identique à celui décrit pour l'exemple 68, la *N*-(2-aminoéthyl)-pyrrolidine remplaçant la *N,N*-diméthyléthylènediamine. Point de fusion : 122 °C.
Les deux composants du mélange peuvent être caractérisés par les déplacements RMN (400 MHz) du seul proton du cycle benzoxazoledione qui sont 5,40 et 5,42 ppm.
SM-CL : MH+ = 416,0 ; t.r. = 8,0 min.

### Exemple 70 : mélange de 2-(2-bromophényl)-5-{[3-(diméthylamino)propyl]amino}-1,3-benzoxazole-4,7-dione et 2-(2-bromophényl)-6-{[3-(diméthylamino)propyl]amino}-1,3-benzoxazole-4,7-dione :

Le protocole expérimental utilisé est identique à celui décrit pour l'exemple 68, la *N,N*-diméthylpropylènediamine remplaçant la *N,N*-diméthyléthylènediamine. Point de fusion: 119°C.
Les deux composants du mélange peuvent être caractérisés par les déplacements RMN (400 MHz) du seul proton du cycle benzoxazoledione qui sont 5,38 et 5,40 ppm.
SM-CL : MH+ = 404,0 ; t.r. = 8,0 min.

### Exemple 71 : mélange de 2-(2-chlorophényl)-5-{[2-(diméthylamino)éthyl]amino}-1,3-benzoxazole-4,7-dione et 2-(2-chlorophényl)-6-{[2-(diméthylamino)éthyl]amino}-1,3-benzoxazole-4,7-dione :

Le protocole expérimental utilisé est identique à celui décrit pour l'exemple 66, le 2-chlorobenzonitrile remplaçant le 2-fluorobenzonitrile. Point de fusion: 137 °C.
Les deux composants du mélange peuvent être caractérisés par les déplacements RMN (400 MHz) du seul proton du cycle benzoxazoledione qui sont 5,39 et 5,41 ppm.
SM-CL : MH+ = 346,1 ; t.r. = 7,8 min.

### Exemple 72 : mélange de 2-(2-chlorophényl)-5-[(2-pyrrolidin-1-yléthyl)amino]-1,3-benzoxazole-4,7-dione et 2-(2-chlorophényl)-6-[(2-pyrrolidin-1-yléthyl)amino]-1,3-benzoxazole-4,7-dione :

Le protocole expérimental utilisé est identique à celui décrit pour l'exemple 71, la *N*-(2-aminoéthyl)-pyrrolidine remplaçant la *N,N*-diméthyléthylènediamine. Point de fusion : 85 °C.
Les deux composants du mélange peuvent être caractérisés par les déplacements RMN (400 MHz) du seul proton du cycle benzoxazoledione qui sont 5,40 et 5,41 ppm.
SM-CL : MH+ = 372,1 ; t.r. = 8,0 min.

### Exemple 73 : mélange de 2-(3-bromophényl)-5-{[2-(diméthylamino)éthyl]amino}-1,3-benzoxazole-4,7-dione et 2-(3-bromophényl)-6-{[2-(diméthylamino)éthyl]amino}-1,3-benzoxazole-4,7-dione :

Le protocole expérimental utilisé est identique à celui décrit pour l'exemple 68, le 3-bromobenzonitrile remplaçant le 2-bromobenzonitrile. Point de fusion: 133 °C.
Les deux composants du mélange peuvent être caractérisés par les déplacements RMN (400 MHz) du seul proton du cycle benzoxazoledione qui sont 5,39 et 5,41 ppm.
SM-CL : MH+ = 390,0 ; t.r. = 8,1 min.

### Exemple 74 : mélange de 2-(4-bromophényl)-5-[(2-pyrrolidin-1-yléthyl)amino]-1,3-benzoxazole-4,7-dione et 2-(4-bromophényl)-6-[(2-pyrrolidin-1-yléthyl)amino]-1,3-benzoxazole-4,7-dione:

Le protocole expérimental utilisé est identique à celui décrit pour l'exemple 68, le 4-bromobenzonitrile étant employé au lieu du 2-bromobenzonitrile, et la *N*-(2-aminoéthyl)-pyrrolidine remplaçant la *N,N*-diméthyléthylènediamine. Point de fusion : 181 °C.
Les deux composants du mélange peuvent être caractérisés par les déplacements RMN (400 MHz) du seul proton du cycle benzoxazoledione qui sont 5,37 et 5,39 ppm.
SM-CL : MH+ = 415,0 ; t.r. = 8,3 min.

### Exemple 75 : mélange de 2-(4-bromophényl)-5-{[2-(diméthylamino)éthyl]amino}-1,3-benzoxazole-4,7-dione et 2-(4-bromophényl)-6-{[2-(diméthylamino)éthyl]amino}-1,3-benzoxazole-4,7-dione:

Le protocole expérimental utilisé est identique à celui décrit pour l'exemple 74, la *N,N*-diméthyléthylènediamine remplaçant la *N*-(2-aminoéthyl)-pyrrolidine. Point de fusion: 184 °C.
Les deux composants du mélange peuvent être caractérisés par les déplacements RMN (400 MHz) du seul proton du cycle benzoxazoledione qui sont 5,38 et 5,40 ppm.
SM-CL : MH+ = 390,1 ; t.r. = 8,2 min.

### Exemple 76 : mélange de 2-(4-fluorophényl)-5-[(2-pyrrolidin-1-yléthyl)amino]-1,3-benzoxazole-4,7-dione et 2-(4-fluorophényl)-6-[(2-pyrrolidin-1-yléthyl)amino]-1,3-benzoxazole-4,7-dione:

### 76.1) 2-(4-fluorophényl)-6,7-dihydro-1,3-benzoxazol-4(5H)-one:

Le protocole expérimental utilisé est identique à celui décrit pour l'étape 66.2, le 4-fluorobenzonitrile remplaçant le 2-fluorobenzonitrile. On obtient un solide jaune.
SM-CL : MH+ = 232,1 ; t.r. = 9,4 min.

### 76.2) 5-bromo-2-(4-fluorophényl)-6,7-dihydro-1,3-benzoxazol-4(5H)-one :

À une solution de l'intermédiaire 76.1 (600 mg; 2,59 mmol) dans de l'acide acétique glacial (25 ml) porté à 50°C est ajouté en trois portions égales espacées par des intervalles de 2-3 min du tribromure de pyridinium (996 mg ; 3,11 mmol). Le mélange réactionnel est maintenu à 50 °C durant 4 h (CCM sur SiO₂: AcOEt/heptane : 1/1). Les volatiles sont évaporés sous pression réduite, puis le résidu est repris à l'eau et extrait avec du dichlorométhane. Le milieu réactionnel est ensuite filtré sur Célite, le filtrat est évaporé sous pression réduite et le résidu est purifié sur colonne (SiO₂: AcOEt/heptane: 1/1) pour donner une poudre jaune clair. Les phases organiques sont regroupées et lavées avec une solution de bicarbonate à 10% puis avec une solution saturée de chlorure de sodium. Après séchage sur sulfate de sodium et élimination des volatiles sous pression réduite, le résidu est purifié par chromatographie sur colonne (SiO₂: AcOEt/heptane : 1/1) pour donner une poudre beige.
SM-CL : MH+ = 312,0 ; t.r. = 10,3 min.

### 76.3) Mélange de 2-(4-fluorophényl)-5-{[2-(diméthylamino)éthyl]amino}-1,3-benzoxazole-4,7-dione et 2-(4-fluorophényl)-6-{[2-(diméthylamino)éthyl]amino}-1,3-benzoxazole-4,7-dione:

Ce composé est obtenu à partir de l'intermédiaire 76.2 selon les modes opératoires décrits pour les étapes 66.4, 66.5 et 66.6. Point de fusion : 162 °C.
Les deux composants du mélange peuvent être caractérisés par les déplacements RMN (400 MHz) du seul proton du cycle benzoxazoledione qui sont 5,37 et 5,39 ppm.
SM-CL : MH+ = 356,1 ; t.r. = 8,0 min.

### Exemple 77 : mélange de 5-{[2-(diméthylamino)éthyl]amino}-2-(4-fluorophényl)-1,3-benzoxazole-4,7-dione et 6-{[2-(diméthylamino)éthyl]amino}-2-(4-fluorophényl)-1,3-benzoxazole-4,7-dione:

Le protocole expérimental utilisé est identique à celui décrit pour l'exemple 76, la *N*-(2-aminoéthyl)-pyrrolidine remplaçant la *N,N*-diméthyléthylènediamine. Point de fusion : 170 °C.
Les deux composants du mélange peuvent être caractérisés par les déplacements RMN (400 MHz) du seul proton du cycle benzoxazoledione qui sont 5,38 et 5,39 ppm.
SM-CL : MH+ = 330,1 ; t.r. = 7,8 min.

### Exemple 78 : mélange de 5-[(1-benzylpyrrolidin-3-yl)amino]-2-(4-fluorophényl)-1,3-benzoxazole-4,7-dione et 6-[(1-benzylpyrrolidin-3-yl)amino]-2-(4-fluorophényl)-1,3-benzoxazole-4,7-dione :

Le protocole expérimental utilisé est identique à celui décrit pour l'exemple 76, la (1-benzylpyrrolidin-3-yl)-amine remplaçant la *N,N*-diméthyléthylènediamine. Point de fusion : 180 °C.
Les deux composants du mélange peuvent être caractérisés par les déplacements RMN (400 MHz) du seul proton du cycle benzoxazoledione qui sont 5,37 et 5,39 ppm.
SM-CL : MH+ = 418,1 ; t.r. = 8,5 min.

### Exemple 79 : mélange de 5-{[3-(diméthylamino)propyl]amino}-2-(4-fluorophényl)-1,3-benzoxazole-4,7-dione et 6-{[3-(diméthylamino)propyl]amino}-2-(4-fluorophényl)-1,3-benzoxazole-4,7-dione:

Le protocole expérimental utilisé est identique à celui décrit pour l'exemple 76, la *N*,*N-*diméthylpropylènediamine remplaçant la *N,N*-diméthyléthylènediamine. Point de fusion: 149 °C.
Les deux composants du mélange peuvent être caractérisés par les déplacements RMN (400 MHz) du seul proton du cycle benzoxazoledione qui sont 5,35 et 5,37 ppm.
SM-CL : MH+ = 344,2 ; t.r. = 7,9 min.

### Exemple 80 : mélange de 2-(3,5-difluorophényl)-5-[(2-pyrrolidin-1-yléthyl)amino]-1,3-benzoxazole-4,7-dione et 2-(3,5-difluorophényl)-6-[(2-pyrrolidin-1-yléthyl)amino]-1,3-benzoxazole-4,7-dione:

Le protocole expérimental utilisé est identique à celui décrit pour l'exemple 68, le 3,5-difluorobenzonitrile remplaçant le 2-bromobenzonitrile et la *N*-(2-aminoéthyl)-pyrrolidine remplaçant la *N,N*-diméthyléthylènediamine. Point de fusion : 158 °C.
Les deux composants du mélange peuvent être caractérisés par les déplacements RMN (400 MHz) du seul proton du cycle benzoxazoledione qui sont 5,41 et 5,43 ppm.
SM-CL : MH+ = 374,0 ; t.r. = 8,0 min.

### Exemple 81 : mélange de 2-(3,5-difluorophényl)-5-{[2-(diméthylamino)éthyl]amino}-1,3-benzoxazole-4,7-dione et 2-(3,5-difluorophényl)-6-{[2-(diméthylamino)éthyl]amino}-1,3-benzoxazole-4,7-dione:

Le protocole expérimental utilisé est identique à celui décrit pour l'exemple 68, le 3,5-difluorobenzonitrile remplaçant le 2-bromobenzonitrile. Point de fusion : 175 °C. Les deux composants du mélange peuvent être caractérisés par les déplacements RMN (400 MHz) du seul proton du cycle benzoxazoledione qui sont 5,33 et 5,41 ppm.
SM-CL : MH+ = 348,0 ; t.r. = 7,9 min.

### Exemple 82 : mélange de 2-(2,5-difluorophényl)-5-[(2-pyrrolidin-1-yléthyl)amino]-1,3-benzoxazole-4,7-dione et 2-(2,5-difluorophényl)-6-[(2-pyrrolidin-1-yléthyl)amino]-1,3-benzoxazole-4,7-dione :

Le protocole expérimental utilisé est identique à celui décrit pour l'exemple 68, le 2,5-difluorobenzonitrile remplaçant le 2-bromobenzonitrile et la *N*-(2-aminoéthyl)-pyrrolidine remplaçant la *N,N*-diméthyléthylènediamine.
Point de fusion: 163 °C.
Les deux composants du mélange peuvent être caractérisés par les déplacements RMN (400 MHz) du seul proton du cycle benzoxazoledione qui sont 5,40 et 5,42 ppm.
SM-CL : MH+ = 374,0 ; t.r. = 7,9 min.

### Exemple 83 : mélange de 2-(2,5-difluorophényl)-5-{[2-(diméthylamino)éthyl]amino}-1,3-benzoxazole-4,7-dione et 2-(2,5-difluorophényl)-6-{[2-(diméthylamino)éthyl]amino}-1,3-benzoxazole-4,7-dione:

Le protocole expérimental utilisé est identique à celui décrit pour l'exemple 68, le 2,5-difluorobenzonitrile remplaçant le 2-bromobenzonitrile.
Les deux composants du mélange peuvent être caractérisés par les déplacements RMN (400 MHz) du seul proton du cycle benzoxazoledione qui sont 5,41 et 5,43 ppm.
SM-CL : MH+ = 348,0 ; t.r. = 7,7 min.

### Exemple 84 : mélange de 2-(2,3-difluorophényl)-5-{[2-(diméthylamino)éthyl]amino}-1,3-benzoxazole-4,7-dione et 2-(2,3-difluorophényl)-6-{[2-(diméthylamino)éthyl]amino}-1,3-benzoxazole-4,7-dione:

Le protocole expérimental utilisé est identique à celui décrit pour l'exemple 68, le 2,3-difluorobenzonitrile remplaçant le 2-bromobenzonitrile. Point de fusion : 167 °C. Les deux composants du mélange peuvent être caractérisés par les déplacements RMN (400 MHz) du seul proton du cycle benzoxazoledione qui sont 5,41 et 5,43 ppm.
SM-CL : MH+ = 348,1 ; t.r. = 7,8 min.

### Exemple 85 : mélange de 2-(2,3-difluorophényl)-5-[(2-pyrrolidin-1-yléthyl)amino]-1,3-benzoxazole-4,7-dione et 2-(2,3-difluorophényl)-6-[(2-pyrrolidin-1-yléthyl)amino]-1,3-benzoxazole-4,7-dione:

Le protocole expérimental utilisé est identique à celui décrit pour l'exemple 68, le 2,3-difluorobenzonitrile remplaçant le 2-bromobenzonitrile et la *N*-(2-aminoéthyl)-pyrrolidine remplaçant la *N,N*-diméthyléthylènediamine. Point de fusion: 150 °C.
Les deux composants du mélange peuvent être caractérisés par les déplacements RMN (400 MHz) du seul proton du cycle benzoxazoledione qui sont 5,40 et 5,42 ppm.
SM-CL : MH+ = 374,1 ; t.r. = 7,9 min.

### Exemple 86 : mélange de 2-(2,3-difluorophényl)-5-{[3-(diméthylamino)propyl]amino}-1,3-benzoxazole-4,7-dione et 2-(2,3-difluorophényl)-6-{[3-(diméthylamino)propyl]amino}-1,3-benzoxazole-4,7-dione:

Le protocole expérimental utilisé est identique à celui décrit pour l'exemple 68, le 2,3-difluorobenzonitrile étant employé au lieu du 2-bromobenzonitrile, et la *N,N*-diméthylpropylènediamine remplaçant la *N,N*-diméthyléthylènediamine. Point de fusion: 169 °C.
Les deux composants du mélange peuvent être caractérisés par les déplacements RMN (400 MHz) du seul proton du cycle benzoxazoledione qui sont 5,38 et 5,41 ppm.
SM-CL : MH+ = 362,1 ; t.r. = 7,8 min.

### Exemple 87 : mélange de 5-[(2-pyrrolidin-1-yléthyl)amino]-2-(3,4,5-trifluorophényl)-1,3-benzoxazole-4,7-dione et 6-[(2-pyrrolidin-1-yléthyl)amino]-2-(3,4,5-trifluorophényl)-1,3-benzoxazole-4,7-dione :

Le protocole expérimental utilisé est identique à celui décrit pour l'exemple 68, le 3,4,5-trifluorobenzonitrile remplaçant le 2-bromobenzonitrile, et la *N*-(2-aminoéthyl)-pyrrolidine remplaçant la *N,N*-diméthyléthylènediamine.
Les deux composants du mélange peuvent être caractérisés par les déplacements RMN (400 MHz) du seul proton du cycle benzoxazoledione qui sont 5,39 et 5,41 ppm.
SM-CL : MH+ = 392,0 ; t.r. = 8,2 min.

### Exemple 88 : mélange de 5-{[2-(diméthylamino)éthyl]amino}-2-(3,4,5-trifluorophényl)-1,3-benzoxazole-4,7-dione et 6-{[2-(diméthylamino)éthyl]amino}-2-(3,4,5-trifluorophényl)-1,3-benzoxazole-4,7-dione:

Le protocole expérimental utilisé est identique à celui décrit pour l'exemple 68, le 3,4,5-trifluorobenzonitrile remplaçant le 2-bromobenzonitrile.
Les deux composants du mélange peuvent être caractérisés par les déplacements RMN (400 MHz) du seul proton du cycle benzoxazoledione qui sont 5,40 et 5,42 ppm.
SM-CL : MH+ = 366,1 ; t.r. = 8,1 min.

### Exemple 89 : mélange de 5-[(2-pyrrolidin-1-yléthyl)amino]-2-(2,3,4,5-tétrafluorophényl)-1,3-benzoxazole-4,7-dione et 6-[(2-pyrrolidin-1-yléthyl)amino]-2-(2,3,4,5-tétrafluorophényl)-1,3-benzoxazole-4,7-dione:

Le protocole expérimental utilisé est identique à celui décrit pour l'exemple 68, le 2,3,4,5-tétrafluorobenzonitrile remplaçant le 2-bromobenzonitrile et la *N*-(2-aminoéthyl)-pyrrolidine remplaçant la *N,N*-diméthyléthylènediamine.
Les deux composants du mélange peuvent être caractérisés par les déplacements RMN (400 MHz) du seul proton du cycle benzoxazoledione qui sont 5,42 et 5,44 ppm.
SM-CL : MH+ = 410,0 ; t.r. = 8,2 min.

### Exemple 90 : mélange de 5-{[2-(diméthylamino)éthyl]amino}-2-(2,3,4,5-tétrafluorophényl)-1,3-benzoxazole-4,7-dione et 6-{[2-(diméthylamino)éthyl]amino}-2-(2,3,4,5-tétrafluorophényl)-1,3-benzoxazole-4,7-dione:

Le protocole expérimental utilisé est identique à celui décrit pour l'exemple 68, le 2,3,4,5-tétrafluorobenzonitrile remplaçant le 2-bromobenzonitrile. Point de fusion : 160 °C.
Les deux composants du mélange peuvent être caractérisés par les déplacements RMN (400 MHz) du seul proton du cycle benzoxazoledione qui sont 5,42 et 5,45 ppm.
SM-CL : MH+ = 3 84,0 ; t.r. = 8,1 min.

### Exemple 91 : mélange de 5-{[2-(diméthylamino)éthyl]amino}-2-[2-fluoro-6-(trifluorométhyl)phényl]-1,3-benzoxazole-4,7-dione et 6-{[2-(diméthylamino)éthyl]amino}-2-[2-fluoro-6-(trifluorométhyl)phényl]-1,3-benzoxazole-4,7-dione:

Le protocole expérimental utilisé est identique à celui décrit pour l'exemple 68, le 2-fluoro-6-(trifluorométhyl)-benzonitrile remplaçant le 2-bromobenzonitrile.
Les deux composants du mélange peuvent être caractérisés par les déplacements RMN (400 MHz) du seul proton du cycle benzoxazoledione qui sont 5,44 et 5,46 ppm.
SM-CL : MH+ = 398,0 ; t.r. = 8,0 min.

### Exemple 92 : mélange de 2-[2-fluoro-6-(trifluorométhyl)phényl]-5-[(2-pyrrolidin-1-yléthyl)amino]-1,3-benzoxazole-4,7-dione et 2-[2-fluoro-6-(trifluorométhyl)phényl]-6-[(2-pyrrolidin-1-yléthyl)amino]-1,3-benzoxazole-4,7-dione:

Le protocole expérimental utilisé est identique à celui décrit pour l'exemple 68, le 2-fluoro-6-(trifluorométhyl)-benzonitrile remplaçant le 2-bromobenzonitrile et la *N*-(2-aminoéthyl)-pyrrolidine remplaçant la *N,N*-diméthyléthylènediamine. Point de fusion : 166 °C.
Les deux composants du mélange peuvent être caractérisés par les déplacements RMN (400 MHz) du seul proton du cycle benzoxazoledione qui sont 5,43 et 5,45 ppm.
SM-CL : MH+ = 424,1 ; t.r. = 8,1 min.

### Exemple 93 : mélange de 5-{[3-(diméthylamino)propyl]amino}-2-[2-fluoro-6-(trifluorométhyl)phényl]-1,3-benzoxazole-4,7-dione et 6-{[3-(diméthylamino)propyl]amino}-2-[2-fluoro-6-(trifluorométhyl)phényl]-1,3-benzoxazole-4,7-dione:

Le protocole expérimental utilisé est identique à celui décrit pour l'exemple 68, le 2-fluoro-6-(trifluoromethyl)-benzonitrile remplaçant le 2-bromobenzonitrile et la *N,N*-diméthylpropylènediamine remplaçant la *N,N*-diméthyléthylènediamine. Point de fusion : 128 °C.
Les deux composants du mélange peuvent être caractérisés par les déplacements RMN (400 MHz) du seul proton du cycle benzoxazoledione qui sont 5,42 et 5,43 ppm.
SM-CL : MH+ = 412,0 ; t.r. = 8,0 min.

### Exemple 94 : mélange de 2-[2-chloro-5-(trifluorométhyl)phényl]-5-{[2-(diméthylamino)éthyl]amino}-1,3-benzoxazole-4,7-dione et 2-[2-chloro-5-(trifluorométhyl)phényl]-6-{[2-(diméthylamino)éthyl]amino}-1,3-benzoxazole-4,7-dione:

Le protocole expérimental utilisé est identique à celui décrit pour l'exemple 68, le 2-chloro-5-(trifluorométhyl)-benzonitrile remplaçant le 2-bromobenzonitrile. Point de fusion : 182 °C.
Les deux composants du mélange peuvent être caractérisés par les déplacements RMN (400 MHz) du seul proton du cycle benzoxazoledione qui sont 5,43 et 5,46 ppm.
SM-CL : MH+ = 414,0 ; t.r. = 8,3 min.

### Exemple 95 : mélange de 2-[2-chloro-5-(trifluorométhyl)phényl]-5-[(2-pyrrolidin-1-yléthyl)amino]-1,3-benzoxazole-4,7-dione et 2-[2-chloro-5-(trifluorométhyl)phényl]-6-[(2-pyrrolidin-1-yléthyl)amino]-1,3-benzoxazole-4,7-dione:

Le protocole expérimental utilisé est identique à celui décrit pour l'exemple 68, le 2-chloro-5-(trifluoromethyl)-benzonitrile remplaçant le 2-bromobenzonitrile et la *N*-(2-aminoéthyl)-pyrrolidine remplaçant la *N,N*-diméthyléthylènediamine. Point de fusion : 152 °C.
Les deux composants du mélange peuvent être caractérisés par les déplacements RMN (400 MHz) du seul proton du cycle benzoxazoledione qui sont 5,43 et 5,45 ppm.
SM-CL : MH+ = 440,0 ; t.r. = 8,5 min.

### Exemple 96 : mélange de 2-[2-chloro-5-(trifluorométhyl)phényl]-5-{[3-(diméthylamino)propyl]amino}-1,3-benzoxazole-4,7-dione et 2-[2-chloro-5-(trifluorométhyl)phényl]-6-{[3-(diméthylamino)propyl]amino}-1,3-benzoxazole-4,7-dione:

Le protocole expérimental utilisé est identique à celui décrit pour l'exemple 68, le 2-chloro-5-(trifluorométhyl)-benzonitrile remplaçant le 2-bromobenzonitrile et la *N,N*-diméthylpropylènediamine remplaçant la *N,N*-diméthyléthylènediamine. Point de fusion : 121 °C.
Les deux composants du mélange peuvent être caractérisés par les déplacements RMN (400 MHz) du seul proton du cycle benzoxazoledione qui sont 5,41 et 5,43 ppm.
SM-CL : MH+ = 428,0 ; t.r. = 8,4 min.

### Exemple 97 : mélange de 2-[2-chloro-6-fluorophényl]-5-{[3-(diméthylamino)propyl]amino}-1,3-benzoxazole-4,7-dione et 2-[2-chloro-6-fluorophényl]-6-{[3-(diméthylamino)propyl]amino}-1,3-benzoxazole-4,7-dione:

Le protocole expérimental utilisé est identique à celui décrit pour l'exemple 68, le 2-chloro-6-fluorobenzonitrile remplaçant le 2-bromobenzonitrile.
Les deux composants du mélange peuvent être caractérisés par les déplacements RMN (400 MHz) du seul proton du cycle benzoxazoledione qui sont 5,43 et 5,45 ppm.
SM-CL : MH+ = 364,1 ; t.r. = 7,8 min.

### Exemple 98 : mélange de 2-[2-chloro-6-fluorophényl]-5-[(2-pyrrolidin-1-yléthyl)amino]-1,3-benzoxazole-4,7-dione et 2-[2-chloro-6-fluorophényl]-6-[(2-pyrrolidin-1-yléthyl)amino]-1,3-benzoxazole-4,7-dione:

Le protocole expérimental utilisé est identique à celui décrit pour l'exemple 68, le 2-chloro-6-fluorobenzonitrile remplaçant le 2-bromobenzonitrile et la *N*-(2-aminoéthyl)-pyrrolidine remplaçant la *N,N*-diméthyléthylènediamine. Point de fusion : 124 °C.
Les deux composants du mélange peuvent être caractérisés par les déplacements RMN (400 MHz) du seul proton du cycle benzoxazoledione qui sont 5,42 et 5,44 ppm.
SM-CL : MH+ = 390,1 ; t.r. = 7,9 min.

### Exemple 99 : mélange de 2-[3,4-diméthoxyphényl]-5-{[2-(diméthylamino)éthyl]amino}-1,3-benzoxazole-4,7-dione et 2-[3,4-diméthoxyphenyl]-6-{[2-(diméthylamino)éthyl]amino}-1,3-benzoxazole-4,7-dione:

### 99.1) 2-(3,4-diméthoxyphényl)-6,7-dihydro-1,3-benzoxazol-4(5H)-one :

Le protocole expérimental utilisé est identique à celui décrit pour l'étape 66.2, le 3,4-diméthoxybenzonitrile remplaçant le 2-fluorobenzonitrile. On obtient un solide jaune.
SM-CL : MH+ = 274,0 ; t.r. = 8,9 min.

### 99.2) 5-iodo-2-(3,4-diméthoxyphényl)-6,7-dilhydro-1,3-benzoxazol-4(5H)-one :

A une solution de l'intermédiaire 99.1 (500 mg, 1,83 mmol) dans l'acide acétique (30 ml) est traitée pendant 96 h à température ambiante par du poly[styrène-*co-*(4-vinylpyridinium dichloroiodate(1-))] (2,6 g ; 8,25 mEq ; préparé selon B 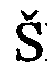 ket et coll., *Bull. Chem. Soc. Jpn* (1989), **62,** 3406-3408) (contrôle CCM sur SiO₂: 2% MeOH/CH₂Cl₂). Le polymère est retiré par filtration et les volatiles sont évaporés sous pression réduite. Le résidu est purifié sur colonne (SiO₂: 1% MeOH/CH₂Cl₂) pour donner une huile jaune.
SM-CL : MH+ = 399,9 ; t.r. = 9,8 min.

### 99.3) Mélange de 2-(3,4-diméthoxyphényl)-5-{[2-(diméthylamino)éthyl]amino}-1,3-benzoxazole-4,7-dione et 2-(3,4-diméthoxyphényl)-6-{[2-(diméthylamino)éthyl]amino}-1,3-benzoxazole-4,7-dione :

Ce composé est obtenu à partir de l'intermédiaire 99.2 selon les modes opératoires décrits pour les étapes 66.4, 66.5 et 66.6. Point de fusion : 181 °C.
Les deux composants du mélange peuvent être caractérisés par les déplacements RMN (400 MHz) du seul proton du cycle benzoxazoledione qui sont 5,35 et 5,36 ppm.
SM-CL : MH+ = 372,1 ; t.r. = 7,6 min.

### Exemple 100 : mélange de 2-[2-bromo-3-pyridyl]-5-{[2-(diméthylamino)éthyl]amino}-1,3-benzoxazole-4,7-dione et 2-[2-bromo-3-pyridyl]-6-{[2-(diméthylamino)éthyl]amino}-1,3-benzoxazole-4,7-dione:

Le protocole expérimental utilisé est identique à celui décrit pour l'exemple 68, le 2-bromonicotinonitrile remplaçant le 2-bromobenzonitrile. Point de fusion : 133 °C.
Les deux composants du mélange peuvent être caractérisés par les déplacements RMN (400 MHz) du seul proton du cycle benzoxazoledione qui sont 5,43 et 5,45 ppm.
SM-CL : MH+ = 391,0 ; t.r. = 7,4 min.

### Exemple 101 : mélange de 2-cyclohexyl-5-[(2-pyrrolidin-1-yléthyl)amino]-1,3-benzothiazole-4,7-dione et 2-cyclohexyl-6-[(2-pyrrolidin-1-yléthyl)amino]-1,3-benzothiazole-4,7-dione :

### 101.1) N-(2,5-diméthoxyphényl)cyclohexanecarboxamide :

A une solution de 1,05 g (6,89 mmol) de 2,5-diméthoxyaniline dans 10 ml d'un mélange toluène/méthanol (1/1) est ajouté 1 ml (7,62 mmol, 1,1 éq.) de chlorure d'acide cyclohexanoïque. Le mélange réactionnel est maintenu sous agitation à 70 °C pendant 1,5 heure, et, après retour à température ambiante, est versé sur 50 ml d'eau. Le produit attendu est extrait par 2 fois avec 50 ml de toluène, puis lavé 2 fois avec 50 ml d'eau. Les phases organiques sont réunies, séchées sur sulfate de magnésium et le solvant évaporé sous pression réduite. 1,46 g (rendement = 67%) de *N*-(2,5-diméthoxyphényl)cyclohexanecarboxamide sont obtenus et utilisés sans autre purification dans l'étape suivante.
RMN ¹H (DMSO d6, 400 MHz, δ): 8,84 (s, 1H, NH); 7,72-7,71 (m, 1H, H arom.) ; 6,93-6,91 (d, 1H, H arom.); 6,60-6,57 (m, 1H, H arom.); 3,76 (s, 3H, CH₃); 3,66 (s, 3H, CH₃); 1,78-1,70 (m, 6H, CH₂, CH) ; 1,38-1,24 (m, 5H, CH₂).
SM-CL : MH+ = 264,14 ; t.r. = 10,76 min.

### 101.2) N-(2,5-diméthoxyphényl)cyclohexanecarbothioamide :

1,46 g (5,54 mmol) de *N*-(2,5-diméthoxyphényl)cyclohexanecarboxamide est mis en solution dans 40 ml de toluène anhydre. La solution est portée à 100 °C, et 3,34 g (8,26 mmol ; 1,5 éq.) de réactif de Lawesson sont ajoutés au milieu réactionnel qui est ensuite maintenu sous agitation à 100 °C pendant 4 heures. Après retour à température ambiante, la solution est versée sur 50 ml d'eau glacée et extraite à l'aide de toluène. Les phases organiques sont séchées sur sulfate de magnésium et le solvant évaporé. Le *N*-(2,5-diméthoxyphenyl)cylohexanecarbothioamide est ensuite purifié par chromatographie sur colonne de silice (éluant : dichlorométhane/heptane: 1/1 puis 3/2). 1,26 g (rendement = 81 %) de produit sont obtenus sous forme d'huile jaune.
RMN ¹H (DMSO d6, 400 MHz, δ): 10,76 (s, 1H, NH) ; 7,28-7,27 (m, 1H, H arom.) ; 7,02-6,99 (d, 1H, H arom.) ; 6,82-6,80 (m, 1H, H arom.) ; 3,73 (s, 3H, CH₃); 3,68 (s, 3H, CH₃); 1,77-1,75 (m, 4H, CH₂); 1,67-1,58 (m, 3H, CH₂, CH); 1,31-1,15 (m, 4H, 2CH₂).
SM-CL : MH+ = 280,12 ; t.r. =11,38 min.

### 101.3) 2-cyclohexyl-4,7-diméthoxy-1,3-benzothiazole :

1,26 g (4,50 mmol) de *N*-(2,5-diméthoxyphényl)cyclohexanecarbothioamide sont dissous dans 100 ml d'une solution d'hydroxyde de sodium à 1,5 M (100 ml) et le milieu réactionnel est refroidi à 0 °C avant d'ajouter 25 ml d'une solution aqueuse fraîchement préparée de ferricyanure de potassium à 20 % (5,05 g de K₃[Fe(CN)₆]; 3,4 éq.). Le mélange réactionnel est maintenu sous agitation à température ambiante pendant 24 heures, puis 1,1 g (rendement = 88 %) du dérivé de benzothiazole attendu est obtenu par filtration, lavage à l'eau froide et séchage sous pression réduite en présence de P₂O₅.
RMN ¹H (DMSO d6, 400 MHz, δ): 6,95-6,85 (dd, 2H, H arom.) ; 3,88 (s, 6H, 2CH₃); 3,10-3,04 (m, 1H, CH); 2,10-2,07 (m, 2H, CH₂); 1,81-1,77 (m, 2H, CH₂); 1,70-1,67 (m, 1H, CH); 1,57-1,51 (m, 2H, CH₂); 1,42-1,39 (m, 2H, CH₂); 1,26-1,28 (m, 1H, CH).
SM-CL : MH+ = 278,09 ; t.r. = 11,91 min.

### 101.4) 2-cyclohexyl-1,3-benzothiazole-4,7-dione :

1 g (3,61 mmol) de 2-cyclohexyl-4,7-diméthoxy-1,3-benzothiazole est mis en suspension dans un mélange acétonitrile/eau (3/1) à 0 °C puis 4,36 g (7,96 mmol ; 2,2 éq.) de nitrate de cérium (IV) et d'ammonium sont ajoutés à la suspension. Le mélange réactionnel est maintenu 1,5 heure sous agitation à température ambiante, puis 0,78 g (rendement = 88 %) de 2-cyclohexyl-1,3-benzothiazole-4,7-dione est obtenu après filtration, lavage à l'eau froide et séchage sous pression réduite.
RMN ¹H (DMSO d6, 400 MHz, δ): 6,90 (s, 2H) ; 3,15-3,10 (m, 1H, CH); 2,10-2,07 (m, 2H, CH₂); 1,81-1,77 (m, 2H, CH₂); 1,65-1,70 (m, 1H, CH); 1,55-1,39 (m, 5H, CH, CH₂).
SM-CL : MH+ = 248,12 ; t.r. = 10,82 min.

### 101.5) N-(2,5-diméthoxyphényl)cyclohexanecarboxamide :

Le protocole expérimental utilisé est identique à celui décrit pour l'étape 15.4 de l'exemple 15, l'intermédiaire 101.4 remplaçant l'intermédiaire 15.3 et la *N*,*N*-diméthyléthylène diamine remplaçant l'aniline. Un mélange de 80 % et 9 % de 2-cyclohexyl-5-{[2-(diméthylamino)éthyl]amino}-1,3-benzothiazole-4,7-dione et de 2-cyclohexyl-6-{[2-(diméthylamino)éthyl]amino}-1,3-benzothiazole-4,7-dione est obtenu.
RMN ¹H (DMSO d6, 400 MHz, δ): 7,20 (t, 1H, NH); 5,49 et 5,43 (2s, H) ; 3,24-3,21 (m, 2H, CH₂); 3,09-3,12 (m, 3H, CH, CH₂); 2,19 (s, 6H, 2CH₃); 2,09-2,06 (m, 2H, CH₂); 1,80-1,77 (m, 3H, CH, CH₂); 1,53-1,49 (m, 4H, 2CH₂); 1,41-1,38 (m, 1H, CH).
SM-CL : MH+ = 334,17 ; t.r. = 7,99 et 8,06 min.

*Les composés des exemples 102 à 113 sont obtenus de façon analogue à celle décrite pour l'exemple 101.*

### Exemple 102 : mélange de 2-cyclohexyl-5-[(2-pyrrolidin-1-yléthyl)amino]-1,3-benzothiazole-4,7-dione et 2-cyclohexyl-6-[(2-pyrrolidin-1-yléthyl)amino]-1,3-benzothiazole-4,7-dione:

SM-CL : MH+ = 3 60,16 ; t.r. = 8,14 et 8,19 min.

### Exemple 103 : mélange de 5-[(2-pyrrolidin-1-yléthyl)amino]-2-thién-2-yl-1,3-benzothiazole-4,7-dione et 6-[(2-pyrrolidin-1-yléthyl)amino]-2-thién-2-yl-1,3-benzothiazole-4,7-dione:

SM-CL : MH+ = 360,01 ; t.r. = 7,78 et 7,86 min.

### Exemple 104 : mélange de 2-(2,5-dichlorothién-3-yl)-5-{[2-(diméthylamino)éthyl]amino}-1,3-benzothiazole-4,7-dione et 2-(2,5-dichlorothién-3-yl)-6-{[2-(diméthylamino)éthyl]amino}-1,3-benzothiazole-4,7-dione:

SM-CL : MH+ = 401,86 ; t.r. = 8,44 et 8,59 min.

### Exemple 105: mélange de 2-(2,5-dichlorothién-3-yl)-5-[(2-pyrrolidin-1-yléthyl)amino]-1,3-benzothiazole-4,7-dione et 2-(2,5-dichlorothién-3-yl)-6-[(2-pyrrolidin-1-yléthyl)amino]-1,3-benzothiazole-4,7-dione:

SM-CL : MH+ = 427,87 ; t.r. = 8,63 et 8,80 min.

### Exemple 106: mélange de 2-(2-furyl)-5-[(2-pyrrolidin-1-yléthyl)amino]-1,3-benzothiazole-4,7-dione et 2-(2-furyl)-6-[(2-pyrrolidin-1-yléthyl)amino]-1,3-benzothiazole-4,7-dione:

SM-CL: MH+ = 344,04 ; t.r. = 7,57 et 7,64 min.

### Exemple 107 : mélange de 5-{[2-(diméthylamino)éthyl]amino}-2-(2-méthoxyphényl)-1,3-benzothiazole-4,7-dione et 6-{[2-(diméthylamino)éthyl]amino}-2-(2-méthoxyphényl)-1,3-benzothiazole-4,7-dione:

SM-CL : MH+ = 358,18 ; t.r. = 7,88 et 7,97 min.

### Exemple 108 : mélange de 5-{[2-(diméthylamino)éthyl]amino}-2-(2-fluorophényl)-1,3-benzothiazole-4,7-dione et 6-{[2-(diméthylamino)éthyl]amino}-2-(2-fluorophényl)-1,3-benzothiazole-4,7-dione :

SM-CL : MH+ = 346,14 ; t.r. = 7,85 et 7,94 min.

### Exemple 109 : mélange de 2-(2-fluorophényl)-5-[(2-pyrrolidin-1-yléthyl)amino]-1,3-benzothiazole-4,7-dione et 2-(2-fluorophényl)-6-[(2-pyrrolidin-1-yléthyl)amino]-1,3-benzothiazole-4,7-dione :

SM-CL : MH+ = 372,14 ; t.r. = 7,97 et 8,06 min.

### Exemple 110 : mélange de 2-(4-fluorophényl)-5-[(2-pyrrolidin-1-yléthyl)amino]-1,3-benzothiazole-4,7-dione et 2-(4-fluorophényl)-6-[(2-pyrrolidin-1-yléthyl)amino]-1,3-benzothiazole-4,7-dione :

SM-CL : MH+ = 372,05 ; t.r. = 7,98 et 8,07 min.

### Exemple 111 : mélange de 5-{[2-(diméthylamino)éthyl]amino}-2-(4-fluorophényl)-1,3-benzothiazole-4,7-dione et 6-{[2-(diméthylamino)éthyl]amino}-2-(4-fluorophényl)-1,3-benzothiazole-4,7-dione :

SM-CL : MH+ = 346,05 ; t.r. = 7,87 et 7,95 min.

### Exemple 112 : mélange de 2-(2,6-difluorophényl)-5-[(2-pyrrolidin-1-yléthyl)amino]-1,3-benzothiazole-4,7-dione et 2-(2,6-difluorophényl)-6-[(2-pyrrolidin-1-yléthyl)amino]-1,3-benzothiazole-4,7-dione :

SM-CL : MH+ = 390,04 ; t.r. = 7,89 et 7,95 min.

### Exemple 113 : mélange de 2-(2,6-difluorophényl)-5-{[2-(diméthylamino)éthyl]amino}-1,3-benzothiazole-4,7-dione et 2-(2,6-difluorophényl)-6-{[2-(diméthylamino)éthyl]amino}-1,3-benzothiazole-4,7-dione :

SM-CL : MH+ = 364,05 ; t.r. = 7,78 et 7,83 min.

### Exemple 114 : 5-[[2-(diméthylamino)éthyl](éthyl)amino]-2-méthyl-1,3-benzothiazole-4,7-dione :

Ce composé est obtenu de façon analogue à celle décrite pour l'exemple 1, la *N,N,N'*-triméthyléthylènediamine remplaçant la 4-(2-aminoéthyl)morpholine.
RMN ¹H (DMSÔ d6, 400 MHz, δ): 5,53 (s, 1H, CH); 3,73-3,70 (t, 2H, CH₂); 2,93 (s, 3H, CH₃); 2,74 (s, 3H, CH₃) ; 2,32-2,30 (t, 2H, CH₂); 1,92 (s, 6H, 2CH₃).
SM-CL : MH+ = 280,11 ; t.r. = 7,03 min.

### Exemple 115 : 5-[[2-(diméthylamino)éthyl](méthyl)amino]-2-méthyl-1,3-benzothiazole-4,7-dione :

Ce composé est obtenu de façon analogue à celle décrite pour l'exemple 1, la *N,N*-diméthyl-*N'*-éthylènediamine remplaçant la 4-(2-aminoéthyl)morpholine.
SM-CL : MH+ = 294,07 ; t.r. = 7,20 min.

### Exemple 116 : mélange de 2-[2,6-dichloro-5-fluoro-3-pyridyl]-5-{[2-(diméthylamino)éthyl]amino}-1,3-benzoxazole-4,7-dione et 2-[2,6-dichloro-5-fluoro-3-pyridyl]-6-{[2-(diméthylamino)éthyl]amino}-1,3-benzoxazole-4,7-dione :

Le protocole expérimental utilisé est identique à celui décrit pour l'exemple 68, le 2,6-dichloro-5-fluoronicotinonitrile remplaçant le 2-bromobenzonitrile.
SM-CL : MH+ = 3 99,1 ; t.r. = 8,1 min.

### Exemple 117 : mélange de 2-[2,6-dichloro-5-fluoro-3-pyridyl]-5-[(2-pyrrolidin-1-yléthyl)amino]-1,3-benzoxazole-4,7-dione et 2-[2,6-dichloro-5-fluoro-3-pyridyl]-6-[(2-pyrrolidin-1-yléthyl)amino]-1,3-benzoxazole-4,7-dione :

Le protocole expérimental utilisé est identique à celui décrit pour l'exemple 68, le 2,6-dichloro-5-fluoronicotinonitrile remplaçant le 2-bromobenzonitrile et la *N*-(2-aminoéthyl)-pyrrolidine remplaçant la *N,N*-diméthyléthylènediamine.
SM-CL : MH+ = 399,1 ; t.r. = 8,1 min.

### Exemple 118 : mélange de 2-(2,4-difluorophényl)-5-{[2-(diméthylamino)éthyl]amino}-1,3-benzoxazole-4,7-dione et 2-(2,4-difluorophényl)-6-{[2-(diméthylamino)éthyl]amino}-1,3-benzoxazole-4,7-dione :

Le protocole expérimental utilisé est identique à celui décrit pour l'exemple 68, le 2,4-difluorobenzonitrile remplaçant le 2-bromobenzonitrile.
SM-CL : MH+ = 348,1 ; t.r. = 7,8 min.

### Exemple 119 : mélange de 5-{[2-(diméthylamino)éthyl]amino}-2-(2,3,4-trifluorophényl)-1,3-benzoxazole-4,7-dione et 6-{[2-(diméthylamino)éthyl]amino}-2-(2,3,4-trifluorophényl)-1,3-benzoxazole-4,7-dione :

Le protocole expérimental utilisé est identique à celui décrit pour l'exemple 68, le 2,3,4-trifluorobenzonitrile remplaçant le 2-bromobenzonitrile. Point de fusion : 156 °C.
SM-CL : MH+ = 366,1 ; t.r. = 8,0 min.

### Exemple 120 : mélange de 5-[(2-pyrrolidin-1-yléthyl)amino]-2-(2,3,4-trifluorophényl)-1,3-benzoxazole-4,7-dione et 6-[(2-pyrrolidin-1-yléthyl)amino]-2-(2,3,4-trifluorophényl)-1,3-benzoxazole-4,7-dione :

Le protocole expérimental utilisé est identique à celui décrit pour l'exemple 68, le 2,3,4-trifluorobenzonitrile remplaçant le 2-bromobenzonitrile et la *N*-(2-aminoéthyl)-pyrrolidine remplaçant la *N,N*-diméthyléthylènediamine.
SM-CL : MH+ = 392,1 ; t.r. = 8,1 min.

### Exemple 121 : mélange de 2-(3-fluoro-4-méthylphényl)-5-{[2-(diméthylamino)éthyl]amino}-1,3-benzoxazole-4,7-dione et 2-(3-fluoro-4-méthylphényl)-6-{[2-(diméthylamino)éthyl]amino}-1,3-benzoxazole-4,7-dione :

Le protocole expérimental utilisé est identique à celui décrit pour l'exemple 68, le 3-fluoro-4-méthylbenzonitrile remplaçant le 2-bromobenzonitrile. Point de fusion : 179 °C.
SM-CL : MH+ = 344,1 ; t.r. = 8,1 min.

### Exemple 122 : mélange de 2-(3-fluoro-4-méthylphényl)-5-[(2-pyrrolidin-1-yléthyl)amino]-1,3-benzoxazole-4,7-dione et 2-(3-fluoro-4-méthylphényl)-6-[(2-pyrrolidin-1-yléthyl)amino]-1,3-benzoxazole-4,7-dione :

Le protocole expérimental utilisé est identique à celui décrit pour l'exemple 68, le 3-fluoro-4-méthylbenzonitrile remplaçant le 2-bromobenzonitrile et la *N*-(2-aminoéthyl)-pyrrolidine remplaçant la *N,N*-diméthyléthylènediamine.
SM-CL : MH+ = 370,1 ; t.r. = 8,2 min.

*Les composés des exemples 123 à 127 sont obtenus de façon analogue à celle décrite pour l'exemple 101*.

### Exemple 123 : mélange de 2-(4-chlorophényl)-5-{[2-(diméthylamino)éthyl]amino}-1,3-benzothiazole-4,7-dione et 2-(4-chlorophényl)-6-{[2-(diméthylamino)éthyl]amino}-1,3-benzothiazole-4,7-dione :

SM-CL : MH+ = 362,07 ; t.r. = 8,11 et 8,20 min.

### Exemple 124 : mélange de 2-(4-chlorophényl)-5-[(2-pyrrolidin-1-yléthyl)amino]-1,3-benzothiazole-4,7-dione et 2-(4-chlorophényl)-6-[(2-pyrrolidin-1-yléthyl)amino]-1,3-benzothiazole-4,7-dione :

SM-CL : MH+ = 388,04 ; t.r. = 8,23 et 8,34 min.

### Exemple 125 : mélange de 5-{[2-(diméthylamino)éthyl]amino}-2-(2,3,4,5-tétrafluorophényl)-1,3-benzothiazole-4,7-dione et 6-{[2-(diméthylamino)éthyl]amino}-2-(2,3,4,5-tétrafluorophényl)-1,3-benzothiazole-4,7-dione :

SM-CL : MH+ = 400,01 ; t.r. = 8,23 et 8,32 min.

### Exemple 126 : mélange de 5-{[2-(diméthylamino)éthyl]amino}-2-(3,4,5-trifluorophényl)-1,3-benzothiazole-4,7-dione et 6-{[2-(diméthylamino)éthyl]amino}-2-(3,4,5-trifluorophényl)-1,3-benzothiazole-4,7-dione :

SM-CL : MH+ = 382,03 ; t.r. = 8,10 et 8,19 min.

### Exemple 127 : mélange de 5-[(2-pyrrolidin-1-yléthyl)amino]-2-(2,4,6-trifluorophényl)-1,3-benzothiazole-4,7-dione et 6-[(2-pyrrolidin-1-yléthyl)amino]-2-(2,4,6-trifluorophényl)-1,3-benzothiazole-4,7-dione :

SM-CL : MH+ = 408,02; t.r. = 7,97 et 8,05 min.

*Les composés des exemples 128 à 131 sont obtenus de façon analogue à celle décrite pour l'exemple 66.*

### Exemple 128 : mélange de 2-(1,3-benzodioxol-5-yl)-5-{[2-(diméthylamino)éthyl]amino}-1,3-benzoxazole-4,7-dione et de 2-(1,3-benzodioxol-5-yl)-6-{[2-(diméthylamino)éthyl]amino}-1,3-benzoxazole-4,7-dione :

Les deux composants du mélange peuvent être caractérisés par les déplacements RMN (400 MHz) du seul proton du cycle benzoxazoledione qui sont 5,35 et 5,37 ppm.
SM-CL : MH+ = 356,07 ; t.r. = 7,72 min.

### Exemple 129 : mélange de 5-{[2-(diméthylamino)éthyl]amino}-2-(4-éthylphényl)-1,3-benzoxazole-4,7-dione et de 6-{[2-(diméthylamino)éthyl]amino}-2-(4-éthylphényl)-1,3-benzoxazole-4,7-dione :

Les deux composants du mélange peuvent être caractérisés par les déplacements RMN (400 MHz) du seul proton du cycle benzoxazoledione qui sont 5,36 et 5,38 ppm.
SM-CL : MH+ = 340,18 ; t.r. = 8,24 min.

### Exemple 130 : mélange de 2-(4-éthylphényl)-5-[(2-pyrrolidin-1-yléthyl)amino]-1,3-benzoxazole-4,7-dione et 2-(4-éthylphényl)-6-[(2-pyrrolidin-1-yléthyl)amino]-1,3-benzoxazole-4,7-dione :

Les deux composants du mélange peuvent être caractérisés par les déplacements RMN (400 MHz) du seul proton du cycle benzoxazoledione qui sont 5,35 et 5,36 ppm.
SM-CL : MH+ = 366,15 ; t.r. = 8,34 min.

### Exemple 131 : mélange de 5-{[2-(diméthylamino)éthyl]amino}-2-(2-fluoro-6-méthoxyphényl)-1,3-benzoxazole-4,7-dione et 6-{[2-(diméthylamino)éthyl]amino}-2-(2-fluoro-6-méthoxyphényl)-1,3-benzoxazole-4,7-dione :

Les deux composants du mélange peuvent être caractérisés par les déplacements RMN (400 MHz) du seul proton du cycle benzoxazoledione qui sont 5,39 et 5,40 ppm.
SM-CL : MH+ = 360,09 ; t.r. = 7,67 min.

### Exemple 132 : 2-(2,6-difluorophényl)-5-{[2-(diméthylamino)éthyl]amino}-1,3-benzothiazole-4,7-dione :

### 132.1) N-(3,5-diméthoxyphényl)-2,6-difluorobenzamide :

5,5 ml (39,2 mmol; 1,2 équivalent) de triéthylamine et 4,5 ml (35,9 mmol; 1,1 équivalent) de chlorure de 2,6-difluorobenzoyle sont ajoutés à 5 g (32,6 mmol) de 3,5-diméthoxyaniline en solution dans 100 ml de toluène anhydre. Le milieu réactionnel est maintenu sous agitation à 70 °C pendant 1 h 30, puis, après retour à température ambiante, est lavé par 3 fois 50 ml d'eau. La phase organique résultante est séchée sur sulfate de magnésium puis le solvant est évaporé sous pression réduite. Le produit attendu est obtenu sous forme d'une poudre blanche (8,75 g; rendement = 97 %) utilisée dans l'étape suivante sans autre purification.
SM-CL : MH+ = 294,11 ; t.r. = 9,93 min.

### 132.2) N-(3,5-diméthoxyphényl)-2,6-difluorobenzenecarbothioamide :

A 9,8 g (33,4 mmol) de *N*-(3,5-diméthoxyphényl)-2,6-difluorobenzamide en solution dans 150 ml de toluène anhydre sont ajoutés 20,3 g (50 mmol ; 1,5 équivalents) de réactif de Lawesson. Le milieu réactionnel est maintenu sous agitation à 120 °C pendant 8 heures, puis, après retour à température ambiante, est lavé par 3 fois 75 ml d'eau. La phase organique résultante est séchée sur sulfate de magnésium puis le solvant est évaporé sous pression réduite. Le résidu est purifié par chromatographie sur colonne de silice (éluant : dichlorométhane/méthanol 98/2) et le produit attendu est obtenu sous forme d'une huile verte (10 g ; rendement = 96 %).
SM-CL : MH+ = 310,06 ; t.r. = 10,53 min.

### 132.3) 2-(2,6-difluorophényl)-5,7-diméthoxy-1,3-benzothiazole :

A 10,3 g (33,3 mmol) de *N*-(3,5-diméthoxyphényl)-2,6-difluorobenzenecarbothioamide dissous dans 150 ml d'une solution de soude à 1,5*M* sont ajoutés 170 ml (103 mmol ; 3 équivalents) d'une solution aqueuse fraîchement préparée de ferricyanure de potassium à 20%. Le milieu réactionnel est maintenu sous agitation à température ambiante pendant 24 heures, puis le précipité beige formé est filtré, lavé par de l'eau et séché (6,8 g ; rendement = 66%). Les eaux-mères peuvent être extraites par 3 fois 75 ml de dichlorométhane, puis les phases organiques lavées par une solution saturée en chlorure de sodium. Après concentration sous pression réduite, le résidu obtenu peut être purifié sur colonne de silice (éluant : acétate d'éthyle/heptane : 1/3) pour fournir 2 g supplémentaires de produit attendu (rendement global = 86%). Point de fusion : 136-138°C.
RMN ¹H (DMSO d6, 400 MHz, δ) : 7,65 (m, 1H, H arom.); 7,36-7,31 (m, 3H, H arom.) ; 6,75 (m, 1H, H arom.) ; 3,96 (s, 3H, CH₃) ; 3,87 (s, 3H, CH₃).
SM-CL : MH+ = 308,12 ; t.r. = 11,48 min.

### 132.4) 2-(2,6-difluorophényl)-5-méthoxy-1,3-benzothiazole-4,7-dione :

### 132.4.1) 2-(2,6-difluorophényl)-5,7-diméthoxy-4-nitro-1,3-benzothiazole :

A 3 g (9,76 mmol) de 2-(2,6-difluorophényl)-5,7-diméthoxy-1,3-benzothiazole en solution dans 75 ml d'acétate d'éthyle, est ajoutée goutte à goutte une solution de 16 g (29,3 mmol ; 3 équivalents) de nitrate de cérium et d'ammonium dans 40 ml d'eau. Le mélange réactionnel est maintenu sous agitation pendant 2 heures à température ambiante, puis lavé par 3 fois 20 ml d'eau. Les phases organiques sont séchées sur sulfate de magnésium, filtrées puis concentrées sous pression réduite. Le résidu est purifié par chromatographie sur colonne de silice (éluant : acétate d'éthyle/heptane : 3/7). Deux fractions sont séparées :
0,3 g (rendement = 10%) de 2-(2,6-difluorophényl)-5-méthoxy-1,3-benzothiazole-4,7-dione sont obtenus sous forme de poudre jaune.
   SM-CL : MH+ = 308,08 ; t.r. = 10 min.
1,5 g de 2-(2,6-difluorophényl)-5,7-diméthoxy-4-nitro-1,3-benzothiazole (rendement de 45%) sont obtenus sous forme de poudre orangée.
   RMN ¹H (DMSO d6, 400 MHz, δ) : 7,72 (m, 1H, H arom.) ; 7,38 (m, 2H, H arom.) ; 7,11 (m, 1H, H arom.) ; 4,12 (s, 3H, CH₃) ; 4,07 (s, 3H, CH₃).
   SM-CL : MH+ = 353,05 ; t.r. = 11,30 min.

### 132.4.2) 2-(2,6-difluorophényl)-5,7-diméthoxy-1,3-benzothiazol-4-amine :

230 mg (0,65 mmol) d'intermédiaire 132.4.1 mis en solution dans 15 ml d'acide chlorhydrique concentré sont mis en réaction avec 0,5 g (2,2 mmol ; 3,4 équivalents) de chlorure d'étain dihydraté dans 5 ml d'eau. Le mélange réactionnel est maintenu sous agitation pendant 2 heures à 50°C, puis après retour à température ambiante, est versé sur de la glace avant de le neutraliser avec une solution 5*M* de soude. Le produit est ensuite extrait par 3 fois 15 ml de dichlorométhane, les phases organiques sont réunies, lavées par une solution saturée en chlorure de sodium, séchées sur sulfate de magnésium, filtrées puis, après concentration sous pression réduite, le produit attendu est obtenu sous forme d'une huile jaune. Il sera utilisé dans l'étape suivante sans autre purification.
RMN ¹H (DMSO d6, 400 MHz, δ) : 7,67 (m, 1H, H arom.) ; 7,34 (m, 2H, H arom.) ; 6,92 (s, 1H, H arom.) ; 3,91 (s, 3H, CH₃) ; 3,90 (s, 3H, CH₃).
SM-CL : MH+= 323,10 ; t.r. = 9,86 min.

### 132.4.3) 2-(2,6-difluorophényl)-5-méthoxy-1,3-benzothiazole-4,7-dione :

A 343 mg (1,06 mmol) de 2-(2,6-difluorophényl)-5,7-diméthoxy-1,3-benzothiazol-4-amine en solution dans 25 ml d'acétate d'éthyle est ajoutée une solution de 1,22 g de cérium ammonium nitrate (2,23 mmol, 2,1 équivalents) dans 8 ml d'eau. Le mélange réactionnel est maintenu sous vigoureuse agitation à température ambiante pendant 1 heure 30 puis la phase organique est séparée et lavée par 3 fois 20 ml d'eau, puis séchée sur sulfate de magnésium, filtrée et le solvant est évaporé sous pression réduite. Le résidu est purifié par chromatographie sur colonne de silice (éluant : acétate d'éthyle/heptane : 3/7) et 280 mg (rendement = 86%) de 2-(2,6-difluorophényl)-5-méthoxy-1,3-benzothiazole-4,7-dione sont obtenus sous forme de poudre jaune.
RMN ¹H (DMSO d6, 400 MHz, δ) : 7,72 (m, 1H, H arom.) ; 7,39 (m, 2H, H arom.) ; 6,32 (s, 1H, CH) ; 3,88 (s, 3H, CH₃).
SM-CL : MH+ = 308,05 ; t.r. = 9,99 min.

### 132.5) 2-(2,6-difluonophényl)-5-{[2-(diméthylamino)éthyl]amino}-1,3-benzothiazole-4,7-dione :

104 ml (0,95 mmol; 1,5 équivalents) de N,N-diméthyléthylènediamine sont ajoutés à 195 mg de 2-(2,6-difluorophényl)-5-méthoxy-1,3-benzothiazole-4,7-dione en solution dans 20 ml d'éthanol anhydre. Le mélange réactionnel est agité à 70°C pendant 2 heures puis le solvant est évaporé sous pression réduite. Le résidu est purifié sur colonne de silice (éluant : méthanol à 5% dans du dichlorométhane). 130 mg (rendement = 57%) de composé attendu sont obtenus sous forme d'une poudre rouge.
RMN ¹H (DMSO d6, 400 MHz, δ) : 7,72 (m, 1H, H arom.) ; 7,52 (m, 1H, NH.) ; 7,38 (m, 2H, H arom.) ; 5,60 (s, 1H, CH) ; 3,28 (m, 2H, CH₂) ; 2,53 (m, 2H, CH₂) ; 2,20 (s, 6H, 2CH₃).
SM-CL : MH+ = 364,14 ; t.r. = 7,85 min.

*Les composés des exemples 133 à 138 sont obtenus de façon analogues à celle décrite pour l'exemple 132, les chlorures d'acyle adéquats remplaçant le chlorure de 2,6-difluorobenzoyle dans la première étape et la N-(2-aminoéthyl)pyrrolidine remplaçant la N,N-diméthyléthylènediamine dans la dernière étape pour les exemples 134, 136 et 138.*

### Exemple 133 : 2-(2,5-dichlorothién-3-yl)-5-{[2-(diméthylamino)éthyl]amino}-1,3-benzothiazole-4,7-dione :

### 133.1) 2,5-dichloro-N-(3,5-diméthoxyphényl)thiophène-3-carboxamide :

RMN ¹H (DMSO d6, 400 MHz, δ) : 10,20 (s, 1H, NH) ; 7,47 (s, 1H, H arom.) ; 6,95 (s, 1H, H arom.) ; 6,27 (s, 1H, H arom.) ; 3,72 (s, 6H, 2CH₃).
SM-CL : MH+ = 332,01 ; t.r. = 11,08 min.

### 133.2) 2,5-dichloro-N-(3,5-diméthoxyphényl)thiophène-3-carbothioamide :

RMN ¹H (DMSO d6, 400 MHz, δ) : 11,96 (s, 1H, NH) ; 7,30 (s, 1H, H arom.) ; 7,25 (s, 1H, H arom.) ; 6,44 (s, 1H, H arom.) ; 3,74 (s, 6H, 2CH₃).
SM-CL : MH+ = 348,00 ; t.r. = 11,55 min.

### 133.3) 2-(2,5-dichlorothién-3-yl)-5,7-diméthoxy-1,3-benzothiazole :

RMN ¹H (DMSO d6, 400 MHz, δ) : 7,72 (s, 1H, H arom.) ; 7,22 (s, 1H, H arom.) ; 6,73 (s, 1H, H arom.) ; 3,96 (s, 3H, CH₃) ; 3,86 (s, 3H, CH₃).
SM-CL : MH+ = 345,94 ; t.r. = 12,77 min.

### 133.4) 2-(2,5-dichlorothién-3-yl)-5-méthoxy-1,3-benzothiazole-4,7-dione :

RMN ¹H (DMSO d6, 400 MHz, δ) : 7,75 (s, 1H, H arom.) ; 6,31 (s, 1H, CH) ; 3,88 (s, 3H, CH₃).
SM-CL : MH+ = 345,98 ; t.r. = 11,52 min.

### 133.5) 2-(2,5-dichlorothién-3-yl)-5-{[2-(diméthylamino)éthyl]amino}-1,3-benzothiazole-4,7-dione :

RMN ¹H (DMSO d6, 400 MHz, δ) : 7,72 (s, 1H, H arom.) ; 7,51 (m, 1H, NH.) ; 5,58 (s, 1H, CH) ; 3,36 (m, 2H, CH₂) ; 2,54 (m, 2H, CH₂) ; 2,20 (s, 6H, 2CH₃).
SM-CL : MH+ = 402,06 ; t.r. = 8,42 min.

### Exemple 134 : 2-(2,5-dichlorothién-3-yl)-5-[(2-pyrrolidin-1-yléthyl)amino]-1,3-benzothiazole-4,7-dione :

SM-CL : MH+ = 427,97 ; t.r. = 8,70 min.

### Exemple 135 : 5-{[2-(diméthylamino)éthyl]amino}-2-(4-fluorophényl)-1,3-benzothiazole-4,7-dione :

135.1) *N-(3,5-diméthoxyphényl)-4-fluorobenzamide :*

RMN ¹H (DMSO d6, 400 MHz, δ) : 10,15 (s, 1H, NH) ; 8,01 (m, 2H, H arom.) ; 7,36 (m, 2H, H arom.); 7,05 (m, 2H, H arom.); 6,26 (s, 1H, H arom.); 3,73 (s, 6H, 2CH₃).
SM-CL : MH+ = 276,17 ; t.r. = 10,07 min.

### 135.2) N-(3,5-diméthoxyphénzyl)-4-fluorobenzenecarbothioamide :

SM-CL : MH+ = 292,17 ; t.r. = 10,72 min.

### 135.3) 2-(4-fluorophényl)-5,7-diméthoxy-1,3-benzothiazole :

RMN ¹H (DMSO d6, 400 MHz, δ) : 8,11 (m, 2H, H arom.) ; 7,40 (m, 2H, H arom.) ; 7,22 (s, 1H, H arom.) ; 6,69 (s, 1H, H arom.) ; 3,95 (s, 3H, CH₃) ; 3,86 (s, 3H, CH₃). SM-CL : MH+ = 290,07 ; t.r. = 11,93 min.

### 135.4) 2-(4-fluorophényl)-5-méthoxy-1,3-benzothiazole-4,7-dione :

RMN ¹H (DMSO d6, 400 MHz, δ) : 8,15 (m, 2H, H arom.) ; 7,42 (m, 2H, H arom.) ; 6,28 (s, 1H, CH) ; 3,87 (s, 3H, CH₃).
SM-CL : MH+ = 290,14 ; t.r. = 11,95 min.

### 135.5) 5-{[2-(diméthylamino)éthyl]ammo}-2-(4-fluorophényl)-1,3-benzothiazole-4,7-dione :

RMN ¹H (DMSO d6, 400 MHz, δ) : 8,11 (m, 2H, H arom.) ; 7,48 (m, 1H, NH) ; 7,41 (m, 2H, H arom.) ; 5,57 (s, 1H, CH) ; 3,26 (m, 2H, CH₂) ; 2,55 (m, 2H, CH₂) ; 2,22 (s, 6H, 2CH₃).
SM-CL : MH+ = 346,18 ; t.r. = 8,01 min.

### Exemple 136 : 2-(4-fluorophényl)-5-[(2-pyrrolidin-1-yléthyl)amino]-1,3-benzothiazole-4,7-dione :

RMN ¹H (DMSO d6, 400 MHz, δ) : 8,12 (m, 2H, H arom.) ; 7,58 (m, 1H, NH) ; 7,41 (m, 2H, H arom.) ; 5,55 (s, 1H, CH) ; 3,41 (m, 2H, CH₂) ; 2,69 (m, 2H, CH₂) ; 2,51 (m, 2H, CH₂) ; 2,44 (m, 2H, CH2) ; 1,70 (m, 4H, 2CH2).
SM-CL : MH+ = 372,19 ; t.r. = 8,12 min.

### Exemple 137 : 2-(2-chloro-6-fluorophényl)-5-{[2-(diméthylamino)éthyl]amino}-1,3-benzothiazole-4,7-dione :

### 137.1) 2-chloro-N-(3,5-diméthoxyphényl)-6-fluorobenzamide :

RMN ¹H (DMSO d6, 400 MHz, δ) : 10,69 (s, 1H, NH) ; 7,53 (m, 1H, H arom.) ; 7,43 (m, 1H, H arom.); 7,37 (m, 1H, H arom.); 6,93 (m, 2H, H arom.) ; 6,29 (s, 1H, H arom.) ; 3,72 (s, 6H, 2CH₃).
SM-CL : MH+ = 310,15 ; t.r. = 10,11 min.

### 137.2) 2-chloro-N-(3,5-diméthoxyphéhyl)-6-fluorobenzencarbothioamide :

RMN ¹H (DMSO d6, 400 MHz, δ) : 7,41 (m, 2H, H arom.) ; 7,27 (m, 3H, H arom.) ; 6,46 (s, 1H, H arom.) ; 3,75 (s, 6H, 2CH₃).
SM-CL : MH+ = 326,09 ; t.r. = 10,73 min.

### 137.3) 2-(2-chloro-6-fluorophényl)-5,7-diméthoxy-1,3-benzothiazole :

RMN ¹H (DMSO d6, 400 MHz, δ) : 7,66 (m, 1H, H arom.) ; 7,56 (m, 1H, H arom.) ; 7,47 (m, 1H, H arom.) ; 7,30 (s, 1H, H arom.) ; 6,77 (s, 1H, H arom.) ; 3,96 (s, 3H, CH₃) ; 3,88 (s, 3H, CH₃).
SM-CL : MH+ = 324,03 ; t.r. = 11,60 min.

### 137.4) 2-(2-chloro-6-fluorophényl)-5-méthoxy-1,3-benzothiazole-4,7-dione :

RMN ¹H (DMSO d6, 400 MHz, δ) : 7,69 (m, 1H, H arom.) ; 7,61 (m, 1H, H arom.) ; 7,52 (m, 1H, H arom.) ; 6,32 (s, 1H, CH) ; 3,88 (s, 3H, CH₃).
SM-CL : MH+ = 324,03 ; t.r. = 9,23 min.

### 137.5) 2-(2-chloro-6-fluorophényl)-5-{[2-(dinzéthylamino)éthyl]amino}-1,3-benzothiazole-4,7-dione :

RMN ¹H (DMSO d6, 400 MHz, δ) : 7,67 (s, 1H, H arom.) ; 7,59 (m, 1H, H arom.) ; 7,55 (m, 1H, NH.) ; 7,49 (m, 1H, H arom.) ; 5,61 (s, 1H, CH) ; 3,36 (m, 2H, CH₂) ; 2,54 (m, 2H, CH₂) ; 2,19 (s, 6H, 2CH₃).
SM-CL : MH+ = 380,10 ; t.r. = 7,88 min.

### Exemple 138 : 2-(2-chloro-6-fluorophényl)-5-[(2-pyrrolidin-1-yléthyl)amino]-1,3-benzothiazole-4,7-dione :

SM-CL : MH+ = 406,10 ; t.r. = 8,01 min.

### Etude pharmacologique pour les exemples 1 à 131 de composés de formule générale (I)

### Protocoles des tests

### i) Mesure de l'activité phosphatase de l'enzyme recombinante Cdc25C purifiée

L'activité phosphatase de la protéine MBP-Cdc25C est évaluée par la déphosphorylation du 3-O-méthylfluorescéine-phosphate (OMFP) en 3-O-méthylfluorescéine (OMF) avec une détermination de la fluorescence à 475 nm du produit de la réaction. Cet essai permet d'identifier des inhibiteurs de l'enzyme recombinante Cdc25. La préparation de la protéine de fusion MBP-Cdc25C est décrite dans la demande de brevet PCT WO 01/44467.

La réaction est réalisée en format de plaque 384 puits sous un volume final de 50 µl. La protéine MBP-Cdc25C (préparée comme décrit ci-dessus) est conservée dans le tampon d'élution suivant : 20 mM Tris-HCl pH 7,4 ; 250 mM NaCl ; 1mM EDTA ; 1 mM de dithiothréitol (DTT) ; 10 mM maltose. Elle est diluée à la concentration de 60 µM dans le tampon de réaction suivant : 50 mM Tris-HCl pH 8,2 ; 50 mM NaCl ; 1 mM DTT ; 20% glycérol. La mesure du bruit de fond est effectuée avec le tampon sans addition de l'enzyme. Les produits sont testés à des concentrations décroissantes à partir de 40 µM. La réaction est initiée par l'ajout d'une solution OMFP à 500 µM finale (préparée extemporanément à partir d'une solution stock 12,5 mM dans du DMSO 100% (Sigma #M2629)) . Après 4 heures à 30 °C dans une plaque 384 puits à usage unique, la fluorescence mesurée à DO 475 nm est lue à l'aide d'un lecteur de plaque Victor² (EGG-Wallac). La détermination de la concentration inhibant de 50% la réaction enzymatique est calculée à partir de trois expériences indépendantes. Seules les valeurs contenues dans la partie linéaire de la sigmoïde sont retenues pour l'analyse de régression linéaire.

### ii) Caractérisation de l'activité anti-proliférative :

A titre d'exemple, on étudiera l'effet d'un traitement sur deux lignées de cellules humaines Mia-Paca2 et DU145 par les composés des exemples décrits précédemment. Les lignées cellulaires DU145 (cellules humaines de cancer de la prostate) et Mia-PaCa2 (cellules humaines de cancer du pancréas) ont été acquises auprès de American Tissue Culture Collection (Rockville, Maryland, USA). Les cellules placées dans 80 µl de milieu Eagle modifié de Dulbecco (Gibco-Brl, Cergy-Pontoise, France) complété avec 10% de sérum foetal de veau inactivé par chauffage (Gibco-Brl, Cergy-Pontoise, France), 50000 unités/l de pénicilline et 50 mg/l streptomycine (Gibco-Brl, Cergy-Pontoise, France), et 2 mM de glutamine (Gibco-Brl, Cergy-Pontoise, France) ont été ensemencées sur une plaque de 96 puits au jour 0. Les cellules ont été traitées au jour 1 pendant 96 heures avec des concentrations croissantes de chacun des composés à tester jusqu'à 10 µM. A la fin de cette période, la quantification de la prolifération cellulaire est évaluée par test colorimétrique en se basant sur le clivage du sel de tétrazolium WST1 par les déhydrogénases mitochondriales dans les cellules viables conduisant à la formation de formazan (Boehringer Mannheim, Meylan, France). Ces tests sont effectués en double avec 8 déterminations par concentration testée. Pour chaque composé à tester, les valeurs incluses dans la partie linéaire de la sigmoïde ont été retenues pour une analyse en régression linéaire et utilisées pour estimer la concentration inhibitrice CI₅₀. Les produits sont solubilisés dans le diméthylsulfoxide (DMSO) à 10⁻² M et utilisés en culture avec 0,1% DMSO en final.

### Résultats des tests

a) Les composés des exemples 1 à 98, 101 à 104 et 107 à 115 présentent une CI₅₀ inférieure ou égale à 10 µM sur l'activité phosphatase de l'enzyme recombinante Cdc25-C purifiée.
b) Les composés des exemples 1 à 9, 11, 14 à 34, 36 à 53, 55 à 58, 60 à 98 et 101 à 115 présentent une CI₅₀ inférieure ou égale à 10 µM sur la prolifération cellulaire des lignées Mia-Paca2.
c) Les composés des exemples 1 à 9, 11, 14 à 34, 36 à 53, 55 à 58, 60 à 98 et 101 à 115 présentent une CI₅₀ inférieure ou égale à 10 µm sur la prolifération cellulaire des lignées DU-145.

### EXEMPLES D'ASSOCIATIONS SELON L'INVENTION

### A) TEST DE PROLIFÉRATION CELLULAIRE SUR DES CELLULES HT-29

Les associations présentées à titre d'exemples d'associations selon l'invention peuvent être testées quant à leur activité biologique et les résultats de l'association comparés aux résultats obtenus pour cahcun des composés de l'association utilisé séparément. Le protocole pour le test employé pour obtenir les résultats indiqués est décit ci-après :

### Lignée cellulaire

La lignée cellulaire HT-29 (cellules humaines de cancer du colon) a été acquise auprès de American Tissue Culture Collection (Rockville, Maryland, USA).

### Mesure de la prolifération cellulaire in vitro

Les cellules HT-29 ( 2000 cellules/puits) sont cultivées en plaques 96 puits.
Au jour 0, ces cellules sont ensemencées dans 90 µl de milieu de Eagle modifié par Dulbecco (Gibco-Brl, Cergy-Pontoise, France) complété avec 10% de sérum foetal de veau inactivé par chauffage (Gibco-Brl, Cergy-Pontoise, France ), 50000 unités/l de pénicilline et 50 mg/l streptomycine (Gibco-Brl, Cergy-Pontoise, France), et 2 mM de glutamine (Gibco-Brl, Cergy-Pontoise, France).
Les cellules ont été traitées simultanément avec des concentrations de deux produits seuls ou en association au Jour 1 et cela pendant 120 heures.
A la fin de cette période (J6), la quantification de la prolifération cellulaire est évaluée par test colorimétrique en se basant sur le clivage du sel de tétrazolium WST1 par les déhydrogénases mitochondriales dans les cellules vivantes conduisant à la formation de formazan (Boehringer Mannheim, Meylan, France). Ces tests sont effectués au moins en double avec 4 déterminations pour chaque produit seul et pour chaque association testée. Ceci permet de déterminer le nombre de cellules vivantes à la fin de chaque traitement.

### B) ASSOCIATIONS SELON L'INVENTION

### Association 1 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = 5-fluorouracyle **(B1)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B1 (2,5. 10⁻⁶ M) | Composé B1 (2,5. 10⁻⁶ M) seul |
|---|---|---|
| 35 | 13 | 42 |

### Association 2 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A1 (2,5.10⁻⁷ M) seul | Composé A1 (2,5.10⁻⁷ M) + composé B2 (10⁻⁷ M) | Composé B2 (10⁻⁷ M) seul |
|---|---|---|
| 91 | 36 | 49 |

### Association 3 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B3 (5.10⁻⁹ M) | Composé B3 (5.10⁻⁹ M) seul |
|---|---|---|
| 18 | 9 | 74 |

### Association 4 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B4 (10⁻⁵ M) | Composé B4 (10⁻⁵ M) seul |
|---|---|---|
| 31 | 11 | 33 |

### Association 5 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = méthotrexate **(B5)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B5 (5.10⁻⁸ M) | Composé B5 (5.10⁻⁸ M) seul |
|---|---|---|
| 27 | 17 | 83 |

### Association 6 :

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = doxorubicine **(B6)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B6 (5.10⁻⁷ M) | Composé B6 (5.10⁻⁷ M) seul |
|---|---|---|
| 25 | 9 | 38 |

### Association 7 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = 4-(2-bromophényl)-1-(2-(1-((4-cyano-3-méthoxy)phénylméthyl)imidazo-5-yl)-1-oxoéthyl)-1,2-dihydro-8-fluoroimidazo[1,2a][1,4]-benzodiazépine **(B7)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B7 (10⁻⁵ M) | Composé B7 (10⁻⁵ M) seul |
|---|---|---|
| 51 | 14 | 92 |

### Association 8 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B8 (10⁻⁶ M) | Composé B8 (10⁻⁶ M) seul |
|---|---|---|
| 47 | 6 | 69 |

### Association 9 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = tétrachlorhydrate de (1*R*)-1-[({(2*R*)-2-amino-3-[(8*S*)-8-(cyclohexylméthyl)- 2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine **(B9)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B9 (10⁻⁵ M) | Composé B9 (10⁻⁵ M) seul |
|---|---|---|
| 36 | 9 | 38 |

### Association 10 :

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = amsacrine **(B10)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B10 (10⁻⁷ M) | Composé B10 (10⁻⁷ M) seul |
|---|---|---|
| 42 | 39 | 89 |

### Association 11 :

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B11 (5.10⁻⁹ M) | Composé B11 (5.10⁻⁹ M) seul |
|---|---|---|
| 44 | 7 | 98 |

### Association 12 :

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = diflomotécan **(B12)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B 12 (10⁻⁹ M) | Composé B12 (10⁻⁹ M) seul |
|---|---|---|
| 33 | 22 | 83 |

### Association 13 :

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = BN-80927 ou chlorhydrate de (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione **(B13)**

Les résultats au test de prolifération cellulaire décrit ci-dessus (exprimés en % de cellules survivantes) pour les composés susmentionnés seuls ou en association sont reportés dans le tableau ci-après.

| Composé A1 (5.10⁻⁷ M) seul | Composé A1 (5.10⁻⁷ M) + composé B13 (10⁻⁹ M) | Composé B13 (10⁻⁹ M) seul |
|---|---|---|
| 43 | 32 | 91 |

### Association 14 : ne fait pas partie de l'invention

Inhibiteur de phosphatase Cdc25 = chlorhydrate de 5-{[2-(diméthylamino)éthyl]amino}-2-méthyl-1,3-benzothiazole-4,7-dione **(A1)** Agent anti-cancéreux associé = roscovitine **(B14)**

Les résultats au test de prolifération cellulaire décrit ci-dessus (exprimés en % de cellules survivantes) pour les composés susmentionnés seuls ou en association sont reportés dans le tableau ci-après.

| Composé A1 (10⁻⁶ M) seul | Composé A1 (10⁻⁶ M) + composé B 14 (5.10⁻⁵ M) | Composé B14 (5.10⁻⁵ M) seul |
|---|---|---|
| 36 | 0 | 44 |

### Association 15 : ne fait pas partie de l'invention

Inhibiteur de phosphatase Cdc25 = ménadione **(A2)**

Agent anti-cancéreux associé = roscovitine **(B14)**

Les résultats au test de prolifération cellulaire décrit ci-dessus (exprimés en % de cellules survivantes) pour les composés susmentionnés seuls ou en association sont reportés dans le tableau ci-après.

| Composé A2 (4.10⁻⁵ M) seul | Composé A2 (4.10⁻⁵ M) + composé B14 (5.10⁻⁵ M) | Composé B 14 (5.10⁻⁵ M) seul |
|---|---|---|
| 7 | 0 | 47 |

### Association 16: ne fait pas partie de l'invention

Inhibiteur de phosphatase Cdc25 = ménadione **(A2)**

Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A2 (4.10⁻⁵ M) seul | Composé A2 (4.10⁻⁵ M) + composé B8 (10⁻⁶ M) | Composé B8 (10⁻⁶ M) seul |
|---|---|---|
| 33 | 4 | 17 |

*Les associations 17 à 19 donnent des résultats similaires à ceux observés pour les associations 11 à 13.*

### Association 17 :

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = diflomotécan **(B12)**

### Association 18 :

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = BN-80927 ou chlorhydrate de (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione **(B13)**

### Association 19 :

Inhibiteur de phosphatase Cdc25 = 2-(2-chloro-6-fluorophényl)-5-{[2-(diméthylamino)éthyl]amino}-1,3-benzothiazole-4,7-dione **(A5)**

Agent anti-cancéreux associé = diflomotécan **(B12)**

*Les associations 20 à 22 donnent des résultats similaires à ceux observés pour l'association 8.*

### Association 20 : ne fait pas partie de l'invention

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = 8-bromo-4-[(3-pyridyl)méthylamino]-2-méthylthio-pyrazolo[1,5-a]-1,3,5-triazine **(B15)**

### Association 21 : ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = 8-bromo-2-(1R-isopropyl-2-hydroxyéthylamino)-4-(3-fluorophénylméthylamino)-pyrazolo[1,5-a]-1,3,5-triazine **(B8)**

### Association 22 : ne fait pas partie de l'invention

Inhibiteur de phosphatase Cdc25 = 2-(2-chloro-6-fluorophényl)-5-{[2-(diméthylamino)éthyl]amino}-1,3-benzothiazole-4,7-dione **(A5)**

Agent anti-cancéreux associé = 8-bromo-2-(1*R*-isopropyl-2-hydroxyéthylamino)-4-(3-pyridylméthylamino)pyrazolo[1,5-a]-1,3,5-triazine **(B16)**

### Association 23: ne fait pas partie de l'invention

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A3 (1,25.10⁻⁷ M) seul | Composé A3 (1,25.10⁻⁷ M) + composé B3 (5.10⁻⁹ M) | Composé B3 (5.10⁻⁹ M) seul |
|---|---|---|
| 91 | 32 | 54 |

### Association 24:

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B11 (10⁻⁸ M) | Composé B11 (10⁻⁸ M) seul |
|---|---|---|
| 75 | 40 | 73 |

### Association 25: ne fait pas partie de l'invention

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B2 (5.10⁻⁸ M) | Composé B2 (5.10⁻⁸ M) seul |
|---|---|---|
| 84 | 55 | 82 |

### Association 26:

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = doxorubicine **(B6)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B6 (10⁻⁷ M) | Composé B6 (10⁻⁷ M) seul |
|---|---|---|
| 73 | 58 | 75 |

### Association 27: ne fait pas partie de l'invention

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = 8-bromo-2-(1R-isopropyl-2-hydroxyethylamino)-4-(3-fluorophenylmethylamino)-pyrazolo[1,5-a]-1,3,5-triazine **(B8)**

Les résultats au test de prolifération cellulaire décrit ci-dessus (exprimés en % de cellules survivantes) pour les composés susmentionnés seuls ou en association sont reportés dans le tableau ci-après.

| Composé A3 (1,25.10⁻⁷ M) seul | Composé A3 (1,25.10⁻⁷ M) + composé B8 (10⁻⁶ M) | Composé B8 (10⁻⁶ M) seul |
|---|---|---|
| 89 | 12 | 21 |

### Association 28: ne fait pas partie de l'invention

Inhibiteur de phosphatase Cdc25 = benzoate de 2-méthyl-5-[(2-pyrrolidin-1-yléthyl)amino]-1,3-benzothiazole-4,7-dione **(A3)**

Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A3 (2,5.10⁻⁷ M) seul | Composé A3 (2,5.10⁻⁷ M) + composé B4 (10⁻⁵ M) | Composé B4 (10⁻⁵ M) seul |
|---|---|---|
| 78 | 36 | 75 |

### Association 29: ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = taxol **(B3)**

Les résultats au test de prolifération cellulaire décrit ci-dessus (exprimés en % de cellules survivantes) pour les composés susmentionnés seuls ou en association sont reportés dans le tableau ci-après.

| Composé A4 (2,5.10⁻⁷ M) seul | Composé A4 (2,5.10⁻⁷ M) + composé B3 (5.10⁻⁹ M) | Composé B3 (5.10⁻⁹ M) seul |
|---|---|---|
| 94 | 33 | 51 |

### Association 30:

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = SN-38 **(B11)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B11 (10⁻⁸ M) | Composé B11 (10⁻⁸ M) seul |
|---|---|---|
| 78 | 42 | 67 |

### Association 31:

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = diflomotécan **(B12)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B12 (10⁻⁹ M) | Composé B12 (10⁻⁹ M) seul |
|---|---|---|
| 79 | 58 | 89 |

### Association 32: ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = mitomycine C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B2 (5.10⁻⁸ M) | Composé B2 (5.10⁻⁸ M) seul |
|---|---|---|
| 74 | 83 | 88 |

### Association 33: <①>

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = mitomycin C **(B2)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B2 (5.10⁻⁸ M) | | Composé B2 (5.10⁻⁸ M) seul |
|---|---|---|---|
| 74 | 83 | | 88 |

### Association 34: ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = 5-fluorouracile **(B1)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B1 (2,5.10-6 M) | Composé B1 (2,5.10⁻⁶ M) seul |
|---|---|---|
| 75 | 34 | 51 |

### Association 35: ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = cisplatine **(B4)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B4 (10⁻⁵ M) | Composé B4 (10⁻⁵ M) seul |
|---|---|---|
| 71 | 43 | 85 |

### Association 36: ne fait pas partie de l'invention

Inhibiteur de phosphatase Cdc25 = benzoate de 2-méthyl-5-[(2-pipéridin-1-yléthyl)amino]-1,3-benzothiazole-4,7-dione **(A4)**

Agent anti-cancéreux associé = tétrachlorhydrate de (1*R*)-1-[({(2*R*)-2-amino-3-[(8*S*)-8-(cyclohexylméthyl)-2-phényl-5,6-dihydroimidazo[1,2-*a*]pyrazin-7(8*H*)-yl]-3-oxopropyl}dithio)méthyl]-2-[(8*S*)-8-(cyclohexylméthyl)-2-phényl-5,6-dihydroimidazo[1,2-*a*]pyrazin-7(8*H*)-yl]-2-oxoéthylamine **(B9)**

Les résultats au test de prolifération cellulaire décrit ci-dessus (exprimés en % de cellules survivantes) pour les composés susmentionnés seuls ou en association sont reportés dans le tableau ci-après.

| Composé A4 (5.10⁻⁷ M) seul | Composé A4 (5.10⁻⁷ M) + composé B9 (5.10⁻⁶ M) | Composé B9 (5.10⁻⁶ M) seul |
|---|---|---|
| 78 | 51 | 80 |

## Revendications

1. Produit comprenant au moins un inhibiteur de phosphatase Cdc25 en association avec au moins un autre agent anti-cancéreux choisi parmi des inhibiteurs de topoisomérases de type I et/ou II, pour une utilisation thérapeutique simultanée, séparée ou étalée dans le temps dans le traitement du cancer,
**caractérisé en ce que** l'inhibiteur de phosphatase Cdc25 associé à l'autre agent anti-cancéreux est un composé de formule générale **(I)** 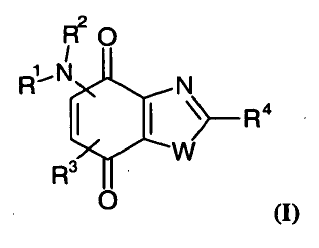 dans laquelle :
R¹ représente un atome d'hydrogène ou un radical alkyle, alkoxyalkyle, alkylthioalkyle, cycloalkyle, -(CH₂)-X-Y, -(CH₂)-Z-NR⁵R⁶ ou un radical -CHR³⁵R³⁶ dans lequel R³⁵ et R³⁶ forment ensemble avec l'atome de carbone qui les porte un radical indanyle ou tétralinyle, ou encore R³⁵ et R³⁶ forment ensemble avec l'atome de carbone qui les porte un hétérocycle saturé comptant de 5 à 7 chaînons et de 1 à 2 hétéroatomes choisis parmi O, N et S, les atomes d'azote dudit hétérocycle étant éventuellement substitués par des radicaux choisis parmi les radicaux alkyle et le radical benzyle,
R¹ pouvant aussi, lorsque W représente O, représenter en outre un radical aryle carbocyclique éventuellement substitué de 1 à 3 fois par des substituants choisis indépendamment parmi un atome halogène et un radical alkyle, haloalkyle ou alkoxy,
X représentant une liaison ou un radical alkylène linéaire ou ramifié comptant de 1 à 5 atomes de carbone,
Y représentant un système cyclique carboné saturé comptant de 1 à 3 cycles condensés choisis indépendamment parmi des cycles de 3 à 7 chaînons, ou bien Y représentant un hétérocycle saturé comptant de 1 à 2 hétéroatomes choisis indépendamment parmi O, N et S et attaché au radical X par un chaînon N ou CH, ledit hétérocycle saturé comptant par ailleurs de 2 à 6 chaînons supplémentaires choisis indépendamment parmi -CHR⁷-, -CO-, -NR⁸-, -O- et -S-, R⁷ représentant un atome d'hydrogène ou un radical alkyle et R⁸ représentant un atome d'hydrogène ou un radical alkyle ou aralkyle, ou encore Y représentant un radical aryle carbocyclique ou hétérocyclique éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR⁹ et un radical NR¹⁰R¹¹, R⁹ représentant un atome d'hydrogène ou un radical alkyle ou phényle, et R¹⁰ et R¹¹ représentant indépendamment des radicaux alkyle, Z représentant une liaison ou un radical alkylène linéaire ou ramifié comptant de 1 à 5 atomes de carbone,
R⁵ et R⁶ étant choisis indépendamment parmi un atome d'hydrogène, un radical alkyle, aralkyle ou -(CH₂)ₙ-OH dans lequel n représente un entier de 1 à 6,
ou R⁵ représentant un radical alkoxycarbonyle, haloalkoxycarbonyle ou aralkoxycarbonyle et R⁶ représentant un atome d'hydrogène ou un radical méthyle,
ou encore R⁵ et R⁶ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR¹²R¹³-, -O-, -S- et -NR¹⁴-, R¹² et R¹³ représentant indépendamment à chaque fois qu'ils interviennent un atome d'hydrogène ou un radical alkyle, et R¹⁴ représentant un atome d'hydrogène ou un radical alkyle ou aralkyle, ou encore R¹⁴ représentant un radical phényle éventuellement substitué de 1 à 3 fois par des substituants choisis indépendamment parmi un atome halogène et un radical alkyle ou alkoxy,
R² représentant un atome d'hydrogène ou un radical alkyle ou aralkyle ;
ou encore R¹ et R² formant ensemble avec l'atome d'azote un hétérocycle de 4 à 8 chaînons comportant de 1 à 2 hétéroatomes, les chaînons nécessaires pour compléter l'hétérocycle étant choisis indépendamment parmi les radicaux -CR¹⁵R¹⁶-, -O-, -S- et -NR¹⁷-, R¹⁵ et R¹⁶ représentant indépendamment à chaque fois qu'ils interviennent un atome d'hydrogène ou un radical alkyle, et R¹⁷ représentant un atome d'hydrogène ou un radical alkyle ou aralkyle ;
R³ représente un atome d'hydrogène, un atome halogène, ou un radical alkyle, haloalkyle, alkoxy ou alkylthio ;
R⁴ représente un radical alkyle, cycloalkyle, cycloalkylalkyle, cyano, amino, -CH₂-COOR¹⁸, -CH₂-CO-NR¹⁹R²⁰ ou -CH₂-NR²¹R²², ou bien R⁴ représente un radical aryle carbocyclique ou hétérocyclique éventuellement substitué de 1 à 4 fois par des substituants choisis indépendamment parmi un atome halogène et un radical alkyle, haloalkyle, alkoxy, haloalkoxy ou NR³⁷R³⁸, ou encore R⁴ représente un radical phényle possédant deux substituants qui forment ensemble un radical méthylènedioxy ou éthylènedioxy,
R¹⁸ représentant un atome d'hydrogène ou un radical alkyle,
R¹⁹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²³ et un radical NR²⁴R²⁵, R²³ représentant un atome d'hydrogène ou un radical alkyle ou phényle, et R²⁴ et R²⁵ représentant indépendamment des radicaux alkyle,
R²⁰ représentant un atome d'hydrogène ou un radical alkyle,
ou encore R¹⁹ et R²⁰ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR²⁶R²⁷-, -O-, -S- et -NR²⁸-, R²⁶ et R²⁷ représentant indépendamment à chaque fois qu'ils interviennent un atome d'hydrogène ou un radical alkyle, et R²⁸ représentant un atome d'hydrogène ou un radical alkyle ou aralkyle, ou encore R²⁸ représentant un radical phényle éventuellement substitué de 1 à 3 fois par des substituants choisis indépendamment parmi un atome halogène et un radical alkyle ou alkoxy,
R²¹ représentant un atome d'hydrogène, un radical alkyle ou un radical aralkyle dont le groupe aryle est éventuellement substitué de 1 à 3 fois par des substituants choisis indépendamment parmi le groupe constitué par un atome halogène, un radical alkyle, un radical haloalkyle, un radical alkoxy, un radical haloalkoxy, un radical hydroxy, un radical nitro, un radical cyano, le radical phényle, un radical SO₂NHR²⁹ et un radical NR³⁰R³¹, R²⁹ représentant un atome d'hydrogène ou un radical alkyle ou phényle, et R³⁰ et R³¹ représentant indépendamment des radicaux alkyle,
R²² représentant un atome d'hydrogène ou un radical alkyle,
ou encore R²¹ et R²² formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³²R³³-, -O-, -S- et -NR³⁴-, R³² et R³³ représentant indépendamment à chaque fois qu'ils interviennent un atome d'hydrogène ou un radical alkyle, et R³⁴ représentant un atome d'hydrogène, un radical alkyle ou aralkyle, ou encore R³⁴ représentant un radical phényle éventuellement substitué de 1 à 3 fois par des substituants choisis indépendamment parmi un atome halogène et un radical alkyle ou alkoxy,
R³⁷ et R³⁸ étant choisis indépendamment parmi un atome d'hydrogène et un radical alkyle ou R³⁷ et R³⁸ formant ensemble avec l'atome d'azote un hétérocycle de 4 à 7 chaînons comportant de 1 à 2 hétéroatomes, les chaînons nécessaires pour compléter l'hétérocycle étant choisis indépendamment parmi les radicaux -CR³⁹R⁴⁰-, -O-, -S- et -NR⁴¹-, R³⁹ et R⁴⁰ représentant indépendamment à chaque fois qu'ils interviennent un atome d'hydrogène ou un radical alkyle, et R⁴¹ représentant un atome d'hydrogène ou un radical alkyle ; et
W représente O ou S ;
ou un sel pharmaceutiquement acceptable d'un composé de formule générale **(I).**

2. Produit selon la revendication 1, **caractérisé en ce que** le composé de formule générale **(I)** ou son sel pharmaceutiquement acceptable est choisi parmi les composés suivants :
- 5-{[2-(diméthylamino)éthyl]amino}-2-méthyl-1,3-benzothiazole-4,7-dione ;
- 2-méthyl-5-[(2-pyrrolidin-1-yléthyl)amino]-1,3-benzothiazole-4,7-dione ;
- 2-méthyl-5-[(2-pipéridin-1-yléthyl)amino]-1,3-benzothiazole-4,7-dione ;
- 2-(2-chloro-6-fluorophényl)-5-{[2-(diméthylamino)éthyl]amino}-1,3-benzothiazole -4,7-dione ;
et les sels pharmaceutiquement acceptables de ces derniers.

3. Produit selon la revendication 1, **caractérisé en ce que** l'inhibiteur de topoisomérases de type I et/ou II est la camptothécine ou un de ses analogues.

4. Produit selon la revendication 3, **caractérisé en ce que** l'inhibiteur de topoisomérases de type I et/ou II est un composé de formule générale **(III)** 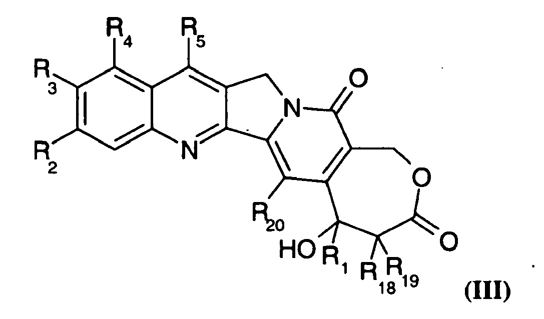 sous forme racémique, d'énantiomère ou toutes combinaisons de ces formes, dans laquelle
R₁ représente un alkyle inférieur, un alkényle inférieur, un alkynyl inférieur, un haloalkyle inférieur, un alkoxy inférieur alkyle inférieur ou un alkylthio inférieur alkyle inférieur;
R₂, R₃ et R₄ représentent, indépendamment, i) H, halo, halo alkyle inférieur, alkyle inférieur, alkényle inférieur, cyano, cyano alkyle inférieur, nitro, nitro alkyle inférieur, amido, amido alkyle inférieur, hydrazino, hydrazino alkyle inférieur, azido, azido alkyle inférieur, (CH₂)ₘNR₆R₇, (CH₂)ₘOR₆, (CH₂)ₘSR₆, (CH₂)mCO₂R₆, (CH₂)ₘNR₆C(O)R₈, (CH2)ₘC(O)R₈, (CH₂)ₘOC(O)R₈, O(CH₂)ₘNR₆R₇, OC(O)NR₆R₇, OC(O)(CH₂)ₘCO₂R₆, ou ii) les radicaux substitués (c'est-à-dire, substitué une à quatre fois sur le groupe aryle ou l'hétérocycle) ou non substitués suivants : (CH₂)ₙ[N=X], OC(O)[N=X], (CH₂)ₘOC(O)[N=X] (dans lequel [N=X], dans cette invention, représente un groupe hétérocyclique de 4 à 7 chaînons avec l'atome d'azote N, qui est un membre du groupe hétérocyclique, et X représente les membres restants, nécessaires pour compléter le groupe hétérocyclique, sélectionnés parmi le groupe constitué de O, S, CH₂, CH, N, NR₉ et COR₁₀), aryle ou aryle alkyle inférieur, dans lesquels les substituants éventuels sont choisis parmi le groupe constitué par un alkyle inférieur, halo, nitro, amino, alkylamino inférieur, haloalkyle inférieur, hydroxy alkyle inférieur, alkoxy inférieur, et alkoxy inférieur alkyle inférieur ; ou R₂ et R₃ forment ensemble une chaîne à 3 ou 4 chaînons, dans laquelle les éléments de la chaîne sont sélectionnés parmi le groupe constitué de CH, CH₂, O, S, N ou NR₉;
R₅ représente i) H, halo, halo alkyle inférieur, alkyle inférieur, alkoxy inférieur, alkoxy inférieur alkyle inférieur, alkylthio inférieur alkyle inférieur, cycloalkyle, cycloalkyle alkyle inférieur, cyano, cyano alkyle, alkyle inférieur sulphonyl alkyle inférieur, hydroxy alkyle inférieur, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇, (CH₂)ₘS(O)qR₁₁, (CH₂)ₘP(O)R₁₂R₁₃, (CH₂)₂P(S)R₁₂R₁₃, ou ii) l'un des radicaux substitués (c'est-à-dire une à quatre fois sur le groupe aryle ou hétéroaryle) ou non substitués suivants : (CH₂)ₙ[N=X], OC(O)[N=X], (CH₂)ₘOC(O)[N=X], aryle ou aryle alkyle inférieur, dans lesquels les substituants éventuels sont choisis parmi le groupe constitué par un alkyle inférieur, halo, nitro amino, alkyle inférieur amino, halo alkyle inférieur, hydroxy alkyle inférieur, alkoxy inférieur et alkoxy inférieur alkyle inférieur;
R₆ et R₇ représentent, indépendamment, i) H, un alkyle inférieur, hydroxy alkyle inférieur, alkyle inférieur amino alkyle inférieur, amino alkyle inférieur, cycloalkyle, cycloalkyle alkyle inférieur, alkényle inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, et alkoxy inférieur alkyle inférieur;
R₈ représente i) H, un alkyle inférieur, hydroxy alkyle inférieur, amino, alkyle inférieur amino, alkyle inférieur amino alkyle inférieur, amino alkyle inférieur, cycloalkyle, cycloalkyle alkyle inférieur, alkényle inférieur, alkoxy inférieur, alkoxy inférieur alkyle inférieur, halo alkyle inférieur, ou ii) l'un des radicaux substitués (c'est-à-dire, une à quatre fois sur le groupe aryle) ou non substitués suivants : aryle ou aryle alkyle inférieur, dans lesquels les substituants éventuels sont choisis parmi le groupe constitué par un alkyle inférieur, halo, nitro, amino, alkyle inférieur amino, halo alkyle inférieur, hydroxy alkyle inférieur, alkoxy inférieur, ou alkoxy inférieur alkyle inférieur;
R₉ représente H, un alkyle inférieur, halo alkyle inférieur, aryle, ou aryle substitué par un ou plusieurs groupes choisis parmi le radical alkyle inférieur, halo, nitro, amino, alkyle inférieur amino, halo alkyle inférieur, hydroxy alkyle inférieur, alkoxy inférieur, ou alkoxy inférieur alkyle inférieur;
R₁₀ représente H, un alkyle inférieur, halo alkyle inférieur, alkoxy inférieur, aryle, ou aryle substitué (c'est-à-dire, présentant un à quatre substituants sur le groupe aryle) par un ou plusieurs groupes choisis parmi le radical alkyle inférieur, halo alkyle inférieur, hydroxy alkyle inférieur, ou alkoxy inférieur alkyle inférieur;
R₁₁ représente un alkyle inférieur, aryle, (CH₂)ₘOR₁₄, (CH₂)ₘSR₁₄, (CH₂)₂NR₁₄R₁₅ ou (CH₂)ₘ[N=X];
R₁₂ et R₁₃ représentent, indépendamment, un alkyle inférieur, aryle, alkoxy inférieur, aryloxy ou amino;
R₁₄ et R₁₅ représentent, indépendamment, H, un alkyle inférieur ou aryle;
R₁₈ et R₁₉ représentent, indépendamment, H, halo, alkyl inférieur, alkoxy inférieur ou hydroxy;
R₂₀ représente H ou halo;
m est un nombre entier compris entre 0 et 6;
n est 1 ou 2; et
q représente un nombre entier de 0 à 2; et [N=X] représente un groupe hétérocyclique de 4 à 7 chaînons, X représentant la chaîne nécessaire pour compléter ledit groupe hétérocyclique et sélectionnée dans le groupe constitué de O, S, CH₂, CH, N, NR₉ et COR_{10;}
ou un sel pharmaceutiquement acceptable de ce dernier.

5. Produit selon la revendication 4, **caractérisé en ce que** le composé de formule générale **(III)** ou son sel pharmaceutiquement acceptable sera choisi parmi : le diflomotécan et la (+)-9-chloro-5-éthyl-5-hydroxy-10-méthyl-12-(4-méthylpipéridinométhyl)-4,5,13,15-tétrahydro-1H,3H-oxépino[3',4':6,7]indolizino[1,2-c]quinoline-3,15-dione et ses sels pharmaceutiquement acceptables.

6. Produit selon la revendication 3, **caractérisé en ce que** l'inhibiteur de topoisomérases de type I et/ou II est le composé SN-38.

7. Produit selon la revendication 1, **caractérisé en ce que** l'inhibiteur de topoisomérases de type I et/ou II est la doxorubicine ou l'amsacrine.

## Claims

1. Product comprising at least one Cdc25 phosphatase inhibitor in combination with at least one other anti-cancer agent chosen from type I and/or II topoisomerass inhibitors, for a therapeutic use which is simultaneous, separate or spread over time in the treatment of cancer,
**characterized in that** the Cdc25 phosphatase inhibitor combined with the other anti-cancer agent is a compound of general formula **(I)** 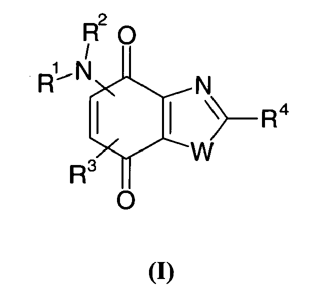 in which:
R¹ represents a hydrogen atom or an alkyl, alkoxyalkyl, alkylthioalkyl, cycloalkyl, -(CH₂)-X-Y, -(CH₂)-Z-NR⁵R⁶ radical or a -CHR³⁵R³⁶ radical in which R³⁵ and R³⁶ form together with the carbon atom which carries them an indanyl or tetralinyl radical, or also R³⁵ and R³⁶ form together with the carbon atom which carries them a saturated heterocycle containing 5 to 7 members and 1 to 2 heteroatoms chosen from O, N and S, the nitrogen atoms of said heterocycle being optionally substituted by radicals chosen from the alkyl radicals and the benzyl radical,
R¹ also being able, when W represents O, to represent moreover a carbocyclic aryl radical optionally substituted 1 to 3 times by substituents chosen independently from a halogen atom and an alkyl, haloalkyl or alkoxy radical,
X representing a bond or a linear or branched alkylene radical containing 1 to 5 carbon atoms,
Y representing a saturated carbon-containing cyclic system with 1 to 3 condensed rings chosen independently from rings with 3 to 7 members, or Y representing a saturated heterocycle containing 1 to 2 heteroatoms chosen independently from O, N and S and attached to the X radical by an N or CH member, said saturated heterocycle moreover containing 2 to 6 additional members chosen independently from -CHR⁷-, -CO-, -NR⁸-, -O- and -S-, R⁷ representing a hydrogen atom or an alkyl radical and R⁸ representing a hydrogen atom or an alkyl or aralkyl radical, or also Y representing a carbocyclic or heterocyclic aryl radical optionally substituted 1 to 3 times by substituents chosen independently from the group constituted by a halogen atom, an alkyl radical, a haloalkyl radical, an alkoxy radical, a haloalkoxy radical, a hydroxy radical, a nitro radical, a cyano radical, the phenyl radical, an SO₂NHR⁹ radical and an NR¹⁰R¹¹ radical, R⁹ representing a hydrogen atom or an alkyl or phenyl radical, and R¹⁰ and R¹¹ independently representing alkyl radicals,
Z representing a bond or a linear or branched alkylene radical containing 1 to 5 carbon atoms,
R⁵ and R⁶ being chosen independently from a hydrogen atom, an alkyl, aralkyl or -(CH₂)ₙ-OH radical in which n represents an integer from 1 to 6,
or R⁵ representing an alkoxycarbonyl, haloalkoxycarbonyl or aralkoxycarbonyl radical and R⁶ representing a hydrogen atom or a methyl radical,
or also R⁵ and R⁶ forming together with the nitrogen atom a heterocycle with 4 to 7 members comprising 1 to 2 heteroatoms, the members necessary to complete the heterocycle being chosen independently from the -CR¹²R¹³-, -O-, -S- and -NR¹⁴- radicals, R¹² and R¹³ representing independently each time that they occur a hydrogen atom or an alkyl radical, and R¹⁴ representing a hydrogen atom or an alkyl or aralkyl radical, or also R¹⁴ representing a phenyl radical optionally substituted 1 to 3 times by substituents chosen independently from a halogen atom and an alkyl or alkoxy radical,
R² representing a hydrogen atom or an alkyl or aralkyl radical;
or also R¹ and R² forming together with the nitrogen atom a heterocycle with 4 to 8 members comprising 1 to 2 heteroatoms, the members necessary to complete the heterocycle being chosen independently from the -CR¹⁵R¹⁶-, -O-, -S- and -NR¹⁷- radicals, R¹⁵ and R¹⁶ independently representing each time that they occur a hydrogen atom or an alkyl radical, and R¹⁷ representing a hydrogen atom or an alkyl or aralkyl radical;
R³ represents a hydrogen atom, a halogen atom, or an alkyl, haloalkyl, alkoxy or alkylthio radical;
R⁴ represents an alkyl, cycloalkyl, cycloalkylalkyl, cyano, amino, -CH₂-COOR¹⁸, -CH₂-CO-NR¹⁹R²⁰ or -CH₂-NR²¹R²² radical, or R⁴ represents a carbocyclic or heterocyclic aryl radical optionally substituted 1 to 4 times by substituents chosen independently from a halogen atom and an alkyl, haloalkyl, alkoxy, haloalkoxy or NR³⁷R³⁸ radical, or also R⁴ represents a phenyl radical possessing two substituents which form together a methylenedioxy or ethylenedioxy radical,
R¹⁸ representing a hydrogen atom or an alkyl radical,
R¹⁹ representing a hydrogen atom, an alkyl radical or an aralkyl radical the aryl group of which is optionally substituted 1 to 3 times by substituents chosen independently from the group constituted by a halogen atom, an alkyl radical, a haloalkyl radical, an alkoxy radical, a haloalkoxy radical, a hydroxy radical, a nitro radical, a cyano radical, the phenyl radical, an SO₂NHR²³ radical and an NR²⁴R²⁵ radical, R²³ representing a hydrogen atom or an alkyl or phenyl radical, and R²⁴ and R²⁵ independently representing alkyl radicals,
R²⁰ representing a hydrogen atom or an alkyl radical,
or also R¹⁹ and R²⁰ forming together with the nitrogen atom a heterocycle with 4 to 7 members comprising 1 to 2 heteroatoms, the members necessary to complete the heterocycle being chosen independently from the -CR²⁶R²⁷-, -O-, -S- and -NR²⁸- radicals, R²⁶ and R²⁷ independently representing each time that they occur a hydrogen atom or an alkyl radical, and R²⁸ representing a hydrogen atom or an alkyl or aralkyl radical, or also R²⁸ representing a phenyl radical optionally substituted 1 to 3 times by substituents chosen independently from a halogen atom and an alkyl or alkoxy radical,
R²¹ representing a hydrogen atom, an alkyl radical or an aralkyl radical the aryl group of which is optionally substituted 1 to 3 times by substituents chosen independently from the group constituted by a halogen atom, an alkyl radical, a haloalkyl radical, an alkoxy radical, a haloalkoxy radical, a hydroxy radical, a nitro radical, a cyano radical, the phenyl radical, an SO₂NHR²⁹ radical and an NR³⁰R³¹ radical, R²⁹ representing a hydrogen atom or an alkyl or phenyl radical, and R³⁰ and R³¹ independently representing alkyl radicals,
R²² representing a hydrogen atom or an alkyl radical,
or also R²¹ and R²² forming together with the nitrogen atom a heterocycle with 4 to 7 members comprising 1 to 2 heteroatoms, the members necessary to complete the heterocycle being chosen independently from the -CR³²R³³-, -O-, -S- and -NR³⁴- radicals, R³² and R³³ independently representing each time that they occur a hydrogen atom or an alkyl radical, and R³⁴ representing a hydrogen atom, an alkyl or aralkyl radical, or also R³⁴ representing a phenyl radical optionally substituted 1 to 3 times by substituents chosen independently from a halogen atom and an alkyl or alkoxy radical,
R³⁷ and R³⁸ being chosen independently from a hydrogen atom and an alkyl radical or R³⁷ and R³⁸ forming together with the nitrogen atom a heterocycle with 4 to 7 members comprising 1 to 2 heteroatoms, the members necessary to complete the heterocycle being chosen independently from the -CR³⁹R⁴⁰-, -O-, -S- and -NR⁴¹- radicals, R³⁹ and R⁴⁰ independently representing each time that they occur a hydrogen atom or an alkyl radical, and R⁴¹ representing a hydrogen atom or an alkyl radical; and
W represents O or S;
or a pharmaceutically acceptable salt of a compound of general formula **(I).**

2. Product according to claim 1, **characterized in that** the compound of general formula **(I)** or its pharmaceutically acceptable salt is chosen from the following compounds:
- 5-{[2-(dimethylamino)ethyl]amino}-2-methyl-1,3-benzothiazole-4,7-dione;
- 2-methyl-5-[(2-pyrrolidin-1-ylethyl)amino]-1,3-benzothiazole-4,7-dione;
- 2-methyl-5-[(2-piperidin-1-ylethyl)amino]-1,3-benzothiazole-4,7-dione;
- 2-(2-chloro-6-fluorophenyl)-5-{[2-(dimethylamino)ethyl]amino}-1,3-benzothiazole-4,7-dione;
and the pharmaceutically acceptable salts of the latter.

3. Product according to claim 1, **characterized in that** the type I and/or II topoisomerase inhibitor is camptothecin or one of its analogues.

4. Product according to claim 3, **characterized in that** the type I and/or II topoisomerase inhibitor is a compound of general formula **(III)** 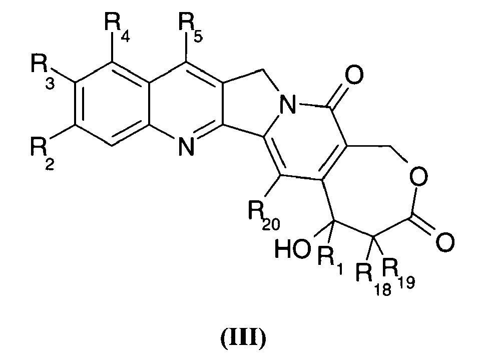 in racemic, enantiomeric form or all combinations of these forms, in which
R₁ represents a lower alkyl, a lower alkenyl, a lower alkynyl, a lower haloalkyl, a lower alkoxy lower alkyl or a lower alkylthio lower alkyl;
R₂, R₃ and R₄ represent, independently, i) H, halo, lower halo alkyl, lower alkyl, lower alkenyl, cyano, lower cyano alkyl, nitro, lower nitro alkyl, amido, lower amido alkyl, hydrazino, lower hydrazino alkyl, azido, lower azido alkyl, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘC(O)R₈, (CH₂)ₘOC(O)R₈, O(CH₂)ₘNR₆R₇, OC(O)NR₆R₇, OC(O)(CH₂)ₘCO₂R₆, or ii) the following radicals substituted (i.e., substituted one to four times on the aryl group or the heterocycle) or not substituted: (CH₂)ₙ[N=X], OC(O)[N=X], (CH₂)ₘOC(O)[N=X] (in which [N=X], in this invention, represents a heterocyclic group with 4 to 7 members with the nitrogen atom N, which is a member of the heterocyclic group, and X represents the remaining members, necessary to complete the heterocyclic group, selected from the group constituted by O, S, CH₂, CH, N, NR₉ and COR₁₀), aryl or lower aryl alkyl, in which the optional substituents are chosen from the group constituted by a lower alkyl, halo, nitro, amino, lower alkylamino, lower haloalkyl, lower hydroxy alkyl, lower alkoxy, and lower alkoxy lower alkyl; or R₂ and R₃ together form a chain with 3 or 4 members, in which the elements of the chain are selected from the group constituted by CH, CH₂, O, S, N or NR₉;
R₅ represents i) H, halo, lower halo alkyl, lower alkyl, lower alkoxy, lower alkoxy lower alkyl, lower alkylthio lower alkyl, cycloalkyl, lower cycloalkyl alkyl, cyano, cyano alkyl, lower alkyl lower sulphonyl alkyl, lower hydroxy alkyl, nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)ₘOC(O)NR₆R₇, (CH₂)ₘS(O)_{q}R₁₁, (CH₂)ₘP(O)R₁₂R₁₃, (CH₂)₂P(S)R₁₂R₁₃, or ii) one of the following radicals substituted (i.e. one to four times on the aryl or heteroaryl group) or not substituted: (CH₂)ₙ[N=X], OC(O)[N=X], (CH₂)ₘOC(O)[N=X], aryl or lower aryl alkyl, in which the optional substituents are chosen from the group constituted by a lower alkyl, halo, nitro, amino, lower alkyl amino, lower halo alkyl, lower hydroxy alkyl, lower alkoxy and lower alkoxy lower alkyl;
R₆ and R₇ represent, independently, i) H, a lower alkyl, lower hydroxy alkyl, lower alkyl lower amino alkyl, lower amino alkyl, cycloalkyl, lower cycloalkyl alkyl, lower alkenyl, lower alkoxy lower alkyl, lower halo alkyl, or ii) one of the following radicals substituted (i.e., one to four times on the aryl group) or not substituted: aryl or lower aryl alkyl, in which the optional substituents are chosen from the group constituted by a lower alkyl, halo, nitro, amino, lower alkyl amino, lower halo alkyl, lower hydroxy alkyl, lower alkoxy, and lower alkoxy lower alkyl;
R₈ represents i) H, a lower alkyl, lower hydroxy alkyl, amino, lower alkyl amino, lower alkyl amino lower alkyl, lower amino alkyl, cycloalkyl, lower cycloalkyl alkyl, lower alkenyl, lower alkoxy, lower alkoxy lower alkyl, lower halo alkyl, or ii) one of the following radicals substituted (i.e., one to four times on the aryl group) or not substituted: aryl or lower aryl alkyl, in which the optional substituents are chosen from the group constituted by a lower alkyl, halo, nitro, amino, lower alkyl amino, lower halo alkyl, lower hydroxy alkyl, lower alkoxy, or lower alkoxy lower alkyl;
R₉ represents H, a lower alkyl, lower halo alkyl, aryl, or aryl substituted by one or more groups chosen from the lower alkyl radical, halo, nitro, amino, lower alkyl amino, lower halo alkyl, lower hydroxy alkyl, lower alkoxy, or lower alkoxy lower alkyl;
R₁₀ represents H, a lower alkyl, lower halo alkyl, lower alkoxy, aryl, or aryl substituted (i.e., presenting one to four substituents on the aryl group) by one or more groups chosen from the lower alkyl radical, lower halo alkyl, lower hydroxy alkyl, or lower alkoxy lower alkyl;
R₁₁ represents a lower alkyl, aryl, (CH₂)ₘOR₁₄, (CH₂)ₘSR₁₄, (CH₂)₂NR₁₄R₁₅ or (CH₂)ₘ[N=X];
R₁₂ and R₁₃ representing, independently, a lower alkyl, aryl, lower alkoxy, aryloxy or amino;
R₁₄ and R₁₅ representing, independently, H, a lower alkyl or aryl;
R₁₈ and R₁₉ representing, independently, H, halo, lower alkyl, lower alkoxy or hydroxy;
R₂₀ represents H or halo;
m is a whole number comprised between 0 and 6;
n is 1 or 2; and
q represents a whole number from 0 to 2; and [N=X] represents a heterocyclic group with 4 to 7 members, X representing the chain necessary to complete said heterocyclic group and selected from the group constituted by O, S, CH₂, CH, N, NR₉ and COR₁₀;
or a pharmaceutically acceptable salt of the latter.

5. Product according to claim 4, **characterized in that** the compound of general formula **(III)** or its pharmaceutically acceptable salt is chosen from diflomotecan and (+)-9-chloro-5-ethyl-5-hydroxy-10-methyl-12-(4-methylpiperidinomethyl)-4,5,13,15-tetrahydro-1H,3H-oxepino[3',4':6,7]indolizino[1,2-c]quinoline-3,15-dione and its pharmaceutically acceptable salts.

6. Product according to claim 3, **characterized in that** the type I and/or II topoisomerass inhibitor is the compounds SN-38.

7. Product according to claim 1, **characterized in that** the type I and/or II topoisomerass inhibitor is doxorubicine or amsacrine.

## Patentansprüche

1. Produkt, das mindestens einen Hemmer der Phosphatase Cdc25 in Kombination mit mindestens einem anderen Antikrebsmittel umfasst, das ausgewählt ist aus Topoisomerase-I- und/oder -11-Hemmern, für eine gleichzeitige, getrennte oder zeitlich verschobene therapeutische Verwendung in der Krebsbehandlung,
**dadurch gekennzeichnet, dass** der mit dem anderen Antikrebsmittel kombinierte Hemmer der Phosphatase Cdc25 eine Verbindung der allgemeinen Formel (I) 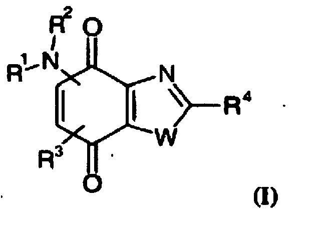 ist, in der:
R¹ ein Wasserstoffatom oder einen Rest Alkyl, Alkoxyalkyl, Alkylthioalkyl, Cycloalkyl, -(CH₂)-X-Y, -(CH₂)-Z-NR⁵R⁶ oder einen Rest -CHR³⁵R³⁶ darstellt, in dem R³⁵ und R³⁶ zusammen mit dem Kohlenstoffatom, an das sie gebunden sind, einen Indanyl- oder Tetralinylrest bilden oder R³⁵ und R³⁶ zusammen mit dem Kohlenstoffatom, an das sie gebunden sind, einen gesättigten Heterocyclus mit 5 bis 7 Gliedern und 1 bis 2 Heteroatomen bilden, ausgewählt aus O, N und S, wobei die Stickstoffatome dieses Heterocyclus gegebenenfalls mit Resten substituiert sind, ausgewählt aus den Alkylresten und dem Benzylrest,
wobei R¹, wenn W O darstellt, außerdem auch einen carbocyclischen Arylrest darstellen kann, der gegebenenfalls 1 bis 3 Mal mit Substituenten substituiert ist, unabhängig voneinander ausgewählt aus einem Halogenatom und einem Rest Alkyl, Halogenalkyl oder Alkoxy,
wobei X eine Bindung oder einen linearen oder verzweigten Alkylenrest mit 1 bis 5 Kohlenstoffatomen darstellt,
Y ein gesättigtes cyclisches Kohlenstoffsystem mit 1 bis 3 kondensierten Ringen darstellt, die unabhängig voneinander aus Ringen mit 3 bis 7 Gliedern ausgewählt sind, oder Y einen gesättigten Heterocyclus darstellt, der 1 bis 2 Heteroatome zählt, die unabhängig voneinander aus O, N und S ausgewählt sind, und der an den Rest X durch ein Glied N oder CH gebunden ist, wobei der gesättigte Heterocyclus ferner 2 bis 6 zusätzliche Glieder zählt, die unabhängig voneinander ausgewählt sind aus -CHR⁷-, -CO-, -NR⁸-, -O- und -S-, wobei R⁷ ein Wasserstoffatom oder einen Alkylrest darstellt und R⁸ ein Wasserstoffatom oder einen Alkyl-oder Aralkylrest darstellt, oder Y einen carbocyclischen oder heterocyclischen Arylrest darstellt, der gegebenenfalls 1 bis 3 Mal mit Substituenten substituiert ist, die unabhängig voneinander aus der Gruppe ausgewählt sind, bestehend aus einem Halogenatom, einem Alkylrest, einem Halogenalkylrest, einem Alkoxyrest, einem Halogenalkoxyrest, einem Hydroxyrest, einem Nitrorest, einem Cyanorest, dem Phenylrest, einem Rest SO₂NHR⁹ und einem Rest NR¹⁰R¹¹, wobei R⁹ ein Wasserstoffatom oder einen Alkyl- oder Phenylrest darstellt und R¹⁰ und R¹¹ unabhängig voneinander Alkylreste darstellen, wobei Z eine Bindung oder einen linearen oder verzweigten Alkylenrest mit 1 bis 5 Kohlenstoffatomen darstellt,
R⁵ und R⁶ unabhängig voneinander ausgewählt sind aus einem Wasserstoffatom, einem Rest Alkyl, Aralkyl oder -(CH₂)ₙ-OH, bei dem n eine ganze Zahl von 1 bis 6 darstellt,
oder R⁵ einen Rest Alkoxycarbonyl, Halogenalkoxycarbonyl oder Aralkoxycarbonyl darstellt und R⁶ ein Wasserstoffatom oder einen Methylrest darstellt oder
R⁵ und R⁶ zusammen mit dem Stickstoffatom einen Heterocyclus mit 4 bis 7 Gliedern bilden, die 1 bis 2 Heteroatome umfassen, wobei die zur Vervollständigung des Heterocyclus erforderlichen Glieder unabhängig voneinander aus -CR¹²R¹³-, -O-, -S- und -NR¹⁴- ausgewählt sind, wobei R¹² und R¹³ jeweils unabhängig voneinander ein Wasserstoffatom oder einen Alkylrest darstellen und R¹⁴ ein Wasserstoffatom oder einen Alkyl- oder Aralkylrest darstellt oder R¹⁴ einen Phenylrest darstellt, der gegebenenfalls 1 bis 3 Mal mit Substituenten substituiert ist, unabhängig voneinander ausgewählt aus einem Halogenatom und einem Alkyl- oder Alkoxyrest,
R² ein Wasserstoffatom oder einen Alkyl- oder Aralkylrest darstellt;
oder R¹ und R² zusammen mit dem Stickstoffatom einen Heterocyclus von 4 bis 8 Gliedern mit 1 bis 2 Heteroatomen bilden, wobei die zur Vervollständigung des Heterocyclus erforderlichen Glieder unabhängig voneinander aus den Resten -CR¹⁵R¹⁶-, -O-, -S- und -NR¹⁷- ausgewählt sind, wobei R¹⁵ und R¹⁶ jeweils unabhängig voneinander ein Wasserstoffatom oder einen Alkylrest darstellen und R¹⁷ ein Wasserstoffatom oder einen Alkyl- oder Aralkylrest darstellt;
R³ ein Wasserstoffatom, ein Halogenatom oder einen Rest Alkyl, Halogenalkyl, Alkoxy oder Alkylthio darstellt;
R⁴ einen Rest Alkyl, Cycloalkyl, Cycloalkylalkyl, Cyano, Amino, -CH₂-COOR¹⁸, -CH₂-CO-NR¹⁹R²⁰ oder -CH₂-NR²¹R²² darstellt oder R⁴ einen carbocyclischen oder heterocyclischen Arylrest darstellt, der gegebenenfalls 1 bis 4 Mal mit Substituenten substituiert ist, unabhängig voneinander ausgewählt aus einem Halogenatom und einem Rest Alkyl, Halogenalkyl, Alkoxy, Halogenalkoxy oder NR³⁷R³⁸, oder R⁴ einen Phenylrest mit zwei Substituenten darstellt, die zusammen einen Rest Methylendioxy oder Ethylendioxy bilden,
R¹⁸ ein Wasserstoffatom oder einen Alkylrest darstellt,
R¹⁹ ein Wasserstoffatom, einen Alkylrest oder einen Aralkylrest darstellt, dessen Arylgruppe gegebenenfalls 1 bis 3 Mal mit Substituenten substituiert ist, die unabhängig voneinander aus der Gruppe ausgewählt sind, bestehend aus einem Halogenatom, einem Alkylrest, einem Halogenalkylrest, einem Alkoxyrest, einem Halogenalkoxyrest, einem Hydroxyrest, einem Nitrorest, einem Cyanorest, dem Phenylrest, einen Rest SO₂NHR²³ und einen Rest NR²⁴R²⁵, wobei R²³ ein Wasserstoffatom oder einen Alkyl- oder Phenylrest darstellt und R²⁴ und R²⁵ unabhängig voneinander Alkylreste darstellen,
R²⁰ ein Wasserstoffatom oder einen Alkylrest darstellt,
oder R¹⁹ und R²⁰ zusammen mit dem Stickstoffatom einen Heterocyclus von 4 bis 7 Gliedern mit 1 bis 2 Heteroatomen bilden, wobei die zur Vervollständigung des Heterocyclus erforderlichen Glieder unabhängig voneinander aus den Resten -CR²⁶R²⁷-, -O-, -S- und -NR²⁸- ausgewählt sind, wobei R²⁶ und R²⁷ jeweils unabhängig voneinander ein Wasserstoffatom oder einen Alkylrest darstellen und R²⁸ ein Wasserstoffatom oder einen Alkyl- oder Aralkylrest darstellt oder R²⁸ einen Phenylrest darstellt, der gegebenenfalls 1 bis 3 Mal mit Substituenten substituiert ist, unabhängig voneinander ausgewählt aus einem Halogenatom und einem Alkyl- oder Alkoxyrest,
R²¹ ein Wasserstoffatom, einen Alkylrest oder einen Aralkylrest darstellt, dessen Arylgruppe gegebenenfalls 1 bis 3 Mal mit Substituenten substituiert ist, die unabhängig voneinander aus der Gruppe ausgewählt sind, bestehend aus einem Halogenatom, einem Alkylrest, einem Halogenalkylrest, einem Alkoxyrest, einem Halogenalkoxyrest, einem Hydroxyrest, einem Nitrorest, einem Cyanorest, dem Phenylrest, einem Rest SO₂NHR²⁹ und einem Rest NR³⁰R³¹, wobei R²⁹ ein Wasserstoffatom oder einen Alkyl- oder Phenylrest darstellt und R³⁰ und R³¹ unabhängig voneinander Alkylreste darstellen,
R²² ein Wasserstoffatom oder einen Alkylrest darstellt,
oder R²¹ und R²² zusammen mit dem Stickstoffatom einen Heterocyclus von 4 bis 7 Gliedern mit 1 bis 2 Heteroatomen bilden, wobei die zur Vervollständigung des Heterocyclus erforderlichen Glieder unabhängig voneinander aus den Resten -CR³²R³³-, -O-, -S- und -NR³⁴- ausgewählt sind, wobei R³² und R³³ jeweils unabhängig voneinander ein Wasserstoffatom oder einen Alkylrest darstellen und R³⁴ ein Wasserstoffatom, einen Alkyl- oder Aralkylrest darstellt oder R³⁴ einen Phenylrest darstellt, der gegebenenfalls 1 bis 3 Mal mit Substituenten substituiert ist, unabhängig voneinander ausgewählt aus einem Halogenatom und einem Alkyl- oder Alkoxyrest,
R³⁷ und R³⁸ unabhängig voneinander aus einem Wasserstoffatom und einem Alkylrest ausgewählt sind oder R³⁷ und R³⁸ zusammen mit dem Stickstoffatom einen Heterocyclus von 4 bis 7 Gliedern mit 1 bis 2 Heteroatomen bilden, wobei die zur Vervollständigung des Heterocyclus erforderlichen Glieder unabhängig voneinander aus den Resten -CR³⁹R⁴⁰-, -O-, -S- und -NR⁴¹- ausgewählt sind, wobei R³⁹und R⁴⁰ jeweils unabhängig voneinander ein Wasserstoffatom oder einen Alkyrest darstellen und R⁴¹ ein Wasserstoffatom oder einen Alkylrest darstellt;
W O oder S darstellt;
oder ein pharmazeutisch annehmbares Salz einer Verbindung der allgemeinen Formel (I).

2. Produkt nach Anspruch 1, **dadurch gekennzeichnet, dass** die Verbindung der allgemeinen Formel (I) oder ihr pharmazeutisch annehmbares Salz aus den folgenden Verbindungen ausgewählt ist:
5-{[2-(Dimethylamino)ethyl]amino}-2-methyl-1,3-benzothiazol-4,7-dion;
2-Methyl-5-[(2-pyrrolidin-1-ylethyl)amino]-1,3-benzothiazol-4,7-dion;
2-Methyl-5-[(2-piperidin-1-ylethyl)amino]-1,3-benzothiazol-4,7-dion;
2-(2-Chlor-6-fluorophenyl)-5-{[2-(dimethylamino)ethyl]amino}-1,3-benzothiazol-4,7-dion;
und die pharmazeutisch annehmbaren Salze dieser Verbindungen.

3. Produkt nach Anspruch 1, **dadurch gekennzeichnet, dass** der Topoisomerase-I- und/oder -11-Hemmer Camptothecin oder eines seiner Analoga ist.

4. Produkt nach Anspruch 3, **dadurch gekennzeichnet, dass** der Topoisomerase-I- und/oder -II-Hemmer eine Verbindung der allgemeinen Formel (III) 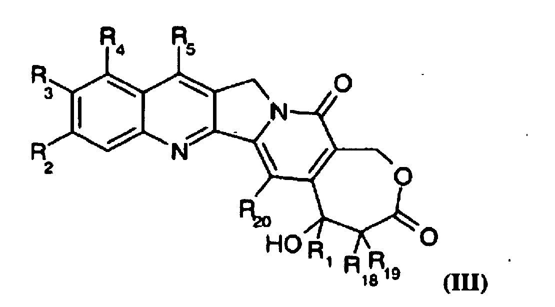 in racemischer, enantiomerer Form oder in allen Kombinationen dieser Formen ist, in der
R₁ ein Niederalkyl, ein Niederalkenyl, ein Niederalkinyl, ein Halogenniederalkyl, ein Niederalkoxyniederalkyl oder ein Niederalkylthioniederalkyl darstellt;
R₂, R₃ und R₄ unabhängig voneinander darstellen i) H, Halogen, Halogenniederalkyl, Niederalkyl, Niederalkenyl, Cyan, Cyanniederalkyl, Nitro, Nitroniederalkyl, Amid, Amidniederalkyl, Hydrazin, Hydrazinniederalkyl, Azid, Azidniederalkyl, (CH₂)ₘNR₆R₇, (CH₂)ₘOR₆, (CH₂)ₘSR₆, (CH₂)ₘCO₂R₆, (CH₂)ₘNR₆C(O)R₈, (CH₂)ₘₘC(O)R₈, (CH₂)OC(O)R₈, O(CH₂)ₘNR₆R₇, OC(O)NR₆R₇, OC(O)(CH₂)ₘCO₂R₆ oder ii) die folgenden substituierten (d.h. ein bis vier Mal an der Arylgruppe oder an dem Heterocyclus substituiert) oder nicht substituierten Reste: (CH₂)ₘ[N=X], OC(O)[N=X], (CH₂)ₘOC(O)[N=X] (worin [N=X] in der vorliegende Erfindung eine heterocyclische Gruppe von 4 bis 7 Gliedern mit dem Stickstoffatom N darstellt, das ein Glied der heterocyclischen Gruppe ist, und X die restlichen Glieder darstellt, die zur Vervollständigung der heterocyclischen Gruppe erforderlich sind, ausgewählt aus der aus O, S, CH₂, CH, N, NR₉ und COR₁₀ bestehenden Gruppe), Aryl oder Arylniederalkyl, bei denen die gegebenenfalls vorhandenen Substituenten ausgewählt sind aus der Gruppe, bestehend aus einem Niederalkyl, Halogen, Nitro, Amino, Niederalkylamino, Halogenniederalkyl, Hydroxyniederalkyl, Niederalkoxy und Niederalkoxyniederalkyl; oder R₂ und R₃ zusammen eine Kette mit 3 oder 4 Gliedern bilden, in der die Elemente der Kette aus der aus CH, CH₂, O, S, N oder NR₉ bestehenden Gruppe ausgewählt sind;
R₅ darstellt i) H, Halogen, Halogenniederalkyl, Niederalkyl, Niederalkoxy, Niederalkoxyniederalkyl, Niederalkylthioniederalkyl, Cycloalkyl, Cycloalkylniederalkyl, Cyan, Cyanalkyl, Niederalkylsulfonylniederalkyl, Hydroxyniederalkyl, Nitro, (CH₂)ₘC(O)R₈, (CH₂)ₘNR₆C(O)R₈, (CH₂)ₘNR₆R₇, (CH₂)ₘN(CH₃)(CH₂)ₙNR₆R₇, (CH₂)ₘOC(O)R₈, (CH₂)mOC(O)NR₆R₇, (CH₂)ₘS(O)_{q}R₁₁, (CH₂)ₘP(O)R₁₂R₁₃, (CH₂)₂P(S)R₁₂R₁₃ oder ii) einen der folgenden substituierten (d.h. ein bis vier Mal an der Aryl- oder Heteroarylgruppe) oder nicht substituierten Reste: (CH₂)ₙ[N=X], OC(O)[N=X], (CH₂)ₘOC(O)[N=X], Aryl oder Arylniederalkyl, in denen die gegebenenfalls vorhandenen Substituenten ausgewählt sind aus der Gruppe, bestehend aus einem Niederalkyl, Halogen, Nitro, Amino, Niederalkylamino, Halogenniederalkyl, Hydroxyniederalkyl, Niederalkoxy und Niederalkoxyniederalkyl;
R₆ und R₇ unabhängig voneinander darstellen i) H, ein Niederalkyl, Hydroxyniederalkyl, Niederalkylaminoniederalkyl, Aminoniederalkyl, Cycloalkyl, Cycloalkylniederalkyl, Niederalkenyl, Niederalkoxyniederalkyl, Halogenniederalkyl oder ii) einen der folgenden substituierten (d.h. ein bis vier Mal an der Arylgruppe) oder nicht substituierten Reste: Aryl oder Arylniederalkyl, in denen die gegebenenfalls vorhandenen Substituenten ausgewählt sind aus der Gruppe, bestehend aus einem Niederalkyl, Halogen, Nitro, Amino, Niederalkylamino, Halogenniederalkyl, Hydroxyniederalkyl, Niederalkoxy und Niederalkoxyniederalkyl;
R₈ darstellt i) H, ein Niederalkyl, Hydroxyniederalkyl, Amino, Niederalkylamino, Niederalkylaminoniederalkyl, Aminoniederalkyl, Cycloalkyl, Cycloalkylniederalkyl, Niederalkenyl, Niederalkoxy, Niederalkoxyniederalkyl, Halogenniederalkyl oder ii) einen der folgenden Substituierten (d.h. ein bis vier Mal an der Arylgruppe) oder nicht substituierten Reste: Aryl oder Arylniederalkyl, in denen die gegebenenfalls vorhandenen Substituenten ausgewählt sind aus der Gruppe, bestehend aus einem Niederalkyl, Halogen, Nitro, Amino, Niederalkylamino, Halogenniederalkyl, Hydroxyniederalkyl, Niederalkoxy oder Niederalkoxyniederalkyl;
R₉ darstellt: H, ein Niederalkyl, Halogeniederalkyl, Aryl oder Aryl, das mit einer oder mehreren Gruppen substituiert ist, ausgewählt aus dem Rest Niederalkyl, Halogen, Nitro, Amino, Niederalkylamino, Halogenniederalkyl, Hydroxyniederalkyl, Niederalkoxy oder Niederalkoxyniederalkyl;
R₁₀ darstellt: H, ein Niederalkyl, Halogenniederalkyl, Niederalkoxy, Aryl oder Aryl, das substituiert ist (d.h. einen bis vier Substituenten an der Arylgruppe aufweist) mit einer oder mehreren Gruppen, ausgewählt aus dem Rest Niederalkyl, Halogenniederalkyl, Hydroxyniederalkyl oder Niederalkoxyniederalkyl;
R₁₁ ein Niederalkyl, Aryl, (CH₂)ₘOR₁₄, (CH₂)ₘSR₁₄, (CH₂)₂NR₁₄R₁₅ oder (CH₂)ₘ[N=X] darstellt;
R₁₂ und R₁₃ unabhängig voneinander ein Niederalkyl, Aryl, Niederalkoxy, Aryloxy oder Amino darstellen;
R₁₄ und R₁₅ unabhängig voneinander H, ein Niederalkyl oder Aryl darstellen;
R₁₈ und R₁₉ unabhängig voneinander H, Halogen, Niederalkyl, Niederalkoxy oder Hydroxy darstellen;
R₂₀ H oder Halogen darstellt;
m eine ganze Zahl von 0 bis 6 ist;
n 1 oder 2 ist; und
q eine ganze Zahl von 0 bis 2 darstellt; und [N=X] eine heterocyclische Gruppe von 4 bis 7 Gliedern darstellt, wobei X die zur Vervollständigung der heterocyclischen Gruppe erforderliche Kette darstellt, die aus der aus O, S, CH₂, CH, N, NR₉ und COR₁₀ bestehenden Gruppe ausgewählt ist;
oder ein pharmazeutisch annehmbares Salz dieser Verbindung.

5. Produkt nach Anspruch 4, **dadurch gekennzeichnet, dass** die Verbindung der allgemeinen Formel (III) oder ihr pharmazeutisch annehmbares Salz ausgewählt ist aus: Diflomotecan und (+)-9-Chlor-5-ethyl-5-hydroxy-10-methyl-12-(4-methylpiperidinomethyl)-4,5,13,15-tetrahydro-1 H,3H-oxepino[3',4':6,7]indolizino[1,2-c]chinolin-3,15-dion und seine pharmazeutisch annehmbaren Salze.

6. Produkt nach Anspruch 3, **dadurch gekennzeichnet, dass** der Topoisomerase-I- und/oder -11-Hemmer die Verbindung SN-38 ist.

7. Produkt nach Anspruch 1, **dadurch gekennzeichnet, dass** der Topoisomerase-I- und/oder -II-Hemmer Doxorubicin oder Amsacrin ist.
